# EUROPEAN PATENT APPLICATION

(11) **EP 2 462 933 A1**
(43) Date of publication of application: **13.06.2012**
(21) Application number: 12001273.7
(22) Date of filing: 02.05.2008
(51) Int. Cl.: A61K 31/40, A61K 9/20, A61P 9/06

(54) **Controlled release oral formulations of ion channel modulating compounds and related methods for preventing arrhythmia**

(30) Priority: 04.05.2007 US 916129 P; 01.08.2007 US 66156 P; 05.03.2008 US 34119 P; 17.03.2008 US 37198 P
(62) Divisional of application: 08755034.9
(71) Applicant: Cardiome Pharma Corp., Vancouver, B.C. V6T 1Z3 (CA)
(72) Inventor: Wheeler, Jeffrey Jerome, Vancouver BC V6T 1Z3 (CA); Beatch, Gregory N., Vancouver BC V6T 1Z3 (CA)
(74) Representative: Grünecker, Kinkeldey, Stockmair & Schwanhäusser

(57) **Abstract**

The present invention provides methods of treating and preventing arrhythmia and other diseases or disorders, using ion channel modulating compounds, including vernakalant hydrochloride. The present invention further provides controlled release oral formulations and dosages of vernakalant hydrochloride, which are effective in preventing arrhythmia. Certain methods and formulations of the present invention are adapted for the treatment and prevention of arrhythmia and other disease or disorders in subjects identified as having altered drug metabolism due to polymorphism of the gene encoding cytochrome P450 2D6.

## Description

### CROSS-REFERENCE(S) TO RELATED APPLICATION(S)

This application claims the benefit under 35 U.S.C. § 119(e) of U.S. Provisional Patent Application No. 60/916,129, filed May 4, 2007; U.S. Provisional Patent Application No. 61/066,156, filed August 1, 2007; U.S. Provisional Patent Application No. 61/034,119, filed March 5, 2008; and U.S. Provisional Patent Application No. 61/037,198, filed March 17, 2008; and, where these (*four*) provisional applications are incorporated herein by reference in their entireties.

### BACKGROUND

### Technical Field

The present invention is directed to controlled release oral formulations of ion channel modulating compounds or pharmaceutically acceptable salts thereof, including unit dosage forms for oral administration. In addition, the present invention is directed to methods of using these compounds, formulations, and unit dosage forms in treating and preventing arrhythmia and other diseases, in particular atrial fibrillation, in mammals, preferably in humans.

### Description of the Related Art

Arrhythmias are abnormal rhythms of the heart. The term "arrhythmia" refers to a deviation from the normal sequence of initiation and conduction of electrical impulses that cause the heart to beat. Arrhythmias may occur in the atria or the ventricles. Atrial arrhythmias are widespread and relatively benign, although they place the subject at a higher risk of stroke and heart failure. Ventricular arrhythmias are typically less common, but very often fatal.

Atrial fibrillation is the most common arrhythmia encountered in clinical practice. It has been estimated that 2.2 million individuals in the United States have paroxysmal or persistent atrial fibrillation. The prevalence of atrial fibrillation is estimated at 0.4% of the general population, and increases with age. Atrial fibrillation is usually associated with age and general physical condition, rather than with a specific cardiac event, as is often the case with ventricular arrhythmia. While not directly life threatening, atrial arrhythmias can cause discomfort and can lead to stroke or congestive heart failure, and increase overall morbidity.

There are two general therapeutic strategies used in treating subjects with atrial fibrillation. One strategy is to allow the atrial fibrillation to continue and to control the ventricular response rate by slowing the conduction through the atrioventricular (AV) node with digoxin, calcium channel blockers or beta-blockers; this is referred to as rate control. The other strategy, known as rhythm control, seeks to convert the atrial fibrillation and then maintain normal sinus rhythm, thus attempting to avoid the morbidity associated with chronic atrial fibrillation. The main disadvantage of the rhythm control strategy is related to the toxicities and proarrhythmic potential of the anti-arrhythmic drugs used in this strategy. Most drugs currently used to prevent atrial or ventricular arrhythmias have effects on the entire heart muscle, including both healthy and damaged tissue. These drugs, which globally block ion channels in the heart, have long been associated with life-threatening ventricular arrhythmia, leading to increased, rather than decreased, mortality in broad subject populations. There is therefore a long recognized need for antiarrhythmic drugs that are more selective for the tissue responsible for the arrhythmia, leaving the rest of the heart to function normally, less likely to cause ventricular arrhythmias.

One specific class of ion channel modulating compounds selective for the tissue responsible for arrhythmia has been described in U.S. Patent No. U.S. Patent No. 7,057,053, including the ion channel modulating compound known as vernakalant hydrochloride. Vernakalant hydrochloride is the non-proprietary name adopted by the United States Adopted Name (USAN) council for the ion channel modulating compound (1 R,2R)-2-[(3R)-hydroxypyrrolidinyl]-1-(3,4-dimethoxyphenethoxy)-cyclohexane monohydrochloride, which compound has the following formula: 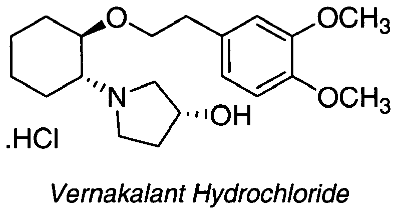 Vernakalant hydrochloride may also be referred to as "vernakalant' herein.

Vernakalant hydrochloride modifies atrial electrical activity through a combination of concentration-, voltage- and frequency-dependent blockade of sodium channels and blockade of potassium channels, including, *e.g.,* the ultra-rapidly activating (I_{Kur}) and transient outward (Iₜₒ) channels. These combined effects prolong atrial refractoriness and rate-dependently slow atrial conduction. This unique profile provides an effective anti-fibrillatory approach suitable for conversion of atrial fibrillation and the prevention of atrial fibrillation.

While significant advances have been made in treating and converting arrhythmias, including the development of vernakalant hydrochloride, there remains a need in the art for improved methods of preventing arrhythmias. Therefore, there also exists a need for controlled release tablet formulations of vernakalant hydrochloride, suitable for the prevention of arrhythmia in mammals, preferably in humans. In addition, there exists a need for methods of treating and preventing arrhythmia in mammals that metabolize anti-arrhythmic drugs at different rates, including mammals having a cytochrome P450(CYP)2D6 genotype associated with poor metabolism of certain drugs, including certain anti-arrhythmic drugs. The present invention fulfills these needs and provides other related advantages.

### BRIEF SUMMARY

The present invention provides new methods for treating or preventing a variety of diseases and disorders with an ion channel modulating compound, including, *e.g.,* ion channel modulating compounds that are metabolized by cytochrome P450(CYP) 2D6.

In one embodiment, the present invention includes a method of treating or preventing arrhythmia in a mammal, comprising: (a) determining if the mammal is a cytochrome P450(CYP)2D6 poor metabolizer (PM) or a cytochrome P450(CYP)2D6 extensive metabolizer; and (b) administering to the mammal a therapeutically effective amount of a composition comprising an ion channel modulating compound having the structure: 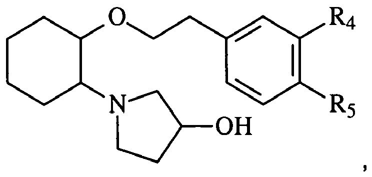 including isolated enantiomeric, diastereomeric and geometric isomers thereof and mixtures thereof, or a solvate or pharmaceutically acceptable salt thereof; wherein R₄ and R₅ are independently selected from hydroxy and C₁-C₆alkoxy.

In a further embodiment, the present invention includes a method of treating or preventing arrhythmia in a mammal, comprising: (a) determining if the mammal is a cytochrome P450(CYP)2D6 poor metabolizer (PM) or a cytochrome P450(CYP)2D6 extensive metabolizer; and (b) administering to the mammal an amount of an ion channel modulating compound sufficient to achieve a blood plasma concentration (Cmax) of between about 0.1 µg/ml and about 10 µg/ml for at least some time, wherein said ion channel modulating compound comprises an ion channel modulating compound of formula: 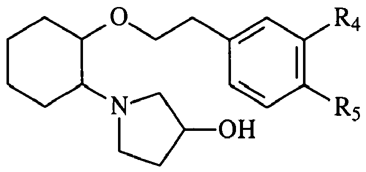 including isolated enantiomeric, diastereomeric and geometric isomers thereof and mixtures thereof, or a solvate or pharmaceutically acceptable salt thereof; wherein R₄ and R₅ are independently selected from hydroxy and C₁-C₆alkoxy.

In one related embodiment, the present invention provides a method of treating or preventing arrhythmia in a mammal who is a cytochrome P450(CYP)2D6 poor metabolizer (PM), comprising: (a) identifying the mammal as a cytochrome P450(CYP)2D6 PM; and (b) administering to the mammal a therapeutically effective amount of a composition comprising an ion channel modulating compound having the structure: 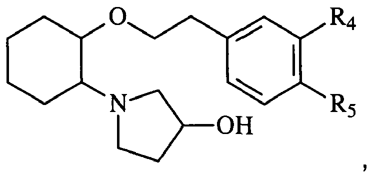 including isolated enantiomeric, diastereomeric and geometric isomers thereof and mixtures thereof, or a solvate or pharmaceutically acceptable salt thereof; wherein R₄ and R₅ are independently selected from hydroxy and C₁-C₆alkoxy.

In another related embodiment, the present invention includes a method of treating or preventing arrhythmia in a mammal who is a cytochrome P450(CYP)2D6 extensive metabolizer (EM), comprising: (a) identifying the mammal as a cytochrome P450(CYP)2D6 EM; and (b) administering to the mammal a therapeutically effective amount of a composition comprising an ion channel modulating compound having the structure: 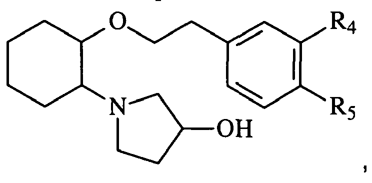 including isolated enantiomeric, diastereomeric and geometric isomers thereof and mixtures thereof, or a solvate or pharmaceutically acceptable salt thereof; wherein R₄ and R₅ are independently selected from hydroxy and C₁-C₆alkoxy.

In a further embodiment, the present invention includes a method of preventing an arrhythmia in a mammal, comprising: (a) identifying a mammal at risk for arrhythmia; (b) determining that the mammal is a cytochrome P450(CYP)2D6 extensive metabolizer (EM); and (c) administering to the mammal a therapeutically effective amount of a composition comprising an ion channel modulating compound having the structure: 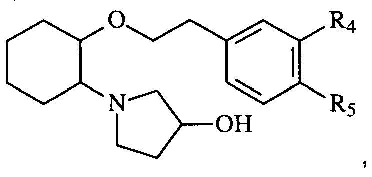 including isolated enantiomeric, diastereomeric and geometric isomers thereof and mixtures thereof, or a solvate or pharmaceutically acceptable salt thereof; wherein R₄ and R₅ are independently selected from hydroxy and C₁-C₆alkoxy, and
wherein said compound is administered to the mammal long-term.

In one particular embodiment, the compound is administered orally.

In yet another related embodiment, the present invention includes a method of identifying a mammal to exclude from long-term treatment with an ion channel modulating compound that is metabolized by cytochrome P450(CYP)2D6 comprising: determining that the mammal is a cytochrome P450(CYP)2D6 poor metabolizer (PM).

In a further embodiment, the present invention provides a method of treating or preventing an arrhythmia in a mammal, comprising: (a) identifying the mammal as a cytochrome P450(CYP)2D6 EM or a cytochrome P450(CYP)2D6 PM; and (b) administering to the mammal a therapeutically effective amount of a composition comprising an ion channel modulating compound having the structure: 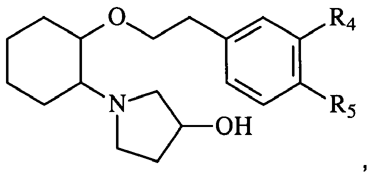 including isolated enantiomeric, diastereomeric and geometric isomers thereof and mixtures thereof, or a solvate or pharmaceutically acceptable salt thereof; wherein R₄ and R₅ are independently selected from hydroxy and C₁-C₆alkoxy, if the mammal is identified as a cytochrome P450(CYP)2D6 EM.

According to various embodiments of the methods of the present invention, administration is by oral, topical, parenteral, sublingual, rectal, vaginal, or intranasal administration.

In particular embodiments, parenteral administration is subcutaneous injection, intravenous injection, intramuscular injection, epidural injection, intrasternal injection, or infusion.

In particular embodiments, oral administration comprises administering an oral dosage form selected from a powder, a granule, a compressed tablet, a pill, a capsule, a cachet, a chewing gum, a wafer, and a lozenge.

In certain embodiments of the methods of the present invention, the arrhythmia is atrial arrhythmia. In one embodiment, the atrial arrhythmia is atrial fibrillation.

In certain embodiments of the methods of the present invention, the arrhythmia is ventricular arrhythmia. In one embodiment, the ventricular arrhythmia is ventricular fibrillation. In on embodiment, the ventricular fibrillation occurs during acute ischemia.

In related embodiments of the methods of the present invention, the arrhythmia is a post-surgical arrhythmia.

In further related embodiments, the arrhythmia is a recurrent arrhythmia in a mammal who has previously undergone one or more arrhythmias.

In additional embodiments of the methods of the present invention the total concentration of the ion channel modulating compound in the blood plasma of the mammal following administration has a mean trough concentration of between about 1 ng/ml and about 10 µg/ml and/or a steady state concentration of between about 1 ng/ml and about 10 µg/ml. In a related embodiment, the total concentration of the ion channel modulating compound in the blood plasma of the mammal following administration has a mean trough concentration of between about 0.3 µg/ml and about 3 µg/ml and/or a steady state concentration of between about 0.3 µg/ml and about 3 µg/ml.

In particular embodiments of the methods of the present invention, the ion channel modulating compound is administered in two or more doses.

In related embodiments of the methods of the present invention, the ion channel modulating compound is administered in one or more doses of a tablet formulation comprising the ion channel modulating compound and at least one hydrophilic matrix system polymer, such as, e.g., carbomer, maltodextrin, hydroxyethyl cellulose, hydroxypropyl cellulose, hydroxypropyl methyl cellulose, and polyoxoacetate.

In one embodiment of the methods of the present invention, the ion channel modulating compound is administered at a dosage of about 50-1500 mg per day.

The present invention also provides, in another embodiment, a method of increasing the bioavailability in a mammal of an ion channel modulating compound that is metabolized by cytochrome P450, comprising administering to said mammal the ion channel modulating compound and an effective amount of cytochrome P450-inhibiting compound.

In a related embodiment, the present invention includes a method of identifying a mammal suitable for long-term treatment with an ion channel modulating compound that is metabolized by cytochrome P450(CYP)2D6 comprising: (a) identifying a mammal at risk for arrhythmia; and (b) determining that the mammal is a cytochrome P450(CYP)2D6 extensive metabolizer (EM). In certain embodiments, the arrhythmia is a recurrent arrhythmia or a post-operative arrhythmia. In particular embodiments, the mammal has previously undergone one or more arrhythmias.

In particular embodiments of the methods of the present invention, the ion channel modulating compound is vernakalant hydrochloride.

In one embodiment, the present invention provides a method of preventing an arrhythmia in a mammal, comprising orally administering to the mammal an effective amount of a controlled release tablet formulation comprising vernakalant hydrochloride and one or more pharmaceutically acceptable excipients for a period of time. In various embodiments, the amount of vernakalant hydrochloride administered to the mammal is greater than 600 mg/day, between 600 mg/day and 1800 mg/day, or about 1000 mg/day. In particular embodiments, the period of time is greater than 48 hours, greater than one week, greater than 30 days, or greater than 90 days. In particular embodiments of formulations used according to the present invention, at least one of the one or more pharmaceutically acceptable excipients is a hydrophilic matrix system polymer selected from the group consisting of carbomer, maltodextrin, hydroxyethyl cellulose, hydroxypropyl cellulose, hydroxypropyl methyl cellulose, and polyoxoacetate. In one specific embodiment, the controlled release tablet formulation comprises: about 250 mg of vernakalant hydrochloride; about 100 mg of hydroxypropyl methyl cellulose; about 25 mg preglatinized starch; about 75 mg silicified microcrystalline cellulose; about 67.5 mg of lactose monohydrate; about 3.75 mg stearic acid; and about 3.75 mg magnesium stearate. In another specific embodiment, the controlled release tablet formulation comprises about 300 mg of vernakalant hydrochloride; about 120 mg of hydroxypropyl methyl cellulose; about 30 mg preglatinized starch; about 90 mg silicified microcrystalline cellulose; about 81 mg of lactose monohydrate; about 4.5 mg stearic acid; and about 4.5 mg magnesium stearate. In another specific embodiment, the controlled release tablet formulation comprises: about 300 mg of vernakalant hydrochloride; about 150 mg cetostearyl alcohol; about 105 mg silicified microcrystalline cellulose; about 111 mg of lactose monohydrate; about 4.5 mg stearic acid; and about 4.5 mg magnesium stearate.

In various embodiments of the methods of the present invention, the effective amount of the controlled release tablet formulation is administered to the mammal in two or more doses per day. In one embodiment, the effective amount of the controlled release tablet formulation is administered to the mammal in two doses per day, wherein each dose comprises about 500 mg of vernakalant hydrochloride. In other embodiments, the effective amount of the controlled release tablet formulation is administered in two doses per day, wherein each dose comprises about 300 mg of vernakalant hydrochloride.

In another related embodiment, the present invention includes a unit dosage form of vernakalant hydrochloride, comprising between 150 mg and 300 mg of vernakalant hydrochloride. In one embodiment, the unit dosage form comprises about 250 mg of vernakalant hydrochloride. In another embodiment, the unit dosage form comprises about 250 mg of vernakalant hydrochloride. In a further embodiment, the unit dosage form comprises about 300 mg of vernakalant hydrochloride. In another embodiment, the unit dosage form comprises about 500 mg of vernakalant hydrochloride.

In a further related embodiment, the present invention provides a controlled release tablet formulation of vernakalant hydrochloride, comprising about 250 mg of vernakalant hydrochloride; about 100 mg of hydroxypropyl methyl cellulose; about 25 mg preglatinized starch; about 75 mg silicified microcrystalline cellulose; about 67.5 mg of lactose monohydrate; about 3.75 mg stearic acid; and about 3.75 mg magnesium stearate.

In another related embodiment, the present invention provides a controlled release tablet formulation of vernakalant hydrochloride, comprising: about 300 mg of vernakalant hydrochloride; about 120 mg of hydroxypropyl methyl cellulose; about 30 mg preglatinized starch; about 90 mg silicified microcrystalline cellulose; about 81 mg of lactose monohydrate; about 4.5 mg stearic acid; and about 4.5 mg magnesium stearate.

In yet anoher related embodiment, the present invention provides a controlled release tablet formulation of vernakalant hydrochloride, comprising: about 300 mg of vernakalant hydrochloride; about 150 mg cetostearyl alcohol; about 105 mg silicified microcrystalline cellulose; about 111 mg of lactose monohydrate; about 4.5 mg stearic acid; and about 4.5 mg magnesium stearate.

In certain embodiments of the methods of the present invention, the arrhythmia is a recurring arrhythmia. In other embodiments, the arrhythmia is a post-operative arrhythmia.

In a further embodiment, the present invention includes a method of preventing or postponing the recurrence of an arrhythmia in a mammal, comprising orally administering to the mammal an effective amount of vernakalant hydrochloride, wherein said vernakalant hydrochloride is administered to the mammal at a dosage of about 500 mg b.i.d. In one embodiment, the vernakalant hydrochloride is administered to the mammal in a controlled release oral tablet formulation, wherein each tablet comprises about 250 mg of vernakalant hydrochloride; about 100 mg of hydroxypropyl methyl cellulose; about 25 mg preglatinized starch; about 75 mg silicified microcrystalline cellulose; about 67.5 mg of lactose monohydrate; about 3.75 mg stearic acid; and about 3.75 mg magnesium stearate.

### BRIEF DESCRIPTION OF THE SEVERAL VIEWS OF THE DRAWINGS

Figure 1 shows the dissolution profile of a comparative immediate release tablet formulation comprising 100 mg of the active ingredient over time.
Figure 2 is a graph showing the lack of effect of oral vernakalant on QTc interval over 30 days.
Figure 3 is a graph depicting the maintenance of sinus rhythm resulting from placebo or 300 or 600 mg bid of oral vernakalant over 30 days.
Figure 4 provides graphs depicting the mean plasma concentration-time profiles of vernakalant hydrochloride and its metabolites after IV infusion in extensive metabolizers (EMs; Figure 4A) and poor metabolizers (PMs; Figure 4B). G indicates glucuronide or glucuronidated.
Figure 5 provides graphs depicting the mean plasma concentration-time profiles of vernakalant hydrochloride and its metabolites after oral administration in EMs (Figure 5A) and PMs (Figure 5B).
Figure 6 is a bar graph showing the excretion of radioactivity in urine and feces after IV infusion or oral administration of ¹⁴C-vernakalant hydrochloride.
Figure 7 provides a diagram of the metabolism of vernakalant in EMs and PMs.
Figure 8 provides graphs depicting the effect of CYP2D6 genotype on vernakalant hydrochloride Cmax (Figure 8A) and AUC₀₋₉₀ (Figure 8B).

### DETAILED DESCRIPTION

Vernakalant hydrochloride is an antiarrhythmic drug previously shown to block sodium channels and early activating potassium channels to prolong atrial refractoriness and convert atrial fibrillation (AF) to sinus rhythm when administered intravenously. In a randomized, controlled trial of patients with recent-onset AF or atrial flutter, vernakalant hydrochloride terminated the atrial arrhythmia in 61% compared with a 5% termination rate with placebo (P<.0005).

The present invention is based, in part, upon clinical trials that demonstrate that orally administered vernakalant hydrochloride prevents the recurrence of arrhythmia. As described in the accompanying Examples, appropriate oral dosages of vernakalant hydrochloride formulations in patients with symptomatic AF were able to maintain sinus rhythm following conversion from AF. Accordingly, the present invention provides methods of preventing arrhythmia, as well as unit dosage forms of vernakalant hydrochloride adapted for oral administration, and oral dosing regimes effective in preventing arrhythmia.

The present invention is also directed to controlled release tablet formulations comprising a therapeutically effective amount of ion channel modulating compound, or a pharmaceutically acceptable salt thereof, and one or more pharmaceutically acceptable excipients. In particular, the present invention is directed to controlled release tablet formulations comprising a therapeutically effective amount of vernakalant hydrochloride and one or more pharmaceutically acceptable excipients suitable for controlled release formulations, which, upon oral administration thereto, are effective in preventing arrhythmia in mammals, preferably in humans. In one embodiment, the controlled release tablet formulations of the invention are intended to be administered to a mammal, preferably a human, that has previously undergone one or more arrhythmias, or who is considered at risk of arrythmia.

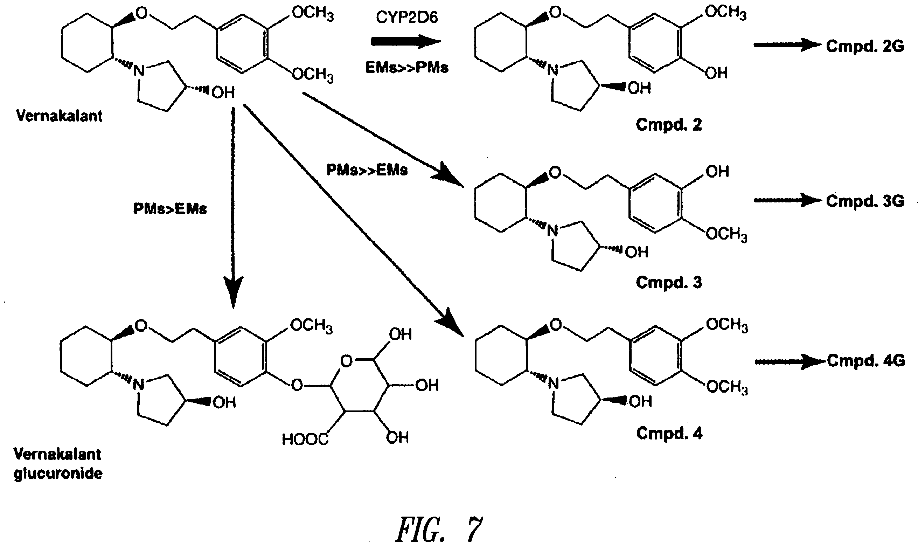

As described herein, vernakalant hydrochloride has been found to be metabolized primarily by cytochrome P450(CYP)2D6 (also referred to as CYP2D6), with Compound 2 (described herein; Figure 7)) being the major metabolite produced by 4-O-demethylation mediated by CYP2D6. Other metabolites include a direct glucuronidation, a 3-O-demethylated compound (Compound 3; Figure 7), and a vernakalant diastereomer (Compound 4; Figure 7).

CYP2D6 is subject to genetic polymorphism that can influence the pharmacokinetics and disposition of drugs that depend on it for metabolism; these differences can affect their efficacy or safety (Kirchheiner, J. and Seeringer, A. Biochim. Biophys. Acta 1770:489-494 (2007)). In most individuals, referred to as extensive metabolizers (EMs), drugs are metabolized effectively by CYP2D6. However, people who carry a homozygous set of a CYP2D6 polymorphism that renders the isoenzyme ineffective are considered poor metabolizers (PMs); approximately 7% of whites and 1% of Asians are PMs (Bertilsson, L. Clin. Pharmacokinet. 29:192-209 (1995)).

The present invention is based, in part, on the discovery that the disposition and metabolic profile of vernakalant hydrochloride depends on a subject's cytochrome P450 (CYP)2D6 genotype. As described in the accompanying Examples, vernakalant hydrochloride underwent rapid and extensive distribution during infusion, with a mean steady state volume of distribution of 123.1 L for extensive metabolizers (EMs) and 112.7 L for poor metabolizers (PMs), which resulted in similar Cₘₐₓ values in EMs and PMs with IV, but not oral, dosing. Vernakalant hydrochloride was metabolized rapidly and extensively to a 4-O-demethylated metabolite with glucuronidation in CYP2D6 EMs, while direct glucuronidation predominated in CYP2D6 PMs. Several minor metabolites were also detected in plasma at higher levels in PMs than in EMs. Slower clearance in PMs contributed to their 3 and 6 times higher overall drug exposure (IV and oral dosing, respectively). Urinary recovery of unchanged vernakalant hydrochloride was higher in PMs, as well, and supported the pharmacokinetic and metabolic profile seen in plasma.

Since the pharmacokinetics and metabolism of vernakalant hydrochloride depend on the CYP2D6 genotype, the genotype of a patient to whom vernakalant hydrochloride is administered may be clinically significant, particularly during long-term or chronic administration of vernakalant hydrochloride (*e.g.,* to prevent arrhythmia or the recurrence of arrhythmia). Accordingly, the present invention provides methods of treating and preventing arrhythmia with vernakalant hydrochloride and other ion channel modulating compounds, which include determining the CYP2D6 genotype (*e.g.,* PM or EM) of a patient. In addition, the present invention provides methods specific for the treatment or preventing arrhythmia in either PM or EM patients, including methods of achieving therapeutically effective blood plasma levels in each of these patient populations, as well as dosage regimes specific for each patient population.

The methods of the present invention may be applied to treat or prevent any disease or disorder that will benefit from treatment with one or more of the ion channel modulating compounds described herein, including those ion channel modulating compounds that are metabolized by CYP2D6.

While the methods of the present invention are particularly advantageous for the treatment and prevention of arrhythmia, they may also be used to treat or prevent other diseases, including, *e.g.,* cardiovascular diseases and diseases and disorders associated with inflammation. Examples of particular diseases and conditions to which the methods of the present invention may be applied include: arrhythmia, diseases of the central nervous system, cardiovascular diseases, convulsion, epileptic spasms, depression, anxiety, schizophrenia, Parkinson's disease, respiratory disorders, cystic fibrosis, asthma, cough, inflammation, arthritis, allergies, gastrointestinal disorders, urinary incontinence, irritable bowel syndrome, cardiovascular diseases, cerebral or myocardial ischemias, hypertension, long-QT syndrome, stroke, migraine, ophthalmic diseases, diabetes mellitus, myopathies, Becker's myotonia, myasthenia gravis, paramyotonia congentia, malignant hyperthermia, hyperkalemic periodic paralysis, Thomsen's myotonia, autoimmune disorders, graft rejection in organ transplantation or bone marrow transplantation, heart failure, hypotension, Alzheimer's disease or other mental disorder, multiple sclerosis, spinal cord injury, and alopecia.

Examples of specific cardiovascular diseases or disorders that may be treated or prevented by the methods of the present invention include, but are not limited to: arrhythmia, atrial arrhythmia, atrial fibrillation, atrial flutter, ventricular arrhythmia, ventricular tachycardia, ventricular fibrillation, myocardial infarction, myocardial ischemia, arrhythmia induced by coronary artery occlusion, myocardial ischaemia, myocardial inflammation, post-operative arrhythmia (*e.g.,* following cardiac surgery such as CABG). In certain embodiments, the methods may be used to treat or prevent sustained atrial fibrillation (atrial fibrillation of longer than 72 hours and less than 6 months duration) or chronic atrial fibrillation. These methods may also be used to prevent the recurrence of an arrhythmia in a warm-blooded animal having previously undergone one or more arrhythmias or considered at risk or arrhythmia, *e.g.,* during or following a surgical procedure.

As used herein, unless the context makes clear otherwise, "treatment," and similar word such as "treated," "treating" etc., is an approach for obtaining beneficial or desired results, including and preferably clinical results. Treatment can involve optionally either the amelioration of symptoms of the disease or condition, or the delaying of the progression of the disease or condition (*e.g.,* arrhythmia).

As used herein, unless the context makes clear otherwise, "prevention," and similar word such as "prevented," "preventing" etc., is an approach for preventing the onset or recurrence of a disease or condition or preventing the occurrence or recurrence of the symptoms of a disease or condition, or optionally an approach for delaying the onset or recurrence of a disease or condition or delaying the occurrence or recurrence of the symptoms of a disease or condition. As used herein, "prevention" and similar words also includes reducing the intensity, effect, symptoms and/or burden of a disease or condition prior to onset or recurrence of the disease or condition.

As used herein, an "effective amount" or a "therapeutically effective amount" of a substance is that amount sufficient to affect a desired biological effect, such as beneficial results, including clinical results. For example, in the context of treating an arrhythmia using the methods of the present invention, an effective amount of ion modulating compound is that amount sufficient to reduce the defibrillation energy threshold required to convert the arrhythmia to normal rhythm.

### A. Ion Channel Modulating Compounds

Generally, any ion channel modulating compound capable of treating or preventing arrhythmia or any other disease or disorder, including those specifically described herein, may be used in the methods, formulations, and unit dosage forms of the present invention.

As noted above, and in one specific embodiment, the ion channel modulating compound is vernakalant hydrochloride, which compound has the following formula:

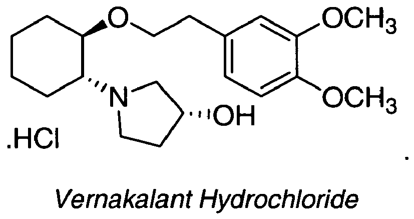

More generally, the ion channel modulating compound is any isomeric or pharmaceutically acceptable salt form of vernakalant, as represented by the following formula (I): 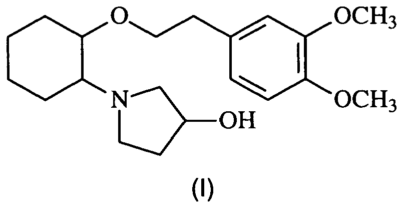 including isolated enantiomeric, diastereomeric and geometric isomers thereof and mixtures thereof, or a solvate or pharmaceutically acceptable salt thereof.

In more specific forms of Formula (I), the ion channel modulating compound is in a trans- or cis-configuration, as represented by Formulas (IIa) and (IIb), respectively: 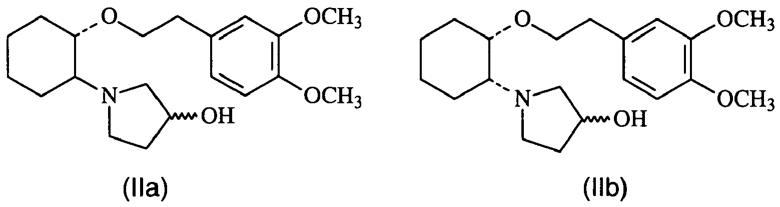 or a solvate or pharmaceutically acceptable salt thereof.

In more general terms of Formula (I), the ion channel modulating compound is by the following formula (Ia): 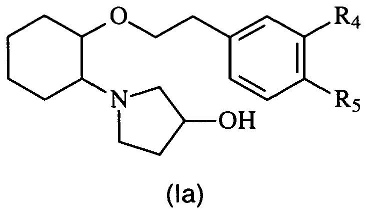 including isolated enantiomeric, diastereomeric and geometric isomers thereof and mixtures thereof, or a solvate or pharmaceutically acceptable salt thereof; wherein R₄ and R₅ are independently selected from hydroxy and C₁-C₆alkoxy.

In further embodiments, the ion channel modulating compounds are represented by Formula (III): 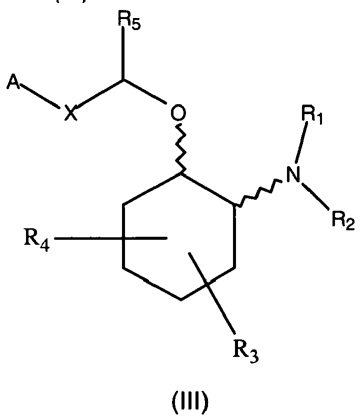 or an isomer or pharmaceutical acceptable salt thereof,
wherein, independently at each occurrence,
X is selected from -C(R₆,R₁₄)-Y-, and -C(R₁₃)=CH-;
Y is selected from a direct bond, O, S, and C₁-C₄alkylene;
R₁₃ is selected from hydrogen, C₁-C₆alkyl, C₃-C₈cydoalkyl, aryl, and benzyl;
R₁ and R₂, when taken together with the nitrogen atom to which they are directly attached in formula (III), form a ring denoted by formula (IV): 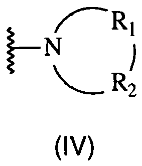 wherein the ring of formula (IV) is formed from the nitrogen as shown as well as three to nine additional ring atoms independently selected from carbon, nitrogen, oxygen, and sulfur; where any two adjacent ring atoms may be joined together by single or double bonds, and where any one or more of the additional carbon ring atoms may be substituted with one or two substituents selected from hydrogen, hydroxy, C₁-C₃hydroxyalkyl, oxo, C₂-C₄acyl, C₁-C₃alkyl, C₂-C₄alkylcarboxy, C₁-C₃alkoxy, C₁-C₂₀alkanoyloxy, or may be substituted to form a spiro five- or six-membered heterocyclic ring containing one or two heteroatoms selected from oxygen and sulfur; and any two adjacent additional carbon ring atoms may be fused to a C₃-C₈carbocyclic ring, and any one or more of the additional nitrogen ring atoms may be substituted with substituents selected from hydrogen, C₁-C₆alkyl, C₂-C₄acyl, C₂-C₄hydroxyalkyl and C₃-C₈alkoxyalkyl; or
R₁ and R₂, when taken together with the nitrogen atom to which they are directly attached in formula (III), may form a bicyclic ring system selected from 3-azabicyclo[3.2.2]nonan-3-yl, 2-aza-bicyclo[2.2.2]octan-2-yl, 3-azabicyclo[3.1.0]-hexan-3-yl, and 3-azabicyclo[3.2.0]-heptan-3-yl;
R₃ and R₄ are independently attached to the cyclohexane ring shown in formula (III) at the 3-, 4-, 5- or 6- positions and are independently selected from hydrogen, hydroxy, C₁-C₆alkyl, and C₁-C₆alkoxy;
R₅, R₆ and R₁₄ are independently selected from hydrogen, C₁-C₆alkyl, aryl and benzyl;
A is selected from C₅-C₁₂alkyl, a C₃-C₁₃carbocyclic ring, and ring systems selected from formulae (V), (VI), (VII), (VIII), (IX) and (X): 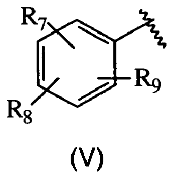 where R₇, R₈ and R₉ are independently selected from bromine, chlorine, fluorine, carboxy, hydrogen, hydroxy, hydroxymethyl, methanesulfonamido, nitro, sulfamyl, trifluoromethyl, C₂-C₇alkanoyloxy, C₁-C₆alkyl, C₁-C₆alkoxy, C₂-C₇alkoxycarbonyl, C₁-C₆thioalkyl and N(R₁₅,R₁₆) where R₁₅ and R₁₆ are independently selected from hydrogen, acetyl, methanesulfonyl, and C₁-C₆alkyl; 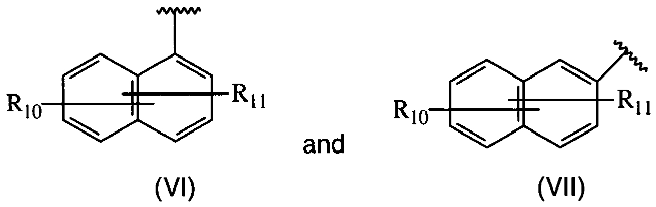 where R₁₀ and R₁₁ are independently selected from bromine, chlorine, fluorine, carboxy, hydrogen, hydroxy, hydroxymethyl, methanesulfonamido, nitro, sulfamyl, trifluoromethyl, C₂-C₇alkanoyloxy, C₁-C₆alkyl, C₁-C₆alkoxy, C₂-C₇alkoxycarbonyl, C₁-C₆thioalkyl, and N(R₁₅,R₁₆) where R₁₅ and R₁₆ are independently selected from hydrogen, acetyl, methanesulfonyl, and C₁-C₆alkyl; 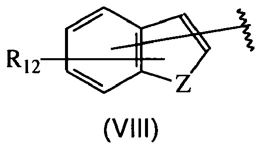 where R₁₂ is selected from bromine, chlorine, fluorine, carboxy, hydrogen, hydroxy, hydroxymethyl, methanesulfonamido, nitro, sulfamyl, trifluoromethyl, C₂-C₇alkanoyloxy, C₁-C₆alkyl, C₁-C₆alkoxy, C₂-C₇alkoxycarbonyl, C₁-C₆thioalkyl, and N(R₁₅,R₁₆) where R₁₅ and R₁₆ are independently selected from hydrogen, acetyl, methanesulfonyl, and C₁-C₆alkyl; and Z is selected from CH, CH₂, O, N and S, where Z may be directly bonded to "X" as shown in formula (III) when Z is CH or N, or Z may be directly bonded to R₁₇ when Z is N, and R₁₇ is selected from hydrogen, C₁-C₆alkyl, C₃-C₈cydoalkyl, aryl and benzyl; 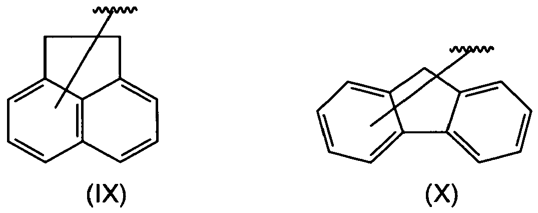 including isolated enantiomeric, diastereomeric and geometric isomers thereof, and mixtures thereof.

In more specific embodiments of Formula (III), the ion channel modulating compound is one or more of the following compounds:
(+)-*trans*-[2-(4-morpholinyl)-1-(2-naphthenethoxy)]cyclohexane;
(-)-*trans*-[2-(4-morpholinyl)-1-(2-naphthenethoxy)]cyclohexane;
(+)-*trans*-[2-(4-morpholinyl)-1-(1-naphthenethoxy)]cyclohexane;
(-)-*trans*-[2-(4-morpholinyl)-1-(1-naphthenethoxy)]cyclohexane;
(+)-*trans*-[2-(4-morpholinyl)-1-(4-bromophenethoxy)]cyclohexane;
(-)-*trans*-[2-(4-morpholinyl)-1-(4-bromophenethoxy)]cyclohexane;
(+)-*trans*-[2-(4-morpholinyl)-1-[2-(2-naphthoxy)ethoxy)]cyclohexane;
(-)-*trans*-[2-(4-morpholinyl)-1-[2-(2-naphthoxy)ethoxy)]cyclohexane;
(+)-*trans*-[2-(4-morpholinyl)-1-[2-(4-bromophenoxy)ethoxy]]cyclohexane;
(-)-*trans*-[2-(4-morpholinyl)-1-[2-(4-bromophenoxy)ethoxy]]cyclohexane;
(+)-*trans*-[2-(4-morpholinyl)-1-(3,4-dimethoxyphenethoxy)]cyclohexane;
(-)-*trans*-[2-(4-morpholinyl)-1-(3,4-dimethoxyphenethoxy)]cyclohexane;
(+)-*trans*-[2-(1-pyrrolidinyl)-1-(1-naphthenethoxy)]cyclohexane;
(-)-*trans*-[2-(1-pyrrolidinyl)-1-(1-naphthenethoxy)]cyclohexane;
(+)-*trans*-[2-(4-morpholinyl)-1-(2-(benzo[b]thiophen-3-yl)ethoxy)]-cyclohexane;
(-)-*trans*-[2-(4-morpholinyl)-1-(2-(benzo[b]thiophen-3-yl)ethoxy)]cyclohexane;
(+)-**trans**-[2-(4-morpholinyl)-1-(2-(benzo[b]thiophen-4-yl)ethoxy)]-cyclohexane;
(-)-**trans**-[2-(4-morpholinyl)-1-(2-(benzo[b]thiophen-4-yl)ethoxy)]-cyclohexane;
(+)-**trans**-[2-(4-morpholinyl)-1-(3-bromophenethoxy)]cyclohexane;
(-)-*trans*-[2-(4-morpholinyl)-1-(3-bromophenethoxy)]cyclohexane;
(+)-*trans*-[2-(4-morpholinyl)-1-(2-bromophenethoxy)]cyclohexane;
(-)-*trans*-[2-(4-morpholinyl)-1-(2-bromophenethoxy)]cyclohexane;
(+)-*trans*-[2-(4-morpholinyl)-1-(3-(3,4-dimethoxyphenyl)-1-propoxy)]-cyclohexane;
(-)-*trans*-[2-(4-morpholinyl)-1-(3-(3,4-dimethoxyphenyl)-1-propoxy)]cyclohexane;
(1R,2R)/(1S,2S)-2-(4-morpholinyl)-1-(3,4-dichlorophenethoxy)-cyclohexane;
(1R,2R)/(1S,25)-2-(3-ketopyrrolidinyl)-1-(1-naphthenethoxy)-cycclohexane;
(1R,2R)/(1S,2S)-2-(1-acetylpiperazinyl)-1-(2-naphthenethoxy)-cyclohexane;
(1R,2R)/(1 S,2S)-2-(3-ketopyrrolidinyl)-1-(2,6-dichlorophenethoxy)-cyclohexane;
(1R,2R)/(1S,2S)-2-[1,4-dioxa-7-azaspiro[4.4]non-7-yl]-1-(1-naphthenethoxy)cyclohexane;
(1R,2S)/(1S,2R)-2-(4-morpholinyl)-1-[(2-trifluoromethyl)phenethoxy]-cyclohexane;
(1R,2R)/(1S,2S)-2-(3-ketopyrrolidinyl)-1-[3-(cyclohexyl)propoxy]-cyclohexane;
(1R,2R)/(1S,2S)-2-(3-acetoxypyrrolidinyl)-1-(1-naphthenethoxy)-cyclohexane ;
(1R,2R)/(1S,25)-2-(3-hydroxypyrrolidinyl)-1-(2,6-dichlorophenethoxy)-cyclohexane;
(1R,2R)/(1S,2S)-2-(3-ketopyrrolidinyl)-1-(2,2-diphenylethoxy)-cyclohexane;
(1R,2R)/(1S,2S)-2-(3-thiazolidinyl)-1-(2,6-dichlorophenethoxy)-cyclohexane; and
(1R,2S)/(1S,2R)-2-(3-ketopyrrolidinyl)-1-(-naphthenethoxy)-cyclohexane;
   including isolated enantiomeric and diastereomeric isomers thereof, and mixtures thereof; and pharmaceutically acceptable salts thereof.

Certain compounds of the present invention contain at least two asymmetric carbon atoms and, thus, exist as enantiomers and diastereomers. Unless otherwise noted, the present invention includes all enantiomeric and diastereomeric forms of the aminocyclohexyl ether compounds of the invention. Pure stereoisomers, mixtures of enantiomers and/or diastereomers, and mixtures of different compounds of the invention are included within the present invention. Thus, compounds of the present invention may occur as racemates, racemic mixtures and as individual diastereomers, or enantiomers with all isomeric forms being included in the present invention. A racemate or racemic mixture does not imply a 50:50 mixture of stereoisomers.

The phrase "independently at each occurrence" is intended to mean (i) when any variable occurs more than one time in a compound of the invention, the definition of that variable at each occurrence is independent of its definition at every other occurrence; and (ii) the identity of any one of two different variables *(e.g.,* R₁ within the set R₁ and R₂) is selected without regard the identity of the other member of the set. However, combinations of substituents and/or variables are permissible only if such combinations result in stable compounds.

As used herein, the following terms are defined to have following meanings, unless explicitly stated otherwise:
"Acid addition salts" refers to those salts which retain the biological effectiveness and properties of the free bases and which are not biologically or otherwise undesirable, formed with inorganic acids such as hydrochloric acid, hydrobromic acid, sulfuric acid, nitric acid, phosphoric acid and the like, or organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid and the like.
"Acyl" refers to branched or unbranched hydrocarbon fragments terminated by a carbonyl -(C=O)- group containing the specified number of carbon atoms. Examples include acetyl [CH₃C=O-, a C₂acyl] and propionyl [CH₃CH₂C=O-, a C₃acyl].
"Alkanoyloxy' refers to an ester substituent wherein the ether oxygen is the point of attachment to the molecule. Examples include propanoyloxy [(CH₃CH₂C=O-O-, a C₃alkanoyloxy] and ethanoyloxy [CH₃C=O-O-, a C₂alkanoyloxy].
"Alkoxy" refers to an O-atom substituted by an alkyl group, for example, methoxy [-OCH₃, a C₁alkoxy].
"Alkoxyalkyl" refers to a alkylene group substituted with an alkoxy group. For example, methoxyethyl [CH₃OCH₂CH₂-] and ethoxymethyl (CH₃CH₂OCH₂-] are both C₃alkoxyalkyl groups.
"Alkoxycarbonyl" refers to an ester substituent wherein the carbonyl carbon is the point of attachment to the molecule. Examples include ethoxycarbonyl [CH₃CH₂OC=O-, a C₃alkoxycarbonyl] and methoxycarbonyl [CH₃0C=O-, a C₂alkoxycarbonyl].
"Alkyl" refers to a branched or unbranched hydrocarbon fragment containing the specified number of carbon atoms and having one point of attachment. Examples include n-propyl (a C₃alkyl), *iso*-propyl (also a C₃alkyl), and t-butyl (a C₄alkyl).
"Alkylene" refers to a divalent radical which is a branched or unbranched hydrocarbon fragment containing the specified number of carbon atoms, and having two points of attachment. An example is propylene [-CH₂CH₂CH₂-, a C₃alkylene].
"Alkylcarboxy" refers to a branched or unbranched hydrocarbon fragment terminated by a carboxylic acid group [-COOH]. Examples include carboxymethyl [HOOC-CH₂-, a C₂alkylcarboxy] and carboxyethyl [HOOC-CH₂CH₂-, a C₃alkylcarboxy].
"Aryl" refers to aromatic groups which have at least one ring having a conjugated pi electron system and includes carbocyclic aryl, heterocyclic aryl (also known as heteroaryl groups) and biaryl groups, all of which may be optionally substituted. Carbocyclic aryl groups are generally preferred in the compounds of the present invention, where phenyl and naphthyl groups are preferred carbocyclic aryl groups.
"Aralkyl" refers to an alkylene group wherein one of the points of attachment is to an aryl group. An example of an aralkyl group is the benzyl group [C₆H₅CH₂-, a C₇aralkyl group].
"Cycloalkyl" refers to a ring, which may be saturated or unsaturated and monocyclic, bicyclic, or tricyclic formed entirely from carbon atoms. An example of a cycloalkyl group is the cyclopentenyl group (C₅H₇-), which is a five carbon (C₅) unsaturated cycloalkyl group.
"Carbocyclic" refers to a ring which may be either an aryl ring or a cycloalkyl ring, both as defined above.
"Carbocyclic aryl" refers to aromatic groups wherein the atoms which form the aromatic ring are carbon atoms. Carbocyclic aryl groups include monocyclic carbocyclic aryl groups such as phenyl, and bicyclic carbocyclic aryl groups such as naphthyl, all of which may be optionally substituted.
"Heteroatom" refers to a non-carbon atom, where boron, nitrogen, oxygen, sulfur and phosphorus are preferred heteroatoms, with nitrogen, oxygen and sulfur being particularly preferred heteroatoms in the compounds of the present invention.
"Heteroaryl" refers to aryl groups having from 1 to 9 carbon atoms and the remainder of the atoms are heteroatoms, and includes those heterocyclic systems described in "Handbook of Chemistry and Physics," 49th edition, 1968, R.C. Weast, editor; The Chemical Rubber Co., Cleveland, OH. See particularly Section C, Rules for Naming Organic Compounds, B. Fundamental Heterocyclic Systems. Suitable heteroaryls include furanyl, thienyl, pyridyl, pyrrolyl, pyrimidyl, pyrazinyl, imidazolyl, and the like.
"Hydroxyalkyl" refers to a branched or unbranched hydrocarbon fragment bearing an hydroxy (-OH) group. Examples include hydroxymethyl (-CH₂OH, a C₁hydroxyalkyl) and 1-hydroxyethyl (-CHOHCH₃, a C₂hydroxyalkyl).
"Thioalkyl" refers to a sulfur atom substituted by an alkyl group, for example thiomethyl (CH₃S-, a C₁thioalkyl).
"Modulating" in connection with the activity of an ion channel means that the activity of the ion channel may be either increased or decreased in response to administration of a compound or composition or method of the present invention. Thus, the ion channel may be activated, so as to transport more ions, or may be blocked, so that fewer or no ions are transported by the channel.
"Pharmaceutically acceptable salt" refers to salts of the compounds of the present invention derived from the combination of such compounds and an organic or inorganic acid (acid addition salts) or an organic or inorganic base (base addition salts). The compounds of the present invention may be used in either the free base or salt forms, with both forms being considered as being within the scope of the present invention.

Representative ion channel modulating compounds are more specifically disclosed in U.S. Patent No. 7,057,053 and U.S. Patent No. 7,345, 087, both of which are incorporated in therein entirety herein by reference. Further, methods of synthesizing and producing the ion channel modulating compounds of the present invention are described, *e.g.,* in U.S. Patent No. 7,259,184 and U.S. Patent Application Serial Nos. 10/838,470, 11/757,880, 11/690,361, 11/719,737, and 11/455,280, all of which are incorporated herein by reference in their entirety.

### B. Methods of Preventing Arrhythmia Using Ion Channel Modulating Compounds Based Upon CYP2D6 Genotype

In certain embodiments, the present invention provides methods of treating or preventing a disease or disorder, *e.g.,* an arrhythmia, comprising providing subjects or patients (*e.g.,* mammals or warm-blooded animals, including humans and other animals) with an ion channel modulating compound that is metabolized by the gene product of the CYP2D6 gene (such as, but not limited to, vernakalant hydrochloride), which methods may include determining the CYP2D6 genotype of the mammal, *e.g.,* whether the patient is a CYP2D6 PM or EM.

As described above, the majority of individuals possess normal CYP2D6 activity and are referred to as extensive metabolizers (EMs). However, certain individuals lack CYP2D6 enzyme activity, due to inactivating mutations in both copies of the CYP2D6 gene, and are unable to metabolize drugs that require the CYP2D6 enzyme. These individuals are referred to as CYP2D6 poor metabolizers (PMs). In addition, individuals possessing slightly reduced activity, *e.g.,* due to the inactivation of a single CYP2D6 gene, are referred to as intermediate metabolizers, and individuals with increased enzyme activity, in part due to gene duplications, are referred to as rapid metabolizers.

While the present methods exemplify, in particular, methods of treatment or prevention directed to EM or PMs, the skilled artisan will understand that these methods may be adapted for intermediate metabolizers and rapid metabolizers. Since intermediate metabolizers possess reduced CYP2D6 enzyme activity, the methods described herein with respect to PMs may also apply to intermediate metabolizers. In addition, since rapid metabolizers have increased CYP2D6 enzyme activity, methods described herein may be readily adapted to apply to rapid metabolizers.

For example, according to the various embodiments of the methods of the present invention, the CYP2D6 genotype may be determined by identifying a patient as a PM, an EM, an intermediate metabolizer, or a rapid metabolizer. In addition, methods of the present invention may be used, in one embodiment, to exclude either intermediate or rapid metabolizers. Accordingly, the methods of the present invention are not limited to EMs and PMs, but may also be practiced on intermediate metabolizers and rapid metabolizers.

In certain embodiments, PMs and/or intermediate metabolizers may be administered a reduced amount of an ion channel modulating compound as compared to the amount administered to an EM. In other embodiments, a rapid metabolizer may be administered an increased amount of an ion channel modulating compound as compared to the amount administered to an EM. The reduced or increased amount may be the total amount administered at any one time or in any one day, or it may refer to the duration of time that the ion channel modulating compound is administered.

As noted above, the present invention is based, in part, upon the discovery that CYP2D6 PMs accumulate a higher concentration of the ion channel modulating compounds of the present invention than EMs. Accordingly, it may be desirous to know, or to determine, the CYP2D6 status of a mammal before administering an ion channel compound to the mammal, particularly if the ion channel compound is metabolized by CYP2D6 and will be administered for an extended duration of time.

Thus, in one embodiment, the present invention includes methods of treating or preventing arrhythmia in a mammal comprising administering to the mammal a therapeutically effective amount of an ion channel modulating compound of the present invention, wherein the mammal is known or determined to be a cytochrome P450(CYP)2D6 poor metabolizer (PM) or a cytochrome P450(CYP)2D6 extensive metabolizer (EM). In another embodiment, a method is disclosed for treating or preventing arrhythmia in a mammal comprising determining if the mammal is a cytochrome P450(CYP)2D6 poor metabolizer (PM) or a cytochrome P450(CYP)2D6 extensive metabolizer (EM), and administering to the mammal a therapeutically effective amount of an ion channel modulating compound of the present invention. In certain embodiments, the therapeutically effective amount administered to a PM is less than the therapeutically effective amount administered to an EM. In particular embodiments, the ion channel modulating compound is administered orally in one or more dosages. In related embodiments, the ion channel modulating compound is administered long-term or chronically.

In other embodiments, the present invention provides methods specific for the treatment of either EMs or PMs. In one embodiment, the present invention includes a method of treating or preventing arrhythmia in a mammal who is a cytochrome P450(CYP)2D6 poor metabolizer (PM), comprising identifying the mammal as a cytochrome P450(CYP)2D6 PM, and administering to the mammal a therapeutically effective amount of an ion channel modulating compound of the present invention. In a related embodiment, the present invention also includes a method of treating or preventing arrhythmia in a mammal who is a cytochrome P450(CYP)2D6 extensive metabolizer (EM), comprising identifying the mammal as a cytochrome P450(CYP)2D6 EM, and administering to the mammal a therapeutically effective amount of an ion channel modulating compound of the present invention.

In further embodiments, the present invention provides methods for identifying a patient to whom an ion channel modulating compound of the present invention is administered to treat or prevent any disease or disorder described herein (e.g., arrhythmia). In certain circumstances, such as long-term or chronic administration, it may be desired to only administer the ion channel modulating compounds to patients who are not PMs. Thus, according to one embodiment, the present invention provides a method of treating or preventing arrhythmia in a mammal, comprising determining if the mammal is a cytochrome P450(CYP)2D6 poor metabolizer (PM) or a cytochrome P450(CYP)2D6 extensive metabolizer (EM), and administering to the mammal a therapeutically effective amount of an ion channel modulating compound of the present invention if the mammal is a cytochrome P450(CYP)2D6 EM.

In a related embodiment, the present invention also includes a method of excluding a mammal from treatment with an ion channel modulating compound that is metabolized by cytochrome P450(CYP)2D6, comprising determining if the mammal is a cytochrome P450(CYP)2D6 poor metabolizer (PM), and not administering to the mammal an ion channel modulating compound that is metabolized by cytochrome P450(CYP)2D6 if the mammal is a PM. This method may be practiced to exclude PMs from treatment with ion channel modulating compounds according to methods of the present invention. In particular embodiments, a PM is excluded from long-term of chronic administration of an ion channel modulating compound, but is not excluded from short-term administration of an ion channel compound.

In another aspect, the present invention includes methods of increasing the bioavailability in a mammal of an ion channel modulating compound that is metabolized by cytochrome P450, comprising administering to said mammal the ion channel modulating compound in combination with an effective amount of cytochrome P450-inhibiting compound, including CYP2D6-inhibiting compounds. The two compounds maybe administered at the same or different times. A variety of cytochrome P450-inhibiting compounds are known in the art, and any of these or any undiscovered P450-inhibiting compound may be used according to the methods of the present invention. These compounds may be, *e.g.,* proteins, polypeptides, small molecules, polynucleotides (*e.g.,* single- or double-stranded), etc. Examples of specific P450-inhibiting compounds are provided in U.S. Patent Application Publication No. 20040224960. They may also include agents that target the gene or mRNA encoding P450, such as antisense RNA or siRNA molecules that specifically bind to the CYP2D6 gene or mRNA.

In related embodiments, methods of the present invention may further include the step of determining the blood plasma concentration or mean trough concentration of the ion channel modulating agent in a mammal to whom it has been administered, at one or more times following administration or during the course of administration. This step is useful to monitor the level of an ion channel modulating in order to determine or maintain an effective amount, such as any one of those concentrations described herein.

### 1. CYP2D6 Genotypes

The term "cytochrome P450," as used herein, refers to a family of enzymes found in mammals that modulate various physiological functions. In mammals, these enzymes are found throughout various tissues. About 30 of the enzymes in the cytochrome P450 family are found primarily in the endoplasmic reticulum of hepatocytes in the liver and small intestine, with smaller quantities found in the kidneys, lungs, and brain (E. L. Michalets, Review of Therapeutics, Update: Clinically significant cytochrome P450 drug interactions, Pharmacotherapy, 1998, 18(1), pp. 84-122; T. F. Woolf, Handbook of Drug Metabolism, Marcel Dekker, Inc., New York, 1999).

More than 200 cytochrome P450 genes which encodes products involved in phase I metabolism have been identified. There are multiple forms of these P450 genes, and each of the individual forms exhibit degrees of specificity towards individual chemicals in the above classes of compounds. In some cases, a substrate, whether it be drug or carcinogen, is metabolized by more than one of the cytochromes P450. Genetic polymorphisms of cytochromes P450 result in phenotypically-distinct subpopulations that differ in their ability to metabolize particular drugs and other chemical compounds. As those skilled in the art will understand, these phenotypic distinctions have important implications for selection of drugs for any given patient. For example, some individuals may have a defect in an enzyme required for detoxification of a particular drug, while some individuals may lack an enzyme required for conversion of the drug to a metabolically active form. Further, individuals lacking a biotransformation enzyme are often susceptible to cancers from environmental chemicals due to inability to detoxify the chemicals (*see* Eichelbaum et al., (1992) Toxicology Letters 64165: 155-122). Accordingly, it is advantageous to identify individuals who are deficient in a particular P450 enzyme.

Cytochrome P450 2D6 (or P45011 D6), also known as debrisoquine hydroxylase, is the best characterized polymorphic P450 in the human population (*see, e.g.,* Gonzalez et al. (1998) Nature 331:442-446). The cytochrome P450 2D6 gene represents a major Phase I drug metabolizing enzyme and is involved in the metabolism of numerous drugs. While CYP2D6 contributes only approximately 1.5% of the P450 protein present in human liver, it is responsible for approximately 24% of P450 drug metabolism activity (see, Wolf & Smith (2000) Brit Med Bull 55: 366-386; Lancet 584-586 (1977); Eur. J. Clin. Pharmacol. 16: 183-187 (1979); and Genomics 2: 174-179 (1988); Nature 331: 442-446 (1998).

Genetic variation of this gene locus results in various altered enzymatic activities of this gene with the majority of individuals possessing normal activity (extensive metabolizers; EMs), some individuals possessing slightly reduced activity (intermediate metabolizers) and some individuals with increased enzyme activity, in part due to gene duplications (rapid metabolizers). Individuals who lack enzyme activity, due to inactivating mutations in both copies of the CYP2D6 gene, are unable to metabolize drugs that require the CYP2D6 enzyme and are referred to as CYP2D6 poor metabolizers (PMs).

A number of mutations in the CYP2D6 gene that result in poor or intermediate metabolizer phenotypes, depending upon whether only one or both copies of the CYP2D6 gene are affected by mutation, have already been described (*see,* for example, U.S. Pat. No. 5,648,482; DNA 8:1-13 (1989); Biochemistry 27: 5447-5454 (1988); Proc. Natl. Acad. Sci. USA 85: 5240-5243 (1988) and U.S. Patent Application Publication No. 20030083485.). One PM phenotype has been reported which behaves as an autosomal recessive trait with an incidence between 5 and 10% in the white population of North America and Europe. PMs generally exhibit negligible amounts of cytochrome P450 2D6 activity. Genetic differences in cytochrome P450 2D6 may be associated with increased risk of developing environmental and occupational based diseases (*see*, Gonzalez & Gelboin (1993) Toxicology and Environmental Health 40: 289-308).

The CYP2D6 genotype of a patient, *e.g.,* whether the patient is an EM or a PM, may be determined by any of a variety of methods and assays known in the art. In particular embodiments, these methods involve examining the CYP2D6 enzymatic activity in a patient or a biological sample obtained from a patient, or they involve determining the presence of a polymorphism or genetic mutation associated with PM in a patient. In particular embodiments, patient metabolic profiles are assessed with a bioassay after a probe drug administration (see, for example, U.S. Pat. Nos. 5,891,696 and 5,989,844). For example, a poor drug metabolizer with a 2D6 defect is identified by administering one of the probe drugs, debrisoquine, sparteine or dextromethorphan, then testing urine for the ratio of unmodified to modified drug. PMs exhibit physiologic accumulation of unmodified drug and have a high metabolic ratio of probe drug to metabolite as compared to EMs.

The presence of a polymorphism or genetic mutation associated with PMs may be determined by genetic screening using basic molecular biological techniques, including, *e.g.,* real time polymerase chain reaction, gene sequencing, and primer extension. Certain CYP2D6 gene inactivating mutations have been identified (*see* Gough et al. (1990) Nature 347: 773-6; and Heim & Meyer (1990) Lancet 336: 529-32), and identifying the presence of any of these may be used to determined whether a patient is a PM. However, it is likely that many other CYP2D6 gene-inactivating polymorphic variations exist and screening for any of these is also contemplated. Genetic screening can also be performed by detecting either gene-inactivating mutations or closely linked polymorphisms found in association with such mutations.

The CYP2D6 genotype of a subject, *e.g.,* whether the subject is an EM or a PM, may also be determined by inquiring of the subject or another individual having such knowledge, or by referring to medical or other records.

### C. Methods of Preventing Arrhythmia Using Oral Formulations of Ion Channel Modulating Compounds

In certain embodiment, the present invention provides methods of preventing arrhythmia in subjects or patients (*e.g.,* mammals or warm-blooded animals, including humans and other animals) at risk for arrhythmia, by administering to such subjects an effective amount of a controlled release formulation of an ion channel modulating compound, such as, e.g. vernakalant hydrochloride. In particular embodiments, the methods of the invention are used to prevent or postpone the onset or recurrence of an arrhythmia. In one embodiment, the subject is a CYP2D6 extensive metabolizer.

Methods of the present invention may be used to prevent arrhythmia in a subject who previously underwent one or more arrhythmias, or in a subject at risk of an arrhythmia. For example, in one particular embodiment, methods of the present invention are used to prevent a post-operative arrhythmia (*e.g.,* following cardiac surgery such as CABG). In another embodiment, methods of the present invention are used to prevent the recurrence of arrhythmia, *i.e.,* a recurrent arrhythmia, in a subject having previously undergone one or more arrhythmias. In particular embodiments, the methods may also be used to treat or prevent sustained atrial fibrillation (atrial fibrillation of longer than 72 hours and less than 6 months duration) and chronic atrial fibrillation.

In particular embodiments, the ion channel modulating agent, *e.g.,* vernakalant hydrochloride, is provided to a subject orally. In certain embodiments, an effective orally administered (*i.e.,* oral) dosage of vernakalant hydrochloride for the prevention of an arrhythmia, (*e.g.,* over 90 days) is greater than 300 mg b.i.d., or greater than 600 mg per day. For example, an effective oral dosage of vernakalant hydrochloride may be in the range greater than 300 mg b.i.d. and up to 900 mg b.i.d. In other embodiments, it may be in the range greater than 300 mg b.i.d. and up to 600 b.i.d. In one embodiment, an effective oral dosage of vernakalant hydrochloride is about 500 mg b.i.d., about 600 mg b.i.d., about 700 mg b.i.d., about 800 mg b.i.d., or about 900 mg b.i.d. As shown in accompanying Examples, a dosage of about 500 mg b.i.d. significantly prevented the recurrence of AF over 90 days.

In particular embodiments, the ion channel modulating compound is administered long-term, chronically, or regularly, *e.g.,* to prevent arrhythmia in a mammal. Such long term or chronic administration may be, *e.g.,* at least 90 minutes, at least 2 hours, at least 3 hours, at least 4 hours, at least 8 hours, at least 12 hours, at least 24 hours, at least 48 hours, at least 3 days, at least 4 days, at least 5 days, at least 6 days, at least one week, at least 2 weeks, at least one month, at least 2 months, at least 4 months, at least 6 months, at least one year, at least 2 years, or greater than 2 years. In one embodiment, long-term treatment is characterized as administration for 3 days or longer, since this is the approximate time in which ion channel modulating compounds reach steady state plasma levels with twice daily oral dosing.

In related embodiments related to preventing a disease or disorder, e.g., an arrythmia, the ion channel modulating compound is administered for at least one week to one year, which may be, *e.g.,* following surgery or an arrhythmia. Such methods are particularly useful in preventing post-surgical arrhythmia or the recurrence or arrhythmia. In various embodiments, the ion channel modulating compound is administered to the mammal in two or more doses over the duration of administration. In certain embodiments, the ion channel modulating compound is administered orally using a controlled release formulation described herein or a unit dosage form described herein.

### D. Routes of Administration and Dosing

Ion channel modulating compounds may be administered according to the methods of the present invention in any therapeutically effective dosing regime. The dosage amount and frequency are selected to create an effective level of the agent without harmful effects. The therapeutically effective amount of a compound of the present invention will depend on the route of administration, the type of warm-blooded animal being treated, and the physical characteristics of the specific warm-blooded animal under consideration. These factors and their relationship to determining this amount are well known to skilled practitioners in the medical arts. This amount and the method of administration can be tailored to achieve optimal efficacy but will depend on such factors as weight, diet, concurrent medication and other factors which those skilled in the medical arts will recognize.

The methods of the present invention may be practiced by administering the ion channel modulating compound in one, two, or more doses. For example, in certain embodiments, an ion channel modulating compound is administered as a single dose, in repeated doses, or by continuous infusion over a period of time. In one embodiment, the ion channel modulating compound is administered over a long period of time, e.g., greater than 48 hours, or chronically. In another embodiment, the ion channel modulating compound is administered over a short period of time, e.g., less than 90 minutes.

In particular embodiments, the amount of ion channel modulating compound administered will generally range from a dosage of from about 0.1 to about 100 mg/kg/day, and typically from about 0.1 to 10 mg/kg where administered orally or intravenously for antiarrhythmic effect. In particular embodiments, a dosage is 5 mg/kg or 7.5 mg/kg. In various embodiments, the ion channel modulating compound is administered at a dosage of about 50-2500 mg per day, 100-2500 mg/day, 300-1800 mg/day, or 500-1800 mg/day. In one embodiment, the dosage is between about 100 to 600 mg/day. In another embodiment, the dosage is between about 300 and 1200 mg/day. In particular embodiments, the ion channel compound is administered at a dosage of 100 mg/day, 300 mg/day, 600 mg/day, 1000 mg/day, 1200 mg/day, or 1800 mg/day, in one or more doses per day (i.e., where the combined doses achieve the desired daily dosage). In related embodiments, a dosage is 100 mg bid, 150 mg bid, 300 mg bid, 500 mg bid, 600 mg bid, or 900 mg b.i.d. In particular embodiments, these dosages are administered orally to a subject at risk for arrhythmia, to prevent such arrhythmia. In particular embodiments, these dosages are administered to an EM patient. Examples of other suitable dosages and dosing regimes are described, e.g., in U.S. Patent Application Nos. 11/667,139, 11/832,580, 60/916,129, and 60/953,431. In particular embodiments, the ion channel modulating compound is administered in repeat dosing, and the initial dosage and subsequent dosages may be the same or different.

In certain embodiments, an ion channel modulating compound is administered for the treatment of a disease or disorder, e.g., arrhythmia, in a single dosage of 0.1 to 10 mg/kg or 0.5 to 5 mg/kg. In other embodiments, an ion channel modulating compound is administered to prevent a disease or disorder, e.g., arrhythmia including recurrence thereof, in a dosage of 0.1 to 50 mg/kg/day, 0.5 to 20 mg/kg/day, or 5 to 20 mg/kg/day.

In particular embodiments of repeat dosing, e.g., to treat arrhythmia, the first dosage is 3.0 to 5.0 mg/kg, and a second dosage is 0.5 to 2.0 mg/kg or 1.0 to 2.0 mg/kg. In related embodiments, a first dosage is 2.0 mg/kg, and a second dosage is 0.5 mg/kg. In another embodiment, a first dosage is 4 mg/kg, and a second dosage is 1.0 mg/kg. In another embodiment, a first dosage is 2 mg/kg, and a second dosage is 3.0 mg/kg. In a further embodiment, a first dosage is 0.5 mg/kg, and a second dosage is 1.0 mg/kg. In one embodiment, a first dosage is 3.0 mg/kg, and a second dosage is 2.0 mg/kg. In other embodiments of repeat dosing, the first and second dosages are between 0.1 to 10 mg/kg. For examples, the first dosage is 0.1 to 5 mg/kg, and the second dosage is 0.5 to 10 mg/kg; the first dosage is 1 to 5 mg/kg, and the second dosage is 1 to 5 mg/kg; the first dosage is 1 to 3 mg/kg, and the second dosage is 1 to 5 mg/kg; or the first dosage is 0.5 mg/kg followed by a second dosage of 1.0 mg/kg.

In certain embodiments, particularly when administered to treat an arrhythmia, the second dosage is not administered if the mammal to whom the compound is being administered converts to sinus rhythm after the first dosage. In certain embodiments, the ion channel modulating compound is administered orally or intravenously, *e.g.,* by infusion over a period of time of, *e.g.,* 10 minutes to 90 minutes.

In other related embodiments, an ion channel modulating compound is administered by continuous infusion, *e.g.,* at a dosage of between about 0.1 to about 10 mg/kg/hr over a time period. While the time period can vary, in certain embodiments the time period may be between about 10 minutes to about 24 hours or between about 10 minutes to about three days.

In particular embodiments, methods of the present invention involve administering to the mammal an amount of the ion channel modulating compound sufficient to achieve a total concentration of the ion channel modulating compound in the blood plasma of the subject with a Cₘₐₓ of between about 0.1 µg/ml and about 20 µg/ml, between about 0.3 µg/ml and about 15 µg/ml, or between about 0.3 µg/ml and about 20 µg/ml for some time. In certain embodiments, an oral dosage is an amount sufficient to achieve a blood plasma concentration (Cₘₐₓ) of between about 0.1 µg/ml to about 5 µg/ml or between about 0.3 µg/ml to about 3 µg/ml. In certain embodiments, an intravenous dosage is an amount sufficient to achieve a blood plasma concentration (Cₘₐₓ) of between about 1 µg/ml to about 10 µg/ml or between about 2 µg/ml and about 6 µg/ml.

In a related embodiment, the total concentration of the ion channel modulating compound in the blood plasma of the subject has a mean trough concentration of less than about 20 µg/ml, less than about 10ug/ml, less than about 1 ug/ml, less than about .5 ug/ml, less than about .2 ug/ml, or less than about 0.1 ug/ml and/or a steady state concentration of less than about 20 µg/ml, less than about 10ug/ml, less than about 1 ug/ml, less than about .5 ug/ml, less than about .2 ug/ml, or less than about 0.1 ug/ml. In one particular embodiment, the total concentration of the ion channel modulating compound in the blood plasma of the subject has a mean trough concentration of less than about 10 µg/ml and/or a steady state concentration of less than about 10 µg/ml.

In one embodiment, the total concentration of the ion channel modulating compound in the blood plasma of the subject has a mean trough concentration of between about 1 µg/ml and about 10 µg/ml, between about 100 ng/ml and about 1 µg/ml, or between about 0.3 µg/ml and about 3 µg/ml. In yet another embodiment, the total concentration of the ion channel modulating compound in the blood plasma of the subject has a mean trough concentration of between about 1 ng/ml and about 10 µg/ml and/or a steady state concentration of between about 1 ng/ml and about 10 µg/ml. In one embodiment, the total concentration of the ion channel modulating compound in the blood plasma of the subject has a mean trough concentration of between about 0.3 µg/ml and about 3 µg/ml and/or a steady state concentration of between about 0.3 µg/ml and about 3 µg/ml.

In particular embodiments, methods of the present invention involve administering to the mammal an amount of the ion channel modulating compound sufficient to achieve a blood plasma concentration described above for at least some time. In particular embodiments of the present invention, the effective amount of the ion channel modulating compound, the blood plasma concentration of the ion channel modulating compound, or the mean trough concentration of the ion channel modulating compound is achieved or maintained, *e.g.,* for at least 15 minutes, at least 30 minutes, at least 45 minutes, at least 60 minutes, at least 90 minutes, at least 2 hours, at least 3 hours, at least 4 hours, at least 8 hours, at least 12 hours, at least 24 hours, at least 48 hours, at least 3 days, at least 4 days, at least 5 days, at least 6 days, at least one week, at least 2 weeks, at least one month, at least 2 months, at least 4 months, at least 6 months, at least one year, at least 2 years, or greater than 2 years.

In related embodiment, methods of the present invention may further include the step of determining the blood plasma concentration or mean trough concentration of the ion channel modulating agent in a mammal to whom is has been administered at one or more times following administration or during the course of administration (*e.g.,* in PM patients). This step may be useful to monitor the level of an ion channel modulating in order to determine or maintain an effective amount, such as any one of those concentrations described herein.

In particular embodiments, the ion channel modulating compound is administered long-term, chronically, or regularly, *e.g.,* to prevent arrhythmia in a mammal. Such long term or chronic administration may be, *e.g.,* at least 90 minutes, at least 2 hours, at least 3 hours, at least 4 hours, at least 8 hours, at least 12 hours, at least 24 hours, at least 48 hours, at least 3 days, at least 4 days, at least 5 days, at least 6 days, at least one week, at least 2 weeks, at least one month, at least 2 months, at least 4 months, at least 6 months, at least one year, at least 2 years, or greater than 2 years. In one embodiment, long-term treatment is characterized as administration for 3 days or longer, since this is the approximate time in which ion channel modulating compounds reach steady state plasma levels with twice daily oral dosing.

In particular embodiments related to preventing a disease or disorder, *e.g.,* an arrythmia, the ion channel modulating compound is administered for at least one week to one year, which may be, *e.g.,* following surgery or an arrhythmia. Such methods are particularly useful in preventing post-surgical arrhythmia or the recurrence or arrhythmia. In various embodiments, the ion channel modulating compound is administered to the mammal in two or more doses over the duration of administration. In particular embodiments, the ion channel modulating compound is administered orally for long-term or chronic use.

As recognized by the present invention, the amount of ion channel modulating compound administered to achieve or maintain the above blood plasma levels or mean rough concentrations may be different when administered to a PM patient as compared to an EM patient, since the PM patient does not metabolize the ion channel modulating compound as rapidly as the EM patient. One of skill in the art will recognize that the effective dosage for PM patients will usually be no be different than the effective dose for EM patients when administered in a single bolus or a short-term treatment, such as those described for i.v. administration for cardioversion following arrhythmia. However, the effective dose for PM patients may be significantly lower for long-term or chronic treatment, *e.g.,* for the prevention of arrhythmia or recurrence or arrhythmia.

Thus, in certain embodiments of the present invention, a PM patient is administered an effective dosage or amount of ion channel modulating compound, *e.g.,* per day, that is significantly lower than the effective dosage or amount administered to an EM patient. In particular examples, the effective amount administered to a PM patient is less than 75%, less than 50%, or less than 25% of the effective amount administered to an EM patient. Generally, long-term or chronic administration is performed by oral administration. In particular embodiments, the effective dosage achieves the blood plasma levels or mean trough concentration of ion channel modulating compound described above. These may be readily determined using routine procedures, including those described herein and in U.S. Patent Application Serial No. 10/838,470.

In particular embodiments of the methods of the present invention, administration is by a route selected from the group consisting of: oral, topical, parenteral, sublingual, rectal, vaginal, and intranasal. In various embodiments, parenteral administration is subcutaneous injection, intravenous injection, intramuscular injection, epidural injection, intrasternal injection, or infusion. In various embodiments, the oral administration comprises administering an oral dosage form selected from a powder, a granule, a compressed tablet, a pill, a capsule, a cachet, a chewing gum, a wafer, or a lozenge. In certain embodiments of the methods related to preventing a disease or disorder, *e.g.,* arrhythmia, the ion channel modulating compound, *e.g.,* vernakalant hydrochloride, is provided orally to the subject, *e.g.,* in a tablet, such as a controlled release or extended release formulation.

### E. Controlled Release Formulations of Ion Channel Modulating Compounds

In certain embodiments, the present invention is directed to controlled release formulations, *e.g.,* tablets, comprising a therapeutically effective amount of ion channel modulating compound, or a pharmaceutically acceptable salt thereof, and one or more pharmaceutically acceptable excipients. In particular, this invention is directed to controlled release tablet formulations comprising a therapeutically effective amount of vernakalant hydrochloride and one or more pharmaceutically acceptable excipients suitable for controlled release formulations, which, upon oral administration thereto, are effective in preventing arrhythmia, *e.g.,* onset or recurrence of arrhythmia, in mammals, preferably in humans.

Accordingly, in certain embodiments, the controlled release tablet formulations of the invention are intended to be administered to a mammal, preferably a human, to prevent a disease or disorder, *e.g.,* an arrhythmia, including mammals at risk for arrhythmia or who have previously undergone one or more arrhythmias.

As used herein, and described in more detail below, a "pharmaceutically acceptable excipient" can be any pharmaceutically acceptable material, composition, or vehicle suitable for allowing the active ingredient to be released from the formulation in a controlled manner, including but not limited to, a liquid or solid filler, diluent, excipient, solvent or encapsulating material, which is involved in carrying or transporting the active ingredient to an organ, or portion of the body. Each pharmaceutically acceptable excipient must be compatible with the other ingredients of the formulation. Some examples of materials which can serve as pharmaceutically acceptable excipients include, but are not limited to, sugars, such as lactose, glucose and sucrose; starches, such as corn starch and potato starch; cellulose, and its derivatives, such as sodium carboxymethyl cellulose, ethyl cellulose and cellulose acetate; tragacanth; malt; gelatin; talc; cocoa butter, waxes, animal and vegetable fats, paraffins, silicones, bentonites, silicic acid, zinc oxide; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; and any other compatible substances routinely employed in pharmaceutical formulations and any substance identified herein as a pharmaceutically acceptable excipient.

As used herein, "controlled release" refers to the release of the active ingredient from the formulation in a sustained and regulated manner over a longer period of time than an immediate release formulation containing the same amount of the active ingredient would release during the same time period. For example, in certain embodiments, an immediate release formulation comprising vernakalant hydrochloride may release 80% of the active ingredient from the formulation within 15 minutes of administration to a human subject, whereas a controlled release formulation of the invention comprising the same amount of vernakalant hydrochloride would release 80% of the active ingredient within a period of time longer than 15 minutes, preferably within 6 to 12 hours. Controlled release formulations allows for less frequency of dosing to the mammal in need thereof. In addition, controlled release formulations may improve the pharmacokinetic or toxicity profile of the compound upon administration to the mammal in need thereof.

Exemplary excipients that are suitable for the controlled release formulations of the invention are listed in Tables 1 to 5 below, along with their chemical/ brand name, compendial status and function.

**Table 1: Excipients for a controlled release formulation utilizing a hydrophilic matrix system**

| **Excipient** | **Compendial Status** | **Function** |
|---|---|---|
| Avicel (Microcrystalline Cellulose) | USP/EP/JP | Filler/Diluent |
| Carbopol 971 P or Carbopol 71 G (Carbomer) | USP-NF | Hydrophilic matrix system polymer |
| Colloidal Silicon Dioxide | USP/EP/JP | Glidant |
| Emcompress (Calcium Phosphate Dibasic) | USP-NF | Filler/Diluent |
| Glucidex 9 (Maltodextrin) | USP-NF/EP | Hydrophilic matrix system polymer |
| Hydroxyethyl Cellulose | USP/EP/JP | Hydrophilic matrix system polymer |
| Hydroxypropyl Cellulose | USP/EP/JP | Hydrophilic matrix system polymer |
| Lactose Fast Flo (Lactose Monohydrate) | USP/EP/JP | Filler/Diluent |
| Magnesium Stearate (Non-Bovine) | USP/EP/JP | Lubricant |
| Methocel E4M Premium (Hydroxypropyl methyl cellulose, controlled release grade) | USA | Hydrophobic polymer |
| Methocel K4M (Hydroxypropyl methyl cellulose, controlled release grade) | USP | Hydrophilic matrix system polymer |
| Polyox WSR 1105 (Polyoxoacetate) | House | Hydrophilic matrix system polymer |
| Polyvinyl pyrrolidone | USP | Hydrophilic matrix system polymer/Binder |
| Prosolv SMCC 90 (Silicified microcrystalline cellulose) | USP-NF/EP | Filler/Glidant |
| Sodium carboxymethyl cellulose | USP | Hydrophobic polymer |
| Starch 1500 (Pregelatinized starch) | USP-NF | Binder for wet densification |
| Stearic Acid | USP/EP | Lubricant |

**Table 2: Excipients for a controlled release formulation utilizing a hydrophobic matrix system**

| **Excipient** | **Compendial Status** | **Function** |
|---|---|---|
| Avicel (Microcrystalline Cellulose) | USP/EP/JP | Filler/Diluent |
| Colloidal Silicon Dioxide | USP/EP/JP | Glidant |
| Emcompress (Calcium Phosphate Dibasic) | USP-NF | Filler/Diluent |
| Ethocel STD-4 (Ethyl Cellulose) | USP-NF | Hydrophobic matrix system polymer |
| Eudragit RSPO (Methacrylic acid copolymer) | USP-NF | Hydrophobic matrix system polymer |
| Eudragit S-100 (Methacrylic acid copolymer | USP-NF | Hydrophobic matrix system polymer r |
| Kollidon SR (Polyvinyl acetate pyrrolidone) | USP-NF | Hydrophobic matrix system polymer |
| Lactose Fast Flo (Lactose monohydrate) | USP/EP/JP | Filler/Diluent |
| Magnesium Stearate (Non-Bovine) | USP/EP/JP | Lubricant |
| Plasdone K29/32 (Povidone, polyvinyl pyrrolidone) | USP-NF | Binder |
| Polyethylene Glycol 8000 (Polyethylene glycol) | USP-NF | Hydrophobic matrix system polymer |
| Stearic Acid | USP/EP | Lubricant |

**Table 3: Excipients for a controlled release formulation utilizing a fat-wax system**

| **Excipient** | **Compendial Status** | **Function** |
|---|---|---|
| Avicel (Microcrystalline cellulose) | USP/EP/JP | Filler/Diluent |
| Colloidal Silicon Dioxide | USP/EP/JP | Glidant |
| Emcompress (Calcium phosphate dibasic) | USP-NF | Filler/Diluent |
| Kalcol 6098 (Cetyl alcohol) | USP | Erodable Retardant |
| Kalcol 6850P (Cetostearyl alcohol) | USN | Erodable Retardant |
| Lactose Fast Flo (Lactose Monohydrate) | USP/EP/JP | Filler/Diluent |
| Magnesium Stearate (Non-Bovine) | USP/EP/JP | Lubricant |
| Prosolv SMCC 90 (Silicified microcrystalline cellulose) | USP-NF/EP | Filler |
| Stearic Acid | USP/EP | Lubricant |

**Table 4: Excipients for a controlled release formulation utilizing a hydrophilic/hydrophobic matrix system**

| **Excipient** | **Compendial Status** | **Function** |
|---|---|---|
| Ethocel (Ethyl Cellulose) | USP-NF | Hydrophobic matrix system polymer |
| Eudragit RSPO (Methacrylic acid copolymer) | USP-NF | Hydrophobic matrix system polymer |
| Eudragit S-1 00 (Methacrylic acid copolymer) | USP-NF | Hydrophobic matrix system polymer |
| Kollidon SR (Polyvinyl acetate) | USP-NF | Hydrophobic matrix system polymer |
| Methocel E4M Premium (Hydroxypropyl methyl cellulose, controlled release grade) | USP | Hydrophobic matrix system polymer |
| Methocel K4M (Hydroxypropyl methyl cellulose, controlled release grade) | USP | Hydrophilic matrix system polymer |
| Polyvinyl pyrrolidone | USP | Hydrophilic matrix system polymer/Binder |
| Lactose Fast Flo (Lactose monohydrate) | USP/EP/JP | Filler/Diluent |
| Avicel (Microcrystalline cellulose) | USP/EP/JP | Filler/Diluent |
| Emcompress (Calcium Phosphate Dibasic) | USP-NF | Filler/Diluent |
| Colloidal Silicon Dioxide | USP/EP/JP | Glidant |
| Magnesium Stearate (Non-Bovine) | USP/EP/JP | Lubricant |
| Stearic Acid | USP/EP | Lubricant |

**Table 5: Excipients for a controlled release formulation utilizing a film-coated particulate system**

| **Excipient** | **Compendial Status** | **Function** |
|---|---|---|
| Ac-Di-Sol (Sodium crosscarmellose) | USP-NF | Disintegrant |
| Avicel (Microcrystalline Cellulose) | USP/EP/JP | Filler/Diluent |
| Colloidal Silicon Dioxide | USP/EP/JP | Glidant |
| Emcompress (Calcium Phosphate Dibasic) | USP-NF | Filler/Diluent |
| Explotab (Sodium Starch Glycolate) | USP-NF | Disintegrant |
| Lactose Fast Flo (Lactose monohydrate) | USP/EP/JP | Filler/Diluent |
| Magnesium Stearate (Non-Bovine) | USP/EP/JP | Lubricant |
| Plasdone K29/32 (Povidone, polyvinyl pyrrolidone) | USP | Binder |
| Starch 1500 (Pre-gelatinized Starch) | USP-NF | Glidant/Disintegrant |
| Stearic Acid | USP/EP | Lubricant |

The controlled release tablet formulations of the invention are formulated so that a final dosage form exhibits many desirable properties including, but not limited to, good tabletting characteristics (*e.g.,* good flow, compression, appearance, weight variation, hardness, friability, content uniformity and dissolution rate properties), good bioavailability profiles (*e.g.,* greater than 6 hours *in vivo* active ingredient release profile for a controlled release formulation of the invention), excellent stress and long-term stability, small tablet size, and simple, but efficient, and cost-effective manufacturing.

Controlled release tablet formulations of the invention may be made by incorporating the ion channel modulating compound, or its pharmaceutically effective salt, (collectively referred to herein as the "active ingredient"), preferably vernakalant hydrochloride, within a matrix system, including, but not limited to, a hydrophilic matrix system, a hydrophilic non-cellulose matrix system, a hydrophobic (plastic matrix system), or a hydrophilic/hydrophobic matrix system; within a fat-wax system; or within a film-coated particulate system.

Hydrophilic matrix systems show uniform and constant diffusion of the active ingredient from a tablet prepared with a hydrophilic, gelling polymer (*i.e.,* a hydrophilic matrix system polymer) after the tablet is placed in an aqueous environment. Release of the active ingredient from the system is controlled by a gel diffusional barrier which is formed by a process that is usually a combination of gel hydration, diffusion of the active ingredient, and gel erosion.

Hydrophobic (plastic) matrix systems utilize inert, insoluble polymers (*i.e.,* hydrophobic matrix system polymers) and copolymers to form a porous skeletal structure in which the active ingredient is embedded. Controlled release is effected by diffusion of the active ingredient through the capillary wetting channels and pores of the matrix, and by erosion of the matrix itself.

Hydrophilic/hydrophobic matrix systems utilize a combination of hydrophilic and hydrophobic polymers that forms a soluble/insoluble matrix in which the active ingredient is embedded. Controlled release of the active ingredient is by pore and gel diffusion as well as tablet matrix erosion. The hydrophilic polymer is expected to delay the rate of gel diffusion.

In fat-wax systems, the active ingredient is incorporated in a hot melt of a fat-wax (*i.e.,* erodable retardant matrix), solidified, sized and compressed with appropriate tablet excipients. Controlled release of the active ingredient is effected by pore diffusion and erosion of the fat-wax system. The addition of a surfactant as a wicking agent helps water penetration of the system to cause erosion.

Film-coated particulate systems include time-release granulations, prepared by extrusion-spheronization process or by conventional granulation process that have been film-coated to produce differing species of controlled release particles with specific active ingredient release characteristics. Controlled release particles may be compressed together with appropriate excipients to produce tablets with the desired controlled release profile. The release of the active ingredient is by particle erosion in either acid (gastric) or alkaline (intestinal) pH.

Immediate release tablet formulations comprising the active ingredient were prepared for comparison purposes only by compounding the active ingredient with appropriate excipients, including, but not limited to, immediate release fillers, binders, glidants, disintegrants and lubricants, to give satisfactory tabletting characteristics and subsequent rapid disintegration and dissolution of the tablets.

Controlled release tablet formulations of the invention may be manufactured by methods including, but not limited to, direct compression (dry blending the active ingredient with flowable excipients, followed by compression), wet granulation (application of a binder solution to powder blend, followed by drying, sizing, blending and compression), dry granulation or compaction (densifying the active ingredient or active ingredient/powder blend through slugging or a compactor to obtain flowable, compressible granules), fat-wax (hot melt) granulation (embedding the active ingredient in molten fatty alcohols, followed by cooling, sizing, blending and compression), and film-coating of particulates (dry blend, wet granulation, kneading, extrusion, spheronization, drying, film-coating, followed by blending of different species of film-coated spheres, and compression).

Of particular interest to the invention is the method of manufacturing controlled release tablet formulations of the invention such that each tablet comprise 100 mg, 250 mg, 300 mg, 500 mg, or 600 mg of the active ingredient. The methods for manufacturing these tablets include, but are not limited, to the following methods:
a. Direct compression.
b. Wet densification of the active ingredient and Starch 1500 or Povidone K29/32 with purified water, followed by tray drying to a moisture level of 2-3% w/w/ and blending with direct compression excipients.
c. Fat-wax (hot melt).

In one version of the direct compression method, the desired amount of the active ingredient and the desired amount of Starch 1500, Povidone K29/32, Lactose Fast Flo, Anhydrous Emcompress or Carbopol 71 G are mixed by hand in a small polyethylene (PE) bag or a 500 mL high density polyethylene (HDPE) container for approximately one minute and then passed through a #30 mesh screen. The resulting blend is then mixed with the desired amounts of the remaining excipients in the desired formulation, excluding magnesium stearate and stearic acid, for approximately 2 minutes in either a small PE bag or a 500 mL HDPE container. Approximately 1 g of the resulting mixture is then mixed with the desired amount of magnesium stearate and stearic acid, passed through a #30 mesh screen, added back to the remaining resulting mixture and then blended for approximately one minute. The resulting blend is then compressed into tablets at a final tablet weight of 225 mg or 300 mg (for tablets containing 100 mg active ingredient) or 630 mg or 675 mg (for tablets containing 300 mg active ingredient using a conventional bench top tablet press.

In another version of the direct compression method, the desired amount of the pre-screened (#40 mesh) active ingredient and Starch 1500 are placed in a 4 quart V-shell and blended at 25 rpm for 3 minutes. To the resulting blend is added the desired amounts of pre-screened (#30 mesh) Prosolv SMCC 90, Lactose Fast Flow, Methocel K4M and stearic acid (pre-screened through a #40 mesh) and the resulting mixture is mixed for 5 minutes at 25 rpm. Magnesium stearate is then added to an equal amount of the resulting mixture, which is then blended in a small polyethylene bag for approximately 1 minute, passed through a #30 mesh screen by hand and returned to the resulting mixture. The final resulting mixture is blended for 2 minutes at 25 rpm and then compressed into tablets at a final tablet weight of 630 mg or 675 mg (for tablets containing 300 mg active ingredient) using a conventional tablet press.

In a version of the wet densification method, the desired amount of the active ingredient is mixed with the desired amount of Starch 1500 or Povidone K29/32 and the resulting mixture is passed through a #30 mesh screen. Purified water is added to the screened mixture until it reaches a satisfactory densification end point. The resulting wet mass is passed through a #12 mesh screen onto a tray and dried at 60° C for 2 to 3 hours until a moisture level of 2-3% w/w is obtained. The resulting dry granules are passed through a #20 mesh screen into either a small PE bag or a 500 mL HDPE container. To the screened dry granules is added the desired amounts of the remaining excipients of the formulation, excluding magnesium stearate and stearic acid. The contents are mixed for approximately 2 minutes. Approximately 1 g of the resulting mixture is then mixed with the desired amounts of magnesium stearate and stearic acid, passed through a #30 mesh screen, added back to the remaining resulting mixture and then blended for approximately 1 minute. The final resulting blend is compressed into tables at a final tablet weight of 225 mg or 300 mg (for tablets containing 100 mg active ingredient) and 630 mg or 675 mg (for tablets containing 300 mg active ingredient) using a conventional tablet press.

In a version of the fat-wax (hot melt) method, the desired amount of fat-wax, preferably an erodable retardant selected from cetostearyl alcohol and cetyl alcohol, is placed in a stainless steel container, which is then heated on until the wax completely liquifies (i.e., completely melts). The desired amounts of the active ingredient, Lactose Fast Flo and Prosolv SMCC90 are then added to the melted wax with continuous stirring and heating until completely dispersed. Alternately, only the desired amount of the active ingredient is dispersed in the melted wax. The resulting granular-like particles are passed through a #20 mesh screen and placed in either a small PE bag or a 500 mL HDPE container. In the case of the melted wax containing only the active ingredient, the screened particles are blended with Lactose Fast Flo and Prosolv SMCC 90 for approximately 2 minutes in either a small PE bag or a 500 mL HDPE container. Approximately I g of each blend is mixed with the desired amounts of magnesium stearate and stearic acid, passed through a #30 mesh screen, returned to the blend, and mixed for approximately one minute. The final blend is compressed into tablets at weights of 225 mg (for tablets containing 100 mg active ingredient) and 630 mg or 675 mg (for tablets containing 100 mg active ingredient) using a conventional tablet press.

In another version of the fat-wax (hot melt) method, the desired amount of fat-wax, preferably, cetostearyl alcohol, is melted at approximately 70° C in a mixer until the wax liquifies. The desired amounts of Lactose Fast Flo and Prosolv SMCC90 are blended for approximately 1 minute in a double lined PE bag and set aside. The desired amount of the active ingredient is added to the melted wax with continuous stirring and heating at approximately 70°C until the active ingredient is completely dispersed. The blend of excipients is then added to the melted wax with stirring and maintaining heating between 40° C and 60° C until dispersion is complete. The resulting granular-like particles are cooled to ambient temperature, passed through a #20 mesh screen and placed in a double lined PE bag. The screened particles are then blended with stearic acid in a 4 quart V-shell for approximately 2 minutes at 25 rpm. Magnesium stearate is added to an equal amount of the stearic acid blend, blended in a small PE bag for approximately 1 minute, passed through a #20 mesh screen by hand, returned to the stearic acid blend and the final mixture is blended for 3 minutes at 25 rpm. The final blend was compressed into tablets at a weight of 630 mg or 675 mg (for tablets containing 300 mg of active ingredient) using a conventional tablet press.

In particular embodiments, the ion channel modulating compound is administered in one or more doses of a tablet formulation, typically for oral administration. The tablet formulation may be, e.g., a controlled release formulation. In particular embodiments, a tablet comprises 100, 150, 200, 250, 300, 500, or 600 mg of an ion channel modulating compound, such as vernakalant hydrochloride.

A variety of orally administered formulations of the ion channel modulating compounds of the present invention have been described, e.g., in U.S. Patent Application Nos. 11/832,580 and 60/953,431. Any of these formulations may be used according to the present invention. In one embodiment, a formulation comprises the ion channel modulating compound and at least one hydrophilic matrix system polymer selected from the group consisting of: carbomer, maltodextrin, hydroxyethyl cellulose, hydroxypropyl cellulose, hydroxypropyl methyl cellulose, and polyoxoacetate.

In certain embodiment, the ion channel modulating compound is administered in a controlled release tablet formulation comprising a therapeutically effective amount of ion channel modulating compound, or a pharmaceutically acceptable salt thereof and one or more pharmaceutically acceptable excipients. In one embodiment, the pharmaceutically acceptable excipient is a hydrophilic matrix system polymer selected from the group consisting of carbomer, maltodextrin, hydroxyethyl cellulose, hydroxypropyl cellulose, hydroxypropyl methyl cellulose, and polyoxoacetate.

In various embodiments, a tablet formulation comprises from about 20 mg to about 500 mg of ion channel modulating compound. In related embodiments, the tablet comprises about 50 mg to about 300 mg of ion channel modulating compound. In one embodiment, the tablet comprises about 100 mg to about 300 mg of the ion channel modulating compound. In particular embodiments, the tablet comprises about 100 mg, about 150 mg, about 200 mg, about 250 mg, or about 300 mg of the ion channel modulating compound, *e.g*., vernkalant hydrochloride. In other related embodiments, the tablet comprises about 500 or about 600 mg of the ion channel modulating compound.

In a particular embodiment, the controlled release tablet formulation comprises: about 300 mg of vernakalant hydrochloride; about 120 mg of hydroxypropyl methyl cellulose; about 30 mg preglatinized starch; about 90 mg silicified microcrystalline cellulose; about 81 mg of lactose monohydrate; about 4.5 mg stearic acid; and about 4.5 mg magnesium stearate.

In a particular embodiment, the controlled release tablet formulation comprises: about 250 mg of vernakalant hydrochloride; about 100 mg of hydroxypropyl methyl cellulose; about 25 mg preglatinized starch; about 75 mg silicified microcrystalline cellulose; about 67.5 mg of lactose monohydrate; about 3.75 mg stearic acid; and about 3.75 mg magnesium stearate.

In a particular embodiment, the controlled release tablet formulation comprises: about 500 mg of verakalant hydrochloride; about 200 mg of hydroxypropyl methyl cellulose; about 50 mg preglatinized starch; about 150 mg silicified microcrystalline cellulose; about 135 mg of lactose monohydrate; about 7.5 mg stearic acid; and about 7.5 mg magnesium stearate.

In a particular embodiment, the controlled release tablet formulation comprises: about 200 mg of verakalant hydrochloride; about 80 mg of hydroxypropyl methyl cellulose; about 20 mg preglatinized starch; about 60 mg silicified microcrystalline cellulose; about 54 mg of lactose monohydrate; about 3.0 mg stearic acid; and about 3.0 mg magnesium stearate.

In certain embodiments, the pharmaceutically acceptable excipient is an erodable retardant selected from the group consisting of cetyl alcohol or cetostearyl alcohol.

In one particular embodiment, the controlled release tablet formulation comprises: about 300 mg of vernakalant hydrochloride; about 150 mg cetostearyl alcohol; about 105 mg silicified microcrystalline cellulose; about 111 mg of lactose monohydrate; about 4.5 mg stearic acid; and about 4.5 mg magnesium stearate. In related embodiments, the controlled release tablet formulation comprises about 200 or about 250 mg of vernakalant hydrochloride and the other excipients in the same proportion as indicated for the 300 mg vernakalant hydrochloride formulation.

In certain embodiments, the ion channel modulating compound is administered in an extended release tablet formulation comprising a therapeutically effective amount of ion channel modulating compound, or a pharmaceutically acceptable salt thereof, and one or more pharmaceutically acceptable excipients, including, e.g., carbomer, hydroxyethyl cellulose, hydroxypropyl cellulose, or hydroxypropyl methyl cellulose.

In one particular embodiment, the extended release tablet formulation comprises: about 300 mg of vernakalant hydrochloride; about 323 mg of hydroxypropyl methyl cellulose; about 70 mg of dicalcium phosphate anhydrous; and about 7.0 mg magnesium stearate. In related embodiments, the controlled release tablet formulation comprises about 200 or about 250 mg of vernakalant hydrochloride and the other excipients in the same proportion as indicated for the 300 mg vernakalant hydrochloride formulation.

In particular embodiments, the present invention provides unit dosage forms of vernakalant hydrochloride comprising between 150 and 300 mg of vernakalant hydrochloride, including unit dosage forms comprising about 150 mg, about 200 mg, about 250 mg, about 300 mg, about 400 mg, about 500 mg, or about 600 mg of vernakalant hydrochloride.

Compositions described herein as "containing a compound of" formula (I), (II) or (III) encompass compositions that contain more than one compound of formula (I), (II) or (III).

When used to refer to a numerical value, the term "about" encompasses the indicated numerical value.

The following Examples are provided as a guide to assist in the practice of the invention, and are not intended as a limitation on the scope of the invention.

### EXAMPLE 1

### PHARMOCOKINETICS (PK), SAFETY, AND TOLERABILITY OF ORALLY-ADMINISTERED VERNAKALANT HYDROCHLORIDE

A phase I clinical study was conducted to demonstrate the PK, safety, and tolerability of vernakalant hydrochloride, orally-administered over 7 days of repeat dosing within an escalating dose regimen. The study examined these parameters in 40 healthy volunteers genotyped as CYP2D6 extensive metabolizers and 15 genotyped as CYP2D6 poor metabolizers.

Orally-administered vernakalant hydrochloride was found to be safe and well-tolerated across all dose levels. Dose proportional increases in plasma levels of vernakalant hydrochloride and its metabolites were seen with stead state plasma levels reached within 3-4 days. The maximum dose given for 7 days was 900 mg twice daily (1,800 mg-day), yielding blood levels of vernakalant hydrochloride approaching peak blood levels demonstrated to be effective for atrial fibrillation conversion by intravenous administration. The controlled release oral formulation provided sustained blood levels of drug over an interval deemed suitable for chronic-use oral therapy.

All treatment emergent adverse events were mild except for 2 subjects who experienced moderate itching and tingling of the skin. There were no serious adverse events. No clinically relevant changes were found in clinical laboratory, vital signs, or ECG measurements. Baseline QT interval (413 ± 19 ms) was unchanged (408 ± 25 ms) when measured at Cmax plasma levels after 7 days treatment with 900 mg vernakalant hydrochloride twice daily. Vernakalant hydrochloride appeared to be well-tolerated in subjects treated for 7 days at doses up to 900 mg twice daily.

### EXAMPLE 2

### ORAL ABSORPTION OF VERNAKALANT HYDROCHLORIDE

The oral bioavailability of vernakalant hydrochloride was demonstrated in a prospective, randomized, placebo-controlled, double-blind ascending dose assessment study in a total of 24 healthy human volunteer subjects. The first 8 subjects were fasted overnight and randomized to receive placebo (n=2) or 5 mg/kg p.o. of vernakalant hydrochloride (n=6). A second group of 8 fed subjects was assessed at the same dose (n=6) or placebo (n=2), and a third group of 8 subjects was fasted and randomized to receive placebo (n=2) or 7.5 mg/kg p.o. of vernakalant hydrochloride (n=6).

Vernakalant hydrochloride showed rapid and extensive absorption after a single oral dose in both fasted and fed subjects. The majority of subjects achieved maximal plasma levels (Cmax) within 30-60 min of dosing. The Cmax in fasted volunteers was 1.8+0.4 µg/ml after 5 mg/kg p.o. and 1.9±0.5 µg/ml after 7.5 mg/kg p.o. In fed volunteers, the Cmax was 1.3±0.7 µg/ml after 5 mg/kg p.o. Oral bioavailability calculated using previous i.v. data was 71±21%, 58±19%, and 69±50%, respectively, in the groups. There were no significant (ANOVA) differences in Cmax, Tmax, or bioavailability between groups.

All adverse events were transient and mild and only 5 were considered possibility related to drug (3 subjects). There were no significant changes in clinical laboratory tests, vital signs, ECG intervals, or any clinically significant findings in Holter recordings.

Vernakalant hydrochloride was rapidly and extensively absorbed to therapeutic plasma levels after oral administration in man, indicating that an oral formulation of vernakalant hydrochloride may be used for chronic oral prevention of arrhythmia, including atrial fibrillation.

### EXAMPLE 3

### ORAL VERNAKALANT HYDROCHLORIDE PREVENTS RECURRENCE OF ATRIAL FIBRILLATION FOLLOWING CARDIOVERSION

The safety, tolerability, and short term efficacy of vernakalant hydrochloride in human subjects with sustained atrial fibrillation (AF) was demonstrated in a randomized, double-blind, multi-center, placebo-controlled, dose-escalation study.

221 subjects with symptomatic AF (72 h - 6 month duration) were randomized to placebo or vernakalant hydrochloride for up to 28 days (stratified to background ACE-I or ARB use). In Tier 1, subjects received either 300 mg b.i.d. vernakalant hydrochloride or placebo, and in Tier 2, they received either 600 mg b.i.d. vernakalant hydrochloride or placebo. Dosing was initiated in hospital and cardioversion was performed on day 3, if required. A total of 171 subjects were successfully cardioverted and continued in the study with weekly follow-up visits. Efficacy was assessed as absence of AF recurrence on weekly 12-lead ECG and daily transtelephonic monitoring during the study.

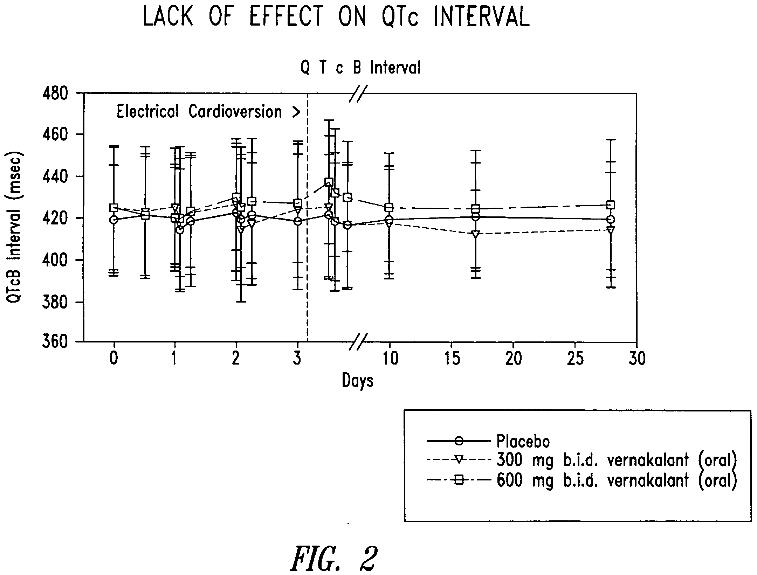

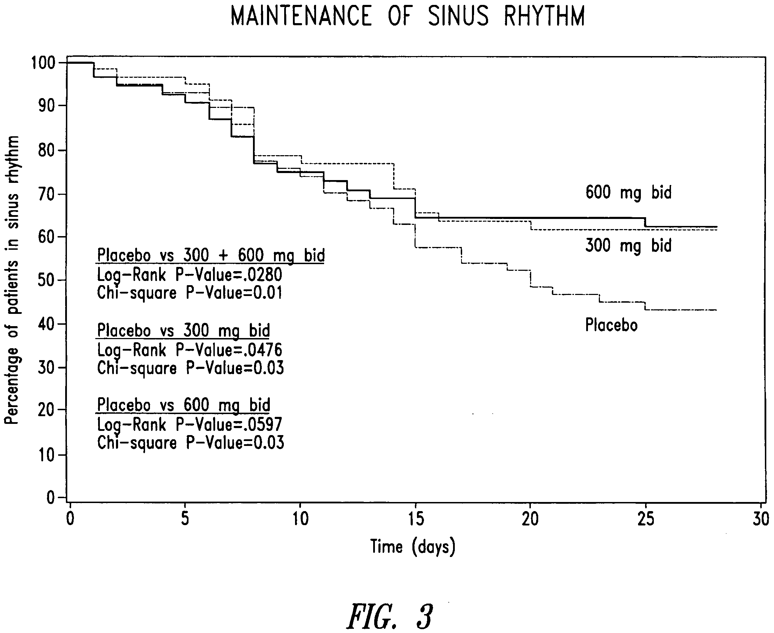

A total of 73 subjects received placebo and 71 and 75 subjects received vernakalant hydrochloride at 300 mg b.i.d. and 600 mg b.i.d., respectively. Demographics were generally comparable between groups. Overall, 63.3% of subjects were male, 47.5% were >65 years, 33.9% had a history of congestive heart failure, and 44.3% had 1 or more prior episodes of AF. The duration of the AF episode at entry was 73±48 days. Main concomitant medications used included ACE-I/ARBs (58%) and beta-blockers (78%). No effect on QTc interval was observed (Figure 2). In the placebo groups, 57% of subjects had a recurrence of AF as compared to 39% of subjects in the 300 mg b.i.d. vernakalant hydrochloride group (Log-rank Test p=0.048), and 39% of subjects in the 600 mg b.i.d. vernakalant hydrochloride group (Log-rank Test p=0.060) (Figure 3).

From the start of dosing to the end of the 30 day follow-up period, serious adverse events occurred in 8% of placebo subjects, 10% of 300 mg b.i.d. vernakalant hydrochloride subjects, and 11 % of 600 mg b.i.d. vernakalant hydrochloride subjects. The most commonly reported adverse events (>5% and at higher incidence in vernakalant hydrochloride subjects than placebo subjects) were bradycardia, sinus bradycardia, and 1^{st} degree AV block. One death due to MI, considered unrelated to drug, occurred. No Torsades de Pointes was observed.

This study demonstrated that vernakalant hydrochloride reduced the short term recurrence of AF.

### EXAMPLE 4

### ORAL VERNAKALANT HYDROCHLORIDE PREVENTS RECURRENCE OF ATRIAL FIBRILLATION OVER 90 DAYS

The safety, tolerability, pharmacokinetics, and preliminary efficacy of vernakalant hydrochloride (oral) over 90 days of dosing in patients with sustained symptomatic atrial fibrillation (AF; AF duration > 72 hours and < 6 months) was demonstrated in a randomized, double-blind, placebo-controlled, dose-ranging study. This study demonstrated statistically significant efficacy for the patient group receiving 500 mg b.i.d. of vernakalant hydrochloride (oral) as compared to placebo. The safety data from the interim analysis also suggested that vernakalant hydrochloride (oral) was well-tolerated in the AF patient population studied during this dosing period.

Vernakalant hydrochloride (oral) tablets were prepared from a blend of vernakalant hydrochloride drug substance with tablet excipients, including silicified microcrystalline cellulose, hydroxypropyl methyl ether cellulose (Hypromellose or HPMC), pregelatinized starch, lactose monohydrate, stearic acid, and magnesium stearate. Tablets were compressed as weight multiples from a common formula, to afford 150 mg, 200 mg, or 300 mg of vernakalant hydrochloride drug substance per tablet. For the purpose of blinding clinical trial materials, the tablets were encapsulated in opaque white gelatin capsule shells. Capsules containing the 150 mg and 200 mg tablets were backfilled with lactose monohydrate to approximate similar capsule weights across dose strengths. The composition of the vernakalant hydrochloride (oral) tablets is shown in Table 6. The composition of vernakalant hydrochloride (oral) encapsulated tablets is shown in Table 7.

**Table 6. Composition of Vernakalant Hydrochloride (Oral) Tablets**

| **Component** | **Reference to Quality Standard** | **Function** | **Amount per 150 mg tablet (mg)** | **Amount per 200 mg tablet (mg)** | **Amount per 300 mg tablet (mg)** |
|---|---|---|---|---|---|
| Vernakalant Hydrochloride | In-house standard | Drug Substance | 150.0† | 200.0† | 300.0† |
| Pregelatinized Starch | NF/Ph.Eur. | Filler | 15.0 | 20.0 | 30.0 |
| Silicified Microcrystalline Cellulose | In-house standard | Filler/Diluent | 45.0 | 60.0 | 90.0 |
| Lactose Monohydrate | NF/Ph.Eur. | Filler/Diluent | 40.5 | 54.0 | 81.0 |
| HPMC, K4M Premium CR Grade | USP/Ph.Eu r. | Hydrophilic matrix polymer | 60.0 | 80.0 | 120.0 |
| Stearic Acid | NF/Ph.Eur. | Lubricant | 2.25 | 3.0 | 4.5 |
| Magnesium Stearate (Non-Bovine) | NF/Ph.Eur. | Lubricant | 2.25 | 3.0 | 4.5 |
| **Total** | | | **315.0** | **420.0** | **630.0** |

| | | | | | |
|---|---|---|---|---|---|
| †Vernakalant hydrochloride is the monohydrochloride salt. The salt factor is 1.10. | | | | | |

**Table 7. Composition of Vernakalant Hydrochloride (Oral) Encapsulated Tablets**

| **Component** | **Reference to Quality Standard** | **Function** | **Amount per 150 mg tablet** | **Amount per 200 mg tablet** | **Amount per 300 mg tablet** |
|---|---|---|---|---|---|
| Vernakalant hydrochloride (oral) tablets (ea) | In-house standard | Drug Product | 1 | 1 | 1 |
| Lactose Monohydrate (mg) | NF/Ph.Eur. | Filler | 237.0 | 178.0 | NA‡ |
| White, opaque hard gelatin capsule shell (ea) | Pharmace utical Grade† | Blinding Agent | 1 | 1 | 1 |

| | | | | | |
|---|---|---|---|---|---|
| †A blend of pharmaceutical gelatins may used; when bovine gelatin is used, it is alkaline processed, pharmaceutical grade, and in full compliance with all pharmaceutical regulatory requirements. ‡ Used in 150 mg and 200 mg dosage strengths only. | | | | | |

Subjects with sustained AF were randomized to placebo or vernakalant hydrochloride for up to 90 days. Subjects treated with vernakalant hydrochloride received either 150 mg b.i.d, 300 mg b.i.d., or 500 mg b.i.d. After the first 3 days, patients still in atrial fibrillation were electrically cardioverted. Successfully cardioverted patients continued to receive vernakalant (oral) or placebo for the remainder of the 90-day trial and were monitored throughout the dosing period.

A Kaplan-Meier analysis of the 446 patients included in the study demonstrated a significant efficacy benefit for the 500 mg b.i.d. dosing group as compared to placebo (two-sided, p<0.05). Median time to recurrence of atrial fibrillation was greater than 90 days for the 500 mg b.i.d. dosing group, compared to 39 days for the placebo group. 52% of patients in the 500mg b.i.d. dosing group (n=110) completed the study in normal heart rhythm as compared to 39% of patients receiving placebo (n=118). The interim efficacy analysis for the 150 mg b.i.d. (n=110) and 300 mg b.i.d. (n=108) dosing groups had not achieved statistical significance at the 90 day timepoint.

The safety data for all dosing groups suggests that vernakalant (oral) was well-tolerated within the interim safety population (n=537), which includes patients randomized who did not enter the maintenance phase of the study. During the dosing period under analysis, there was no difference in the incidence of serious adverse events between treatment groups. Potentially drug-related serious adverse events occurred in 1% of placebo patients, 2% of patients in the 150 mg b.i.d. dosing group, 0% of patients in the 300 mg b.i.d. dosing group and 1% of patients in the 500 mg b.i.d. dosing group. There were no cases of drug-related "Torsades de Pointes", a well-characterized arrhythmia which is an occasional side effect of some current anti-arrhythmic drugs. There were 2 deaths during this period, both unrelated to vernakalant hydrochloride (oral), comprising a patient in the 150 mg b.i.d. dosing group who died of cervical cancer at day 79, and a patient in the placebo group who died at day 86 after suffering an ischemic stroke.

### EXAMPLE 5

### 100 MG CONTROLLED RELEASE TABLET FORMULATION HYDROPHILIC MATRIX SYSTEM

A controlled release tablet formulation of the invention comprising 100 mg of the active ingredient in a hydrophilic matrix system is made by the direct compression method. In this formulation, the active ingredient, vernakalant hydrochloride, is mixed with Starch 1500 in a small polyethylene bag or a 500 mL HDPE container for approximately one minute, then passed through a # 30 mesh screen. The screened mix is then transferred to its original polyethylene bag together with Prosolv SMCC 90, Lactose Fast Flo and Methocel K4M and mixed for 2 minutes. A portion (*e.g*., 1 g) of this blend is then mixed with magnesium stearate and stearic acid in a polyethylene bag, transferred back to the bulk blend via a #30 mesh screen and blended for 1 minute. Tablets may be compressed with a suitable punch. This formulation, hydrophilic formulation #100-1, is described in Table 8 below.

**Table 8: 100 mg Hydrophilic Formulation #100-1**

| **Ingredient** | **mg/tablet** | **%w/w** | **Wt.(g)** |
|---|---|---|---|
| Active ingredient | 100.00 | 33.33 | 5.00 |
| Starch 1500 | 15.00 | 5.00 | 0.75 |
| Prosolv SMCC 90 | 45.70 | 15.23 | 2.29 |
| Lactose Fast Flo | 91.30 | 30.43 | 4.57 |
| Methocel K4M | 45.00 | 15.00 | 2.25 |
| Stearic Acid | 1.50 | 0.50 | 0.08 |
| Magnesium stearate | 1.50 | 0.50 | 0.08 |
| Total Tablet Weight (mg) | 300.00 | 100.00 | 155.00 |

### EXAMPLE 6

### 100 MG CONTROLLED RELEASE TABLET FORMULATIONS HYDROPHILIC MATRIX SYSTEM

The following Table 9 provides for a controlled release tablet formulation of the invention comprising 100 mg of the active ingredient, vernakalant hydrochloride, in a hydrophilic matrix system. This formulation was prepared by the direct compression method disclosed herein using controlled-release grade Methocel K4M as the controlled release agent.

**Table 9: 100 mg Hydrophilic Formulation**

| | **Hydrophilic Formulation #100-2** |
|---|---|
| **Ingredients** | **mg/tablet** |
| Active Ingredient | 100.00 |
| Methocel K4M | 40.00 |
| Starch 1500 | 10.00 |
| Prosolv SMCC90 | 32.00 |
| Lactose Fast Flo | 40.00 |
| Stearic Acid | 1.50. |
| Magnesium Stearate (Non-Bov) | 1.50 |
| Total Tablet Weight (mg) | 225.00 |

Hydrophilic Formulation #100-2 was also prepared by the wet densification method described herein.

### EXAMPLE 7

### 300 MG CONTROLLED RELEASE TABLET FORMULATIONS HYDROPHILIC MATRIX SYSTEM

The following Table 10 provides for a controlled release tablet formulation of the invention comprising 300 mg of the active ingredient, vernakalant hydrochloride, in a hydrophilic matrix system. This formulation was prepared by compressing three times the weight of hydrophilic formulation # 100-3.

**Table 10: 300 mg Hydrophilic Formulations**

| | **Hydrophilic Formulation #300-1** |
|---|---|
| **Ingredients** | **mg/tablet** |
| Active Ingredient | 300.00 |
| Methocel K4M | 120.00 |
| Starch 1500 | 30.00 |
| Prosolv SMCC90 | 96.00 |
| Lactose Fast Flo | 120.00 |
| Stearic Acid | 4.50 |
| Magnesium Stearate (Non-Bov) | 4.50 |
| Total Tablet Weight (mg) | 675.00 |

### EXAMPLE 8

### 300 MG CONTROLLED RELEASE TABLET FORMULATIONS HYDROPHILIC MATRIX SYSTEM

The following Table 11 provides for a controlled release tablet formulation of the invention comprising 300 mg of the active ingredient, vernakalant hydrochloride, in a hydrophilic matrix system. Hydrophilic formulation #300-2 was prepared by reducing the calculated tablet weight of 675 mg of hydrophilic formulation #300-1 to 630 mg by reducing the amount of Lactose Fast Flo and Prosolv SMCC 90.

**Table 11: 300 mg Hydrophilic Formulation**

| | **Hydrophilic Formulation #300-2** |
|---|---|
| **Ingredients** | **mg/tablet** |
| Active Ingredient | 300.00 |
| Methocel K4M | 120.00 |
| Starch 1500 | 30.00 |
| Prosolv SMCC90 | 90.00 |
| Lactose Fast Flo | 81.00 |
| Stearic Acid | 4.50 |
| Magnesium Stearate (Non-Bov) | 4.50 |
| Total Tablet Weight (mg) | 630.00 |

### EXAMPLE 9

### 100 MG CONTROLLED RELEASE TABLET FORMULATIONS HYDROPHILIC (NON-CELLULOSE) MATRIX SYSTEM

The following Table 12 provides for three controlled release tablet formulations of the invention comprising 100 mg of the active ingredient, vernakalant hydrochloride, in a hydrophilic (non-cellulose) matrix system. Hydrophilic (non-cellulose) formulations #100-1 and #100-3 were prepared by the direct compression method. Hydrophilic (non-cellulose) formulation #100-2 was prepared by the wet densification method described herein wherein the active ingredient and starch is mixed with water followed by drying and blending with the direct compression excipients. All three formulations had tablet weights of 225 mg.

**Table 12: 100 mg Hydrophilic (non-cellulose) Formulations**

| | **Hydrophilic (Non-Cellulose) Formulation #100-1** | **Hydrophilic (Non-Cellulose) Formulation #100-2** | **Hydrophilic (Non-Cellulose) Formulation #100-3** |
|---|---|---|---|
| **Ingredients** | **mg/tablet** | **mg/tablet** | **mg/tablet** |
| Active Ingredient | 100.00 | 100.00 | 100.00 |
| Starch 1500 | 1500 | 10.00 | 10.00 |
| Prosolv SMCC90. | 33.75 | 32.00 | 39.50 |
| Lactose Fast Flo | - | 46.25 | 50.00 |
| Polyethylene Glycol | 45.00 | - | - |
| Carbopol 971 P | - | 33.75 | - |
| Polyox WSR 1105 | - | - | 22.50 |
| Anhydrous Emcompress | 27.50 | - | - |
| Eudragit RS PO | 15.75 | - | - |
| Stearic Acid | 1.50 | 1.50 | 1.50 |
| Magnesium Stearate (Non-Bovine) | 1.50 | 1.50 | 1.50 |
| Total Tablet Weight (mg) | 225.00 | 225.00 | 225.00 |

Hydrophilic (non-cellulose) formulation #100-2 was also prepared by the direct compression method described herein.

### EXAMPLE 10

### 300 MG CONTROLLED RELEASE TABLET FORMULATIONS HYDROPHILIC (NON-CELLULOSE) MATRIX SYSTEM

The following Table 13 provides for a controlled release tablet formulation of the invention comprising 300 mg of the active ingredient, vernakalant hydrochloride, in a hydrophilic (non-cellulose) matrix system. Hydrophilic (non-cellulose) formulation #300-1 was prepared by compressing three times the calculated weight of hydrophilic (non-cellulose) formulation #100-2 after reducing the calculated final tablet weight of 675 mg to 630 mg by reducing the amounts of Prosolv SMCC 90, Lactose Fast Flo and Carbopol 71 G present in the final formulation.

**Table 13: 300 mg Hydrophilic (non-cellulose) Formulation**

| | **Hydrophilic (Non-Cellulose) Formulation #300-1** |
|---|---|
| **Ingredients** | **mg/tablet** |
| Active Ingredient | 300.00 |
| Starch 1500 | 30.00 |
| Prosolv SMCC90 | 90.00 |
| Lactose Fast Flo | 105.00 |
| Carbopol 971 P | 96.00 |
| Stearic Acid | 4.50 |
| Magnesium Stearate (Non-Bovine) | 4.50 |
| Total Tablet Weight (mg) | 630.00 |

### EXAMPLE 11

### 100 MG CONTROLLED RELEASE FORMULATIONS HYDROPHOBIC MATRIX SYSTEM AND FAT-WAX SYSTEM

The following Table 14 provides for controlled release tablet formulations of the invention comprising 100 mg of the active ingredient, vernakalant hydrochloride, in a hydrophobic matrix system or in a fat-wax (hotmelt) system. These formulations were prepared by the methods disclosed herein. The hydrophilic matrix system formulation prepared in Example 1 is shown for comparison purposes only.

**Table 14: 100 mg Controlled Release Formulations**

| | **Hydrophobic Formulation #100-1**** | **Fat-wax Formulation #100-1**** | **Hydrophilic Formulation #100-1*** |
|---|---|---|---|
| **Ingredients** | **%w/w** | **%w/w** | **%w/w** |
| Active Ingredient | 44.44 | 44.44 | 33.33 |
| Methocel K4M | - | - | 15.00 |
| Cetyl Alcohol | - | 22.22 | - |
| Ethylcellulose | 8.80 | - | - |
| Kollidon SR | 31.11 | - | - |
| Starch 1500 | - | - | 5.00 |
| Prosolv SMCC90 | - | 15.56 | 15.23 |
| Lactose | 14.22 | 16.44 | 30.43 |
| Stearic Acid | 0.67 | 0.67 | 0.50 |
| Magnesium Stearate (Non-Bov) | 0.67 | 0.67 | 0.50 |
| Total | 100.00 | 100.00 | 100.00 |

| | | | |
|---|---|---|---|
| * Tablet weight: 300 mg ** Tablet weight: 225 mg | | | |

### EXAMPLE 12

### 100 MG CONTROLLED RELEASE FORMULATIONS HYDROPHOBIC MATRIX SYSTEM

The following Table 15 provides for controlled release tablet formulations of the invention comprising 100 mg of the active ingredient, vernakalant hydrochloride, in a hydrophobic matrix system. Hydrophobic formulation #100-2 and hydrophobic formulation #100-3 were prepared using Kollidon SR and Ethylcellulose Standard 4 as the controlled release agents. Hydrophobic formulation #100-4 was prepared using Kollidon SR and Eudragit RS PO as the controlled release polymers. All three formulations were processed by the direct compression method disclosed herein.

**Table 15: 100 mg Hydrophobic Formulations**

| | **Hydrophobic Formulation #100-2** | **Hydrophobic Formulation #100-3** | **Hydrophobic Formulation #100-4** |
|---|---|---|---|
| **Ingredients** | **mg/tablet** | **mg/tablet** | **mq/tablet** |
| Active Ingredient | 100.00 | 100.00 | 100.00 |
| Ethylcellulose | 20.00 | 20.00 | - |
| Kollidon SR | 70.00 | 70.00 | 45.00 |
| Starch 1500 | 32.00 | 32.00 | - |
| Anhydrous Emcompress | - | - | 32.00 |
| Eudragit RS PO | - | - | 45.00 |
| Prosolv SMCC90 | - | 15.56 | - |
| Stearic Acid | 1.50 | 1.50 | 1.50 |
| Magnesium Stearate | 1.50 | 1.50 | 1.50 |
| Total Tablet Weight (mg) | 225.00 | 225.00 | 225.00 |

The following Table 16 provides for a controlled release tablet formulation of the invention comprising 100 mg of the active ingredient, vernakalant hydrochloride, in a hydrophobic matrix system. This formulation, hydrophobic formulation #100-5, was prepared by the wet densification method.

**Table 16: 100 mg Hydrophobic Formulation**

| | **Hydrophobic Formulation #100-5** |
|---|---|
| **Ingredients** | **mg/tablet** |
| Active Ingredient | 100.00 |
| Povidone K 29/32 | 11.00 |
| Kollidon SR | 60.00 |
| Anhydrous Emcompress | 31.00 |
| Eudragit RS PO | 20.00 |
| Stearic Acid | 1.50 |
| Magnesium Stearate | 1.50 |
| Total Tablet Weight (mg) | 225.00 |

### EXAMPLE 13

### 100 MG CONTROLLED RELEASE FORMULATIONS HYDROPHILIC/HYDROPHOBIC MATRIX SYSTEM

The following Table 17 provides for a controlled release tablet formulation of the invention comprising 100 mg of the active ingredient, vernakalant hydrochloride, in a hydrophilic/hydrophobic matrix system. Hydrophilic/hydrophobic formulation #100-1 was prepared using Maltodextrin as the hydrophobic agent and Carbopol 71 G as the hydrophilic controlled release agent. The formulation was prepared using the direct compression method disclosed herein.

**Table 17: 100 mg Hydrophilic/Hydrophobic Formulation**

| | **Hydrophilic/Hydrophobic Formulation #100-1** |
|---|---|
| **Ingredients** | **mg/tablet** |
| Active Ingredients | 100.00 |
| Lactose Fast Flo | 39.00 |
| Carbopol 71G | 11.00 |
| Anhydrous Emcompress | 39.50 |
| Povidone K29/32 | 10.00 |
| Maltodextrin | 22.50 |
| Stearic Acid | 1.50 |
| Magnesium Stearate | 1.50 |
| Total Tablet Weight (mg) | 225.00 |

The following Table 18 provides for a controlled release tablet formulation of the invention comprising 100 mg of the active ingredient, vernakalant hydrochloride, in a hydrophilic/hydrophobic matrix system. Hydrophilic/hydrophobic formulation #100-2 was prepared using the wet densification method disclosed herein and Kollidon SR and Eudragit RS PO as its principal hydrophobic controlled release agent and Methocel K4M as its hydrophilic controlled release agent.

**Table 18: 100 mg Hydrophilic/Hydrophobic Formulation**

| | **Hydrophilic/Hydrophobic Formulation #100-2** |
|---|---|
| **Ingredients** | **mg/tablet** |
| Active Ingredient | 100.00 |
| Methocel K4M | 11.00 |
| Kollidon SR | 50.00 |
| Anhydrous Emcompress | 30.00 |
| Povidone K29/32 | 11.00 |
| Eudragit RS PO | 20.00 |
| Stearic Acid | 1.50 |
| Magnesium Stearate | 1.50 |
| Total Tablet Weight (mg) | 225.00 |

### EXAMPLE 14

### 300 MG CONTROLLED RELEASE FORMULATIONS HYDROPHILIC/HYDROPHOBIC MATRIX SYSTEM

The following Table 19 provides for a controlled release tablet formulation of the invention comprising 300 mg of the active ingredient, vernakalant hydrochloride, in a hydrophilic/hydrophobic matrix system. Hydrophilic/hydrophobic formulation #300-1 was prepared by compressing three times the weight of hydrophilic/hydrophobic formulation #100-2 (the calculated final tablet weight of 675 mg was reduced to 630 mg by reducing and adjusting the amounts of Methocel K4M, Povidone K29/32, Kollidon SR, Eudragit RS PO and Anhydrous Emcompress present in the formulation).

**Table 19: 300 mg Hydrophilic/Hydrophobic Formulation**

| | **Hydrophilic/Hydrophobic Formulation #300-1** |
|---|---|
| **Ingredients** | **mg/tablet** |
| Active Ingredient | 300.00 |
| Methocel K4M | 30.00 |
| Kollidon SR | 135.00 |
| Anhydrous Emcompress | 81.00 |
| Povidone K29/32 | 30.00 |
| Eudragit RS PO | 45.00 |
| Stearic Acid | 4.50 |
| Magnesium Stearate | 4.50 |
| Total Tablet Weight (mg) | 630.00 |

### EXAMPLE 15

### 100 MG CONTROLLED RELEASE FORMULATIONS FAT-WAX SYSTEM

The following Table 20 provides for three controlled release tablet formulations of the invention comprising 100 mg of the active ingredient, vernakalant hydrochloride, in a fat-wax system. All three formulations were prepared by the fat-wax method disclosed herein where all the ingredients, except magnesium stearate and stearic acid, were dispersed in the wax, *i.e*., cetostearyl alcohol.

**Table 20: 100 mg Fat-Wax Formulations**

| | **Fat-wax Formulation #100-2** | **Fat-wax Formulation #100-3** | **Fat-wax Formulation #100-4** |
|---|---|---|---|
| **Ingredients** | **mq/tablet** | **mg/tablet** | **mg/tablet** |
| Active Ingredient | 100.00 | 100.00 | 100.00 |
| Prosolv SMCC 90 | 50.00 | 35.00 | 35.00 |
| Lactose Fast Flo | 37.00 | 37.00 | 37.00 |
| Cetostearyl Alcohol | 35.00 | 50.00 | 50.00 |
| Stearic Acid | 1.50 | 1.50 | 1.50 |
| Magnesium Stearate | 1.50 | 1.50 | 1.50 |
| Total Tablet Weight (mg) | 225.00 | 225.00 | 225.00 |

Fat-wax formulation #100-3 and #100-4 were also prepared by the fat-wax method described herein wherein only the active ingredient is dispersed in the fat-wax.

The following Table 21 provides for a controlled release tablet formulation of the invention comprising 100 mg of the active ingredient, vernakalant hydrochloride, in a fat-wax system. The formulation was prepared by the fat-wax method wherein only the active ingredient is dispersed in the fat-wax, i.e., cetyl alcohol.

**Table 21: 100 mg Fat-Wax Formulations**

| | **Fat-wax Formulation #100-5** |
|---|---|
| **Ingredients** | **mg/tablet** |
| Active Ingredient | 100.00 |
| Prosolv SMCC 90 | 29.75 |
| Lactose Fast Flo | 36.00 |
| Cetyl Alcohol | 56.25 |
| Stearic Acid | 1.50 |
| Magnesium Stearate | 1.50 |
| Total Tablet Weight (mg) | 225.00 |

### EXAMPLE 16

### 300 MG CONTROLLED RELEASE FORMULATIONS FAT-WAX SYSTEM

The following Table 22 provides for a controlled release tablet formulation of the invention comprising 300 mg of the active ingredient, vernakalant hydrochloride, in a fat-wax system. This formulation was prepared by methods disclosed herein.

**Table 22: 300 mg Fat-Wax Formulations**

| | **Fat-wax Formulation #300-1** |
|---|---|
| **ingredients** | **mg/tablet** |
| Active Ingredient | 300.00 |
| Prosolv SMCC 90 | 105.00 |
| Lactose Fast Flo | 111.00 |
| Cetostearyl Alcohol | 150.00 |
| Stearic Acid | 4.50 |
| Magnesium Stearate | 4.50 |
| Total -Tablet Weight (mg) | 675.00 |

### EXAMPLE 17

### 100 MG IMMEDIATE RELEASE FORMULATION

An immediate release tablet formulation comprising 100 mg of the active ingredient, vernakalant hydrochloride, was prepared for comparison purposes only by the direct compression method disclosed herein. The formulation was blended in small PE bags and subsequently compressed manually on a single punch bench tablet press with an appropriate tablet punch. The active ingredient was mixed with Starch 1500 in a small PE bag and then passed through a # 30 mesh screen. The screened mix was then transferred to its original polyethylene bag along with Prosolv SMCC90, Lactose Fast Flo and Explotab and mixed for 2 minutes. A portion (e.g., 1 g) of this blend was then mixed with magnesium stearate and stearic acid in a PE bag, transferred back to the bulk blend via a #30 mesh screen and blended for 1 minute. Tablets were compressed with a suitable punch on a single punch press to obtain a tablet hardness of 7-12 KN. The formulation is described in Table 23 below.

**Table 23: 100 mg Immediate release Tablet Formulation**

| **Ingredient** | **mg/tab** | **%w/w** | **Wt.(g)** |
|---|---|---|---|
| Ion Channel Modulating Compound | 100.00 | 33.33 | 5.00 |

| **Ingredient** | **mg/tab** | **% w/w** | **wt.(g)** |
|---|---|---|---|
| Explotab (Sodium Starch Glycolate) | 3.00 | 1.00 | 0.15 |
| Lactose Fast Flo | 117.50 | 39.17 | 5.88 |
| Magnesium Stearate | 1.50 | 0.50 | 0.08 |
| Prosolv SMCC90 | 60.00 | 20.00 | 3.00 |
| Starch 1500 | 15.00 | 5.00 | 0.75 |
| Stearic Acid | 3.00 | 1.00 | 0.15 |
| Total Tablet Weight | 300.00 | 100.00 | 15.00 |

### EXAMPLE 18

### IN-VITRO DISSOLUTION OF CONTROLLED RELEASE TABLET FORMULATIONS OF THE INVENTION

The *in vitro* release profile of the formulations of the invention may be empirically determined by examining the dissolution of the tablet formulations over time. A USP approved method for dissolution or release test can be used to measure the rate of release in vitro (USP 24; NF 19 (2000) pp. 1941-1951). For example, a weighed tablet containing the active ingredient is added to a measured volume of a solution containing 0.9% NaCl in water, where the solution volume will be such that the active ingredient concentration after release is less than 20% of saturation. The mixture is maintained at 37°C and stirred or shaken slowly to maintain the tablet in suspension. The release of the dissolved active ingredient as a function of time may then be followed by various methods known in the art, such as spectrophotometrically, HPLC, mass spectroscopy, and the like, until the solution concentration becomes constant or until greater than 90% of the active ingredient has been released.

This Example is provided as a guide to assist in the practice of the invention, and is not intended as a limitation on the scope of the invention.

The following Table 24 provides the mean dissolution release percentages of selected controlled tablet formulations of the invention comprising 100 mg of the active ingredient. The dissolved percentages indicated are mean values based on the number of tablets tested for each formulation.

**Table 24: Mean dissolution % Release of 100 mg controlled release formulations**

| | **Formulations** | | | | | |
|---|---|---|---|---|---|---|
| | Hydrophilic #100-2 | Hydrophilic (Non-Celluose) #100-2 | Hydrophobic #100-5 | Fat-wax #100-4 | Fat-wax #100-5 | Hydrophilic / Hydrophobic #100-2 |
| % Dissolved after 0.5 hrs | 23 | 22 | 18 | 29 | 30 | 16 |
| % Dissolved after 1 hour | 32 | 29 | 29 | 42 | 41 | 24 |
| % Dissolved after 2 hours | 45 | 39 | 45 | 58 | 57 | 36 |
| % Dissolved after 4 hours | 61 | 54 | 67 | 78 | 85 | 53 |
| % Dissolved after 6 hours | 71 | 67 | 82 | 91 | 95 | 66 |
| % Dissolved after 8 hours | 78 | 78 | 88 | 98 | 97 | 74 |
| % Dissolved after 10 hrs | 83 | 87 | 92 | 102 | 98 | 81 |
| % Dissolved after 12 hrs | 86 | 93 | 94 | 103 | 99 | 85 |
| # of tablets tested | 6 | 6 | 6 | 6 | 3 | 6 |
| Active Ingredient at 12 hours (%) | 22.6 | 1.6 | 16.7 | 7.6 | 1.6 | 12.7 |

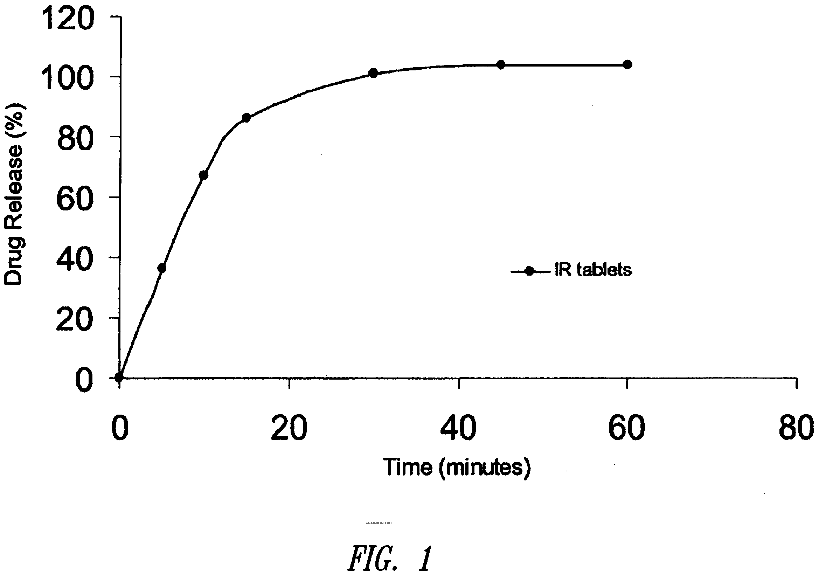

For comparison purposes only, Figure 1 shows a release profile (percent cumulative release over time) for the immediate release tablet formulation comprising 100 mg of the active ingredient (as described above in Example 13). More than 80% of the active ingredient in the immediate release tablet formulation dissolved by fifteen minutes.

The following Table 25 provides the dissolution release percentages of selected controlled tablet formulations of the invention comprising 300 mg of the active ingredient.

**Table 25: Mean dissolution % Release of 300 mg Controlled Release Formulations**

| | **Formulations** | | | |
|---|---|---|---|---|
| | **Hydrophilic #300-2** | **Hydrophilic (Non-Celluose) #300-1** | **Fat-wax #300-1** | **Hydrophilic/ Hydrophobic #300-1** |
| % Dissolved after 0.5 hrs | 18 | 16 | 30 | 26 |
| % Dissolved after 1 hour | 27 | 21 | 41 | 36 |
| % Dissolved after 2 hours | 41 | 28 | 57 | 47 |
| % Dissolved after 4 hours | 61 | 41 | 75 | 63 |
| % Dissolved after 6 hours | 75 | 53 | 87 | 74 |
| % Dissolved after 8 hours | 85 | 66 | 95 | 82 |
| % Dissolved after 10 hours | 92 | 78 | 99 | 89 |
| % Dissolved after 12 hours | 97 | 91 | 101 | 94 |

### EXAMPLE 19

### IN VIVO PHARMACOKINETIC PROFLILES OF FORMULATIONS OF THE INVENTION

The *in vivo* pharmacokinetic profiles of the formulations of the invention were determined as follows. Formulations of the invention were administered to dogs in a controlled experiment to determine pharmacokinetic profile of each formulation tested. A single controlled release tablet formulation of the invention was orally administered to each group of dogs. Blood samples were collected via the jugular or cephalic vein at predose (0), 15, 30, 60, 90, 120, 240, 360, 480, 600 and 1440 minutes after administration or at predose (0), 30, 60, 90, 120, 240, 360, 480, 600, 720 and 1440 minutes after administration.

Concentration levels of the active ingredient in the plasma samples at each timepoint were determined using standard methods known to one skilled in the art. The concentration levels were plotted on a standard pharmacokinetic curve (time in minutes versus concentration in ng/mL) and the area under the curve extrapolated to infinity (AUC_{inf}), the Cₘₐₓ (peak blood plasma concentration level of the active ingredient) and Tₘₐₓ (time after administration of the formulation when peak plasma concentration level occurs) were calculated. In general, a controlled release formulation should provide a broader pharmacokinetic curve while minimizing the Cₘₐₓ when compared to the pharmacokinetic curve of an immediate release formulation. In other words, a large ratio of AUC_{inf}/Cₘₐₓ is desired for each controlled release formulation of the invention. As noted below in the Examples, the controlled release tablet formulations of the invention demonstrated the ability to release the active ingredient into the blood over a longer period of time than the corresponding immediate release formulation.

The following Table 26 presents the plasma concentrations of the active ingredient, vernakalant hydrochloride, in dogs that received the immediate release tablet formulation comprising 100 mg of the active ingredient, vernakalant hydrochloride, as set forth above in Example 17; a hydrophilic controlled release tablet formulation of the invention comprising 100 mg of the active ingredient, (*i.e*., hydrophilic formulation #100-2); a hydrophilic controlled release tablet formulation of the invention comprising 300 mg of the active ingredient (*i.e*., hydrophilic formulation #300-1); a fat-wax controlled release tablet formulation of the invention comprising 100 mg of the active ingredient (*i.e*., fat-wax formulation #100-3); and a hydrophobic controlled release tablet formulation of the invention comprising 100 mg of the active ingredient (*i.e*., hydrophobic formulation #100-3). Concentrations are given as µg/mL and are an average obtained from n = 3 dogs unless otherwise indicated.

**Table 26: Plasma concentrations of active ingredient in dogs after oral administration of various formulations of the invention**

| | **Formulations** | | | | |
|---|---|---|---|---|---|
| **Time (min)** | **Immediate Release** | **Hydrophilic #100-2** | **Hydrophilic #300-1** | **Fat-wax #100-3** | **Hydrophobic #100-3** |
| 0 | ND | ND | ND | ND | ND |
| 15 | 0.398* | ND | 0.078 | 0.224* | 0.072* |
| 30 | 0.770* | 0.047** | 0.152 | 0.445 | 0.135 |
| 60 | 0.522* | 0.077 | 0.194 | 0.596 | 0.201 |
| 90 | 0.303 | 0.211 | 0.230 | 0.599 | 0.244 |
| 120 | 0.259 | 0.255 | 0.413 | 0.448 | 0.236 |
| 240 | 0.094 | 0.375 | 0.986 | 0.113 | 0.197 |
| 360 | 0.046 | 0.239 | 0.795 | 0.118** | 0.100 |
| 480 | 0.029 | 0.147 | 0.581 | 0.077** | 0.069 |
| 600 | 0.025 | 0.077 | 0.236 | 0.045** | 0.048* |
| 1440 | ND | ND | ND | ND | ND |

| | | | | | |
|---|---|---|---|---|---|
| ND = No detection, * n=2, ** n=1 | | | | | |

From the above concentration averages, the area under the curve (AUC_{inf}), Tₘₐₓ and Cₘₐₓ were calculated and their ratio determined (AUC_{inf}/Cₘₐₓ), as shown in Table 27 below. All four controlled release formulations had later Tₘₐₓ than the immediate release formulation. For the ratio of AUC_{inf}/Cₘₐₓ, the immediate release formulation had the lowest ratio out the five formulations, while the hydrophilic and the fat-wax formulations had the best ratios. In addition, a dose-dependent increase in AUC_{inf} was observed for the concentrations of hydrophilic formulation #300-1 as compared to hydrophilic formulation #100-2.

**Table 27: Pharmacokinetic Parameters**

| | **Formulations** | | | | |
|---|---|---|---|---|---|
| | **Immediate Release** | **Hydrophobic #100-2** | **Hydrophilic #300-1** | **Fat-wax #100-3** | **Hydrophobic #100-3** |
| Cₘₐₓ (µ/mL) | 0.770 | 0.375 | 0.986 | 0.599 | 0.244 |
| Tₘₐₓ (minutes) | 30 | 240 | 240 | 90 | 90 |
| AUC_{inf} (µg/ML/ minutes) | 96 | 143 | 407 | 134 | 94 |
| AUC_{inf}/Cₘₐₓ | 125 | 381 | 413 | 223 | 387 |

As shown above, all four controlled release tablet formulations have later Tₘₐₓ than the immediate release table formulations and all four had broader pharmacokinetic curves while minimizing the Cₘₐₓ.

The following Table 28 presents the plasma concentrations of the active ingredient, vernakalant hydrochloride, in dogs that received a hydrophilic controlled release tablet formulation of the invention comprising 300 mg of the active ingredient, (*i.e*., hydrophilic formulation #300-2); a fat-wax controlled release tablet formulation of the invention comprising 300 mg of the active ingredient (*i.e*., fat-wax formulation #300-1); a hydrophilic/hydrophobic controlled release tablet formulation of the invention comprising 300 mg of the active ingredient (*i.e*., hydrophobic formulation #300-1), and a hydrophilic (non-cellulose) tablet formulation of the invention comprising 300 mg of the active ingredient (*i.e*., hydrophilic (non-cellulose) formulation #300-1). Concentrations are given as µg/mL and are an average obtained from n = 3 dogs unless otherwise indicated.

**Table 28: Plasma concentrations of active ingredient in dogs after oral administration of various formulations of the invention**

| | **Formulations** | | | |
|---|---|---|---|---|
| **Time (min)** | **Hydrophilic #300-2** | **Fat-wax #300-1** | **Hydrophilic/ Hydrophobic #300-1** | **Hydrophilic (non-cellulose) #300-1** |
| 0 | ND | ND | ND | ND |
| 30 | 0.321 * | 0.240 | 0.058* | 0.513 |
| 60 | 0.560 | 0.696* | 0.244 | 1.976 |
| 90 | 0.992 | 1.031 | 1.096 | 2.945 |
| 120 | 0.981 | 0.973 | 1.217 | 2.421 |
| 240 | 1.516 | 0.833 | 1.475 | 0.692 |
| 360 | 0.785 | 0.720 | 0.468 | 0.321 |
| 480 | 0.329 | 0.600 | 0.272 | 0.239 |
| 600 | 0.209 | 0.539 | 0.147 | 0.218 |
| 720 | 0.171 | 0.352* | 0.100 | 0.141 |
| 1440 | 0.042 | 0.056** | 0.015* | 0.026* |

| | | | | |
|---|---|---|---|---|
| ND = No detection, * n=2, ** n=1 | | | | |

From the above concentration averages, the area under the curve (AUC_{inf}), Tₘₐₓ and Cₘₐₓ were calculated and their ratio determined (AUC_{inf}/Cₘₐₓ), as shown in Table 29 below. For the ratio of AUC_{inf}/Cₘₐₓ the hydrophilic formulation #300-2 and the fat-wax formulation #300-1 had the best ratios. I

**Table 29: Pharmacokinetic parameters**

| | **Formulations** | | | |
|---|---|---|---|---|
| | **Hydrophilic #300-2** | **Fat-wax #300-1** | **Hydrophilic/ Hydrophobic #300-1** | **Hydrophilic (non-cellulose) #300-1** |
| Cₘₐₓ (µg/mL) | 1.516 | 1.031 | 1.475 | 2.945 |
| Tₘₐₓ (minutes) | 240 | 90 | 240 | 90 |
| AUC_{inf} (µg/ML/minutes) | 578 | 645 | 469 | 600 |
| AUC_{inf}/Cₘₐₓ | 381 | 626 | 318 | 204 |

Compared to a comparable immediate release table formulation, all four formulations have later Tₘₐₓ and broader pharmacokinetic curves while minimizing the Cₘₐₓ.

### EXAMPLE 20

### IN VIVO PHARMACOKINETIC PROFILES OF CONTROLLED RELEASE FORMULATIONS OF THE INVENTION ADMINISTERED TO HUMANS

Hydrophilic formulation #300-2 and Fat Wax formulation #300-2 were each administered as one dose (300 mg of active ingredient) to six healthy male and female subjects (six subjects per formulation). Blood was drawn at pre-dose (0 hours), 0.5, 1, 1.5, 2, 3, 4, 6, 8,10, 12, 16 and 24 hours post dose. The median pharmacokinetic parameters of each formulation are shown in the following Table 30:

**Table 30: Pharmacokinetic parameters of 300 mg active ingredient**

| | **Comparative Hydrophilic #300-2** | **Comparative Fat Wax #300-1** |
|---|---|---|
| Cₘₐₓ (ng/mL) | 290 | 296 |
| Tₘₐₓ (hours) | 2.0 | 1.75 |
| AUC_{0-inf} (ng/mU hour) | 2029 | 1984 |

Based on the above results, hydrophilic formulation #300-2 was administered as a double dose (600 mg of active ingredient) to six healthy male and female subjects. Blood was drawn at pre-dose (0 hours), 0.5, 1, 1.5, 2, 3, 4, 6, 8, 10, 12, 16 and 24 hours post dose. The median pharmacokinetic parameters are shown in the following Table 31:

**Table 31: Pharmacokinetic parameters of 600 MG active ingredient**

| | **Comparative Hydrophilic #300-2 (X2)** |
|---|---|
| Cₘₐₓ (ng/mL) | 706 |
| Tₘₐₓ (hours) | 2.0 |
| AUC_{0-inf} (ng/mUhour) | 5307 |

### EXAMPLE 21

### PREVENTION OF RECURRENCE OF ATRIAL FIBRILLATION/ATRIAL FLUTTER

The following study was conducted to evaluate, *inter alia,* the efficacy of formulations of the invention in human subjects with sustained atrial fibrillation (atrial fibrillation of longer than 72 hours and less than 6 months duration).

Subjects were administered a formulation of the invention on Day 1 and were monitored for the first 3 days of dosing. Subjects who were still in atrial fibrillation on the third day of dosing were electrically converted to sinus rhythm. Subjects who converted to sinus rhythm without intervention (other than study medication) or who were successfully electrocardioverted continued with study medication for a total of 28 days of study treatment administration.

A total of 221 subjects were enrolled in the study, 75 subjects received placebo, 71 subjects received twice daily one capsule containing one controlled release tablet formulation of the invention (300 mg active ingredient b.i.d.), hydrophilic formulation #300-2, 75 subjects received twice daily one capsule containing two controlled release tablet formulations of the invention (600 mg active ingredient b.i.d.), hydrophilic formulation #300-2. The majority of the study subjects were male (61.4%) and Caucasian (100%), with a mean age of 64 ± 10 years (range 32-83 years). A total of 171 subjects were converted to sinus rhythm by Day 3 of the study and continued to received study medication through Day 28.

The time to first documented recurrence of symptomatic sustained atrial fibrillation or atrial flutter was longer in subjects receiving the active ingredient than subjects receiving placebo. 43.1% of placebo subjects were in sinus rhythymm on Day 28 compared to 61.6% of subjects treated with 300 mg. active ingredient b.i.d. and 62.4% of subjects treated with 600 mg active ingredient b.i.d.

This study demonstrated the ability of hydrophilic formulation #300-2 to reduce the short term recurrence of atrial fibrillation.

### EXAMPLE 22

### DISPOSITION AND MASS BALANCE OF VERNAKALANT HYDROCHLORIDE AFTER INTRAVENOUS ADMINISTRATION

This study was designed to examine the pharmacokinetics and disposition of vernakalant hydrochloride, and to estimate mass balance after administration of single intravenous (IV) and oral doses of ¹⁴C-vernakalant hydrochloride to healthy male volunteers. Since vernakalant hydrochloride is primary metabolized by CYP2D6, the subsets genotyped as CYP2D6 EMs and PMs were compared.

### METHODS

### Participants

Eight healthy male volunteers provided written informed consent before any assessments were conducted; all completed the study as planned. The study cohort had a median age of 32 years (range, 21-42 years) and comprised 6 whites, 1 black, and 1 Asian. Median weight was 74.1 kg (range, 68.9-85.7 kg), and median height was 178 cm (range, 169-199 cm). Initial genotyping characterized 6 men as EMs and 2 as PMs; however, 1 EM did not exhibit the expected phenotypic features and after subsequent retesting was determined to be an intermediate metabolizer.

### Study Design

This phase 1 study used an open-label, single-sequence, crossover design. Participants were admitted to the study center the day before dosing (*i.e*., day -1 and day 21) and received ¹⁴C-vernakalant hydrochloride 240 mg in a 10-minute IV infusion in 100 mL of saline on day 1 and in an oral gel capsule taken with 250 mL of water on day 22, approximately 1 hour after a standard meal. The specific activity of the administered dose was 0.329 µCi/mg, yielding a total radioactive dose of 78.96 µCi. Participants remained confined to the center for 7 days after drug administration to ensure collection of urine and fecal samples for recovery of radioactivity. After an additional 2-week follow-up as outpatients, the cohort returned to the center 3 weeks after each dose (*i.e*., days 21 and 42) for end-of-study assessments.

This study complied with the principles of the Declaration of Helsinki and its amendments and with Good Clinical Practices. The study site's institutional ethics committee approved the protocol and informed consent form.

### Sample Collection

Vernakalant hydrochloride and its metabolites were measured in plasma and urine, and total radioactivity (¹⁴C) was determined in plasma, whole blood, urine, feces, saliva, and (if applicable) vomitus. On days 1 and 22, 6-mL blood samples for analysis of plasma vernakalant hydrochloride and total ¹⁴C were collected before dosing, at 5, 15, 30, and 45 minutes, and at 1, 2, 4, 6, 8, 12, 18, and 24 hours after the end of the IV or oral administration, and thereafter every 24 hours for 6 additional days, as well as at the end-of-study assessment. Additional samples were obtained 5 minutes into the IV infusion and at its end. Also, on days 1 and 22, 10-mL blood samples for metabolic profiling were collected 0.5, 2, 8, and 24 hours after dosing.

Urine for measurement of vernakalant hydrochloride, metabolites, and ¹⁴C was collected during the 4 hours before dosing, for the 0 to 1, 1 to 2, 2 to 4, 4 to 8, 8 to 12, and 12 to 24-hour intervals after dosing, and then for each 24-hour interval for 6 additional days. Fecal samples for ¹⁴C analysis were collected for 12 to 24 hours before dosing and for each 24-hour period for 7 days after dosing. Urine and fecal specimens were also collected at the end-of-study assessment. Saliva for ¹⁴C analysis was obtained before dosing, 0.25, 0.5, 2, and 8 hours afterward, and on occurrence of any treatment-related adverse event related to dysgeusia. Vomitus for ¹⁴C analysis was collected at any time over the 7 days after oral administration when vomiting occurred.

### Safety Assessments

Adverse events were evaluated daily, classified by intensity and association with treatment, and coded according to the MedDRA dictionary version 6.1. Vital signs, physical examination, 12-lead electrocardiograph (ECG), and laboratory tests including clinical chemistry, hematology, and urinalysis were evaluated at screening, 7 days after dosing, and at the end-of-study assessment. Telemetric monitoring was started approximately 12 hours before dosing and continued for 24 hours afterward. In addition, vital signs and 12-lead ECG were obtained 15 minutes before dosing; the vital signs assessment was repeated 15, 30, 60, and 90 minutes after dosing and then daily for 7 days.

### CYP2D6 Genotyping

CYP2D6 genotyping was performed by single nucleotide polymorphism analysis via real-time polymerase chain reaction (Capio Diagnostik AB, Eskilstuna, Sweden).

### Pharmacokinetic Analyses

Plasma and urine concentrations of vernakalant hydrochloride, the Compound 2, Compound 3, and Compound 4 metabolites, and their corresponding glucuronidation products (Figure 7; designated by the letter G) were determined by liquid chromatography-tandem mass spectrometry (LC/MS/MS). Plasma samples first underwent solid-phase extraction on SPE cartridges containing a 30-mg MCX phase (Waters, Milford, Mass); urine samples were analyzed directly. For the determination of glucuronide and sulphate conjugates, plasma and urine samples underwent an enzymatic deconjugation with a solution of 0-glucuronidase (5000 units/mL in 0.1M sodium acetate buffer pH 5.0) and were incubated for 30 hours (plasma) or 20 hours (urine) at 37°C. Subsequently, the urine samples were injected directly on to the column and the plasma samples were further treated with SPE as described above. A 100-µL aliquot was injected onto a Cadenza CD-C18 column (250 x 4.6 mm i.d., 3-µm particle size; Imtakt, Kyoto, Japan) maintained at 40°C in an Alliance model 2690 Separations Module (Waters). Samples were eluted at a flow rate of 1.0 mL/min with a mobile phase of methanol (phase A) and 15 mM of ammonium formate in water (phase B) (30%:70%, v:v) for 35 minutes, followed by a step gradient to 90%:10% (v:v) for 7 minutes. Radiochromatograms were recorded by splitting the LC column effluent into 2 parts with an ASI Quicksplit adjustable flow splitter. The major part was directed to a Foxy 200 fraction collector (Isco, Lincoln, Neb); Pico Fluor 30^{®} scintillant was added to each fraction; and radioactivity was counted in a Packard TriCarb™ liquid scintillation analyzer. The other part of the LC column effluent was directed to model API 3000 MS (MDX Sciex, Concord, Canada) with ionspray detection for structural confirmation and identification.

Pharmacokinetic values were analyzed using noncompartmental analysis by means of WinNonlin version 5.0 (Pharsight Corp, Mountain View, Calif). Parameters included area under the drug concentration-time curve (AUC₀₋ₜ), calculated by trapezoidal integration from time 0 to the time with the last measurable concentration (Cₜ); AUC extrapolated from time 0 to infinity, calculated as AUC₀₋ₜ +Cₜ/kₑₗ, where kₑₗ is the terminal elimination-rate constant calculated by linear regression of the terminal linear portion of the log Cₜ-time curve (AUC_{0-∞}); elimination half-life (t_{1/2}), calculated as In 2/kₑₗ; maximum observed drug concentration (Cₘₐₓ) and the time when it was observed without interpolation (Tₘₐₓ); clearance (CL), calculated as dose/AUC_{0-∞}; apparent volume of distribution of the terminal phase (V_{dz}); volume of distribution at steady state (Vₛₛ); and renal clearance (CL_{R}), calculated as total unchanged vernakalant hydrochloride in urine/plasma AUC_{0-∞}. Oral bioavailability (F) was determined from the ratio of AUC_{0-∞} after oral dosing to Aut_{0-∞} after IV dosing, and was used to calculate the oral clearance (CUF), V_{dz} after oral dosing (V_{dz}/F), and Vₛₛ after oral dosing (Vₛₛ/F).

These pharmacokinetic analyses and the measurements of total radioactivity covered the period from dosing through 168 hours (7 days) afterward. The amount of these analytes excreted in urine was calculated according to time period and cumulatively over the 7 days. Total ¹⁴C in urine and feces (and vomitus, if applicable) was also determined by time and cumulatively over the study for the mass-balance estimation.

### Statistical Methods

The concentrations of vernakalant hydrochloride and metabolites in plasma and urine and the total ¹⁴C in plasma, whole blood, urine, feces, and saliva were analyzed descriptively. Concentrations below the lower limit of quantitation were set to 0. Descriptive statistics were used as well for pharmacokinetic values, which were presented separately for the 5 EMs and 2 PMs. The intermediate metabolizer was excluded from the subgroup comparisons. Safety parameters were summarized descriptively for the entire cohort.

### RESULTS

### Pharmacokinetics

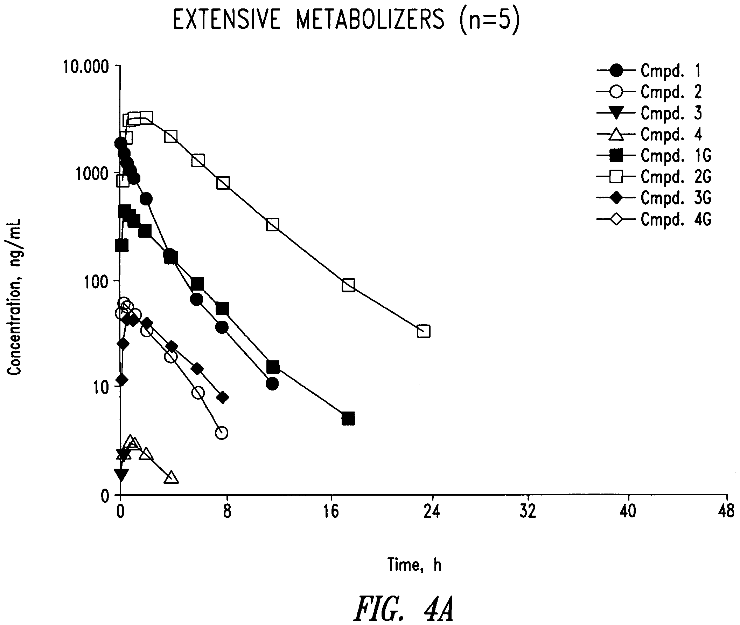

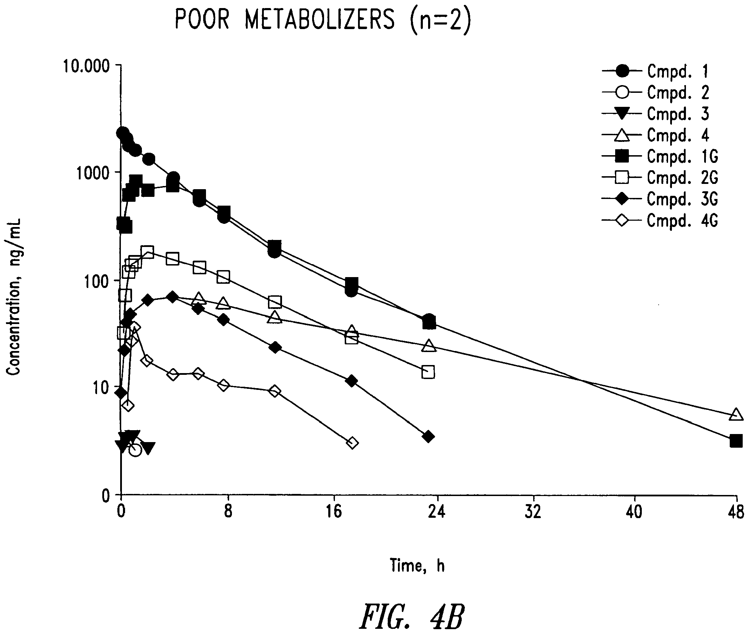

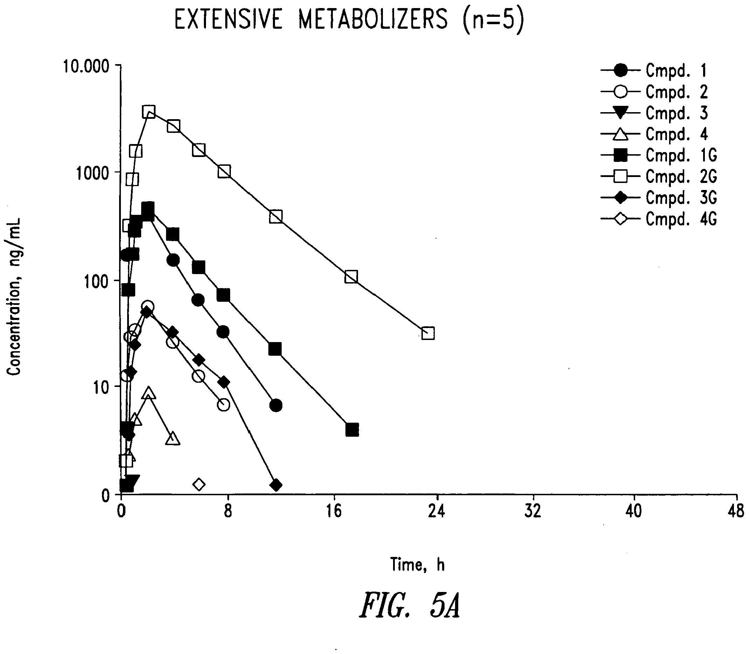

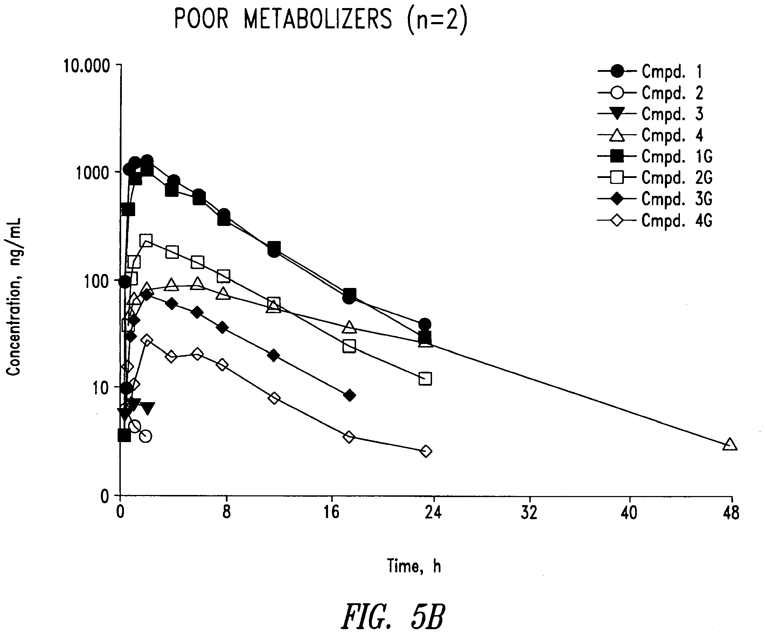

The plasma concentration-time profiles of vernakalant hydrochloride and its metabolites are shown after IV (Figure 4) and oral (Figure 5) dosing. With IV dosing, a similar Cₘₐₓ was obtained in EMs and PMs. With oral dosing, however, a 3-fold higher Cₘₐₓ was seen in PMs than EMs. Following the alpha distribution phase, several differences between EMs and PMs were apparent. Plasma concentration of vernakalant hydrochloride decreased much more rapidly in EMs after 90 minutes. Mean plasma Compound 2G concentration was substantially higher, but mean Compound 4 and Compound 4G concentrations were substantially lower in EMs than in PMs. Glucuronidation of vernakalant hydrochloride was less pronounced in EMs. The concentration-time profiles were qualitatively similar after IV infusion and oral administration.

Vernakalant hydrochloride was distributed extensively into tissues after IV infusion, independent of genotype: the mean Vₛₛ was 123.1 L for EMs and 112.7 L for PMs; respective mean V_{dz} values were 208.9 L and 162.2 L. In contrast, the mean terminal t_{1/2} of vernakalant hydrochloride was longer in PMs than in EMs after IV infusion (5.66 vs 2.19 h) (Table 32). This difference reflected a 3.3-fold lower plasma CL rate in PMs (19.8 vs 64.9 Uh); as a result, total exposure to vernakalant hydrochloride was 3 times higher in PMs (auto_{0-∞}, 11,035 vs 3605 ng.h/L).

Vernakalant hydrochloride was rapidly absorbed after oral administration, with a mean Tₘₐₓ of 1.8 hours in EMs and 1.25 hours in PMs (see Table 32). Plasma CL of vernakalant hydrochloride was again slower in PMs, with the t_{1/2} 2.5 times longer (4.73 vs 1.89 h) and the AUC_{0-∞} 6 times higher (9090 vs 1504 ng.h/mL) in this subset. Vernakalant hydrochloride oral bioavailability-determined from the AUC_{0-∞} ratio after oral versus IV dosing-was 40.2% in EMs and 81.8% in PMs.

Pharmacokinetic values were also determined for the metabolites of vernakalant hydrochloride, including the 4-O-demethylated Compound 2, the 3-O-demethylated Compound 3, the vernakalant diastereomer Compound 4, and their respective glucuronides (vernakalant glucuronide, Compound 2G, Compound 3G, and Compound 4G). The primary metabolite in EMs was Compound 2G, which formed rapidly and was measurable by the end of the 10-minute infusion (Figure 4). Plasma Compound 2G concentration reached maximum by 1.4 hours after IV infusion and 2.4 hours after oral administration in the EMs (Figures 4 and 5) and was eliminated with respective t_{1/2} of 3.3 and 3.2 hours (Table 32). The mean Cₘₐₓ was approximately 15 to 20 times higher, and the mean AUC_{0-∞} was 10 times higher in EMs than in PMs. In contrast, the primary metabolite in PMs was vernakalant glucuronide, with Cₘₐₓ values 2 times higher and AUC_{0-∞} values approximately 4 times higher in PMs.

The Compound 3 metabolite was not detectable in plasma at most time points, although Compound 3G was measurable. The AUC_{0-∞} of Compound 3G was approximately 3 times higher in PMs than EMs, reflecting a longer t_{1/2} rather than a major difference in Cₘₐₓ between the subsets. Finally, Compound 4 and Compound 4G were identified mostly in the PMs. In light of Tₘₐₓ and t_{1/2} values, these metabolites appear to be both produced and cleared more slowly than the other metabolites in PMs.

### Urinary Excretion

Unchanged vernakalant hydrochloride accounted for a larger percentage of urinary recovery in PMs than EMs after IV infusion (22.6% vs 8.5%) and oral administration (21.9% vs 3.5%). The excretion rate of unchanged vernakalant hydrochloride was maximum in the first hour after IV infusion (7.04 and 8.33 mg/h in EMs and PMs, respectively). Urinary recovery of unchanged drug was complete within 24 hours in EMs and 48 hours in PMs. The mean CL_{R} of vernakalant hydrochloride after IV infusion was 5.60 Uh in EMs and 4.43 Uh in PMs. Comparison of the cumulative amounts of vernakalant hydrochloride metabolites by 7 days after dosing supports the differences in plasma concentrations observed between the subgroups. Higher amounts of Compound 2 and Compound 2G were recovered in urine from EMs, whereas higher amounts of vernakalant glucuronide, Compound 3G, Compound 4, and Compound 4G-besides the higher levels of unchanged vernakalant hydrochloride-were recovered in PMs (Table 33).

### Total Radioactivity

The t_{1/2} of total ¹⁴C from plasma was longer in PMs than EMs after IV infusion (9.46 vs 4.59 h) and oral administration (7.78 vs 4.21 h) of ¹⁴C-vernakalant hydrochloride (Table 3). Nevertheless, the mean AUC_{0-∞} for total ¹⁴C was only 12% to 14% higher in PMs. Qualitatively similar results were obtained for total ¹⁴C in whole blood, although the lower Cₘₐₓ and AUC_{0-∞} values suggested that vernakalant hydrochloride and its metabolites are not taken up by circulating blood cells (Table 34). In saliva, total ¹⁴C peaked at 0.4 hour after IV infusion and 2 hours after oral administration, with Cₘₐₓ values being higher after IV than oral dosing in both EMs (1498 vs 257 ng.eq/mL) and PMs (1435 vs 693 ng.eq/mL).

### Mass Balance

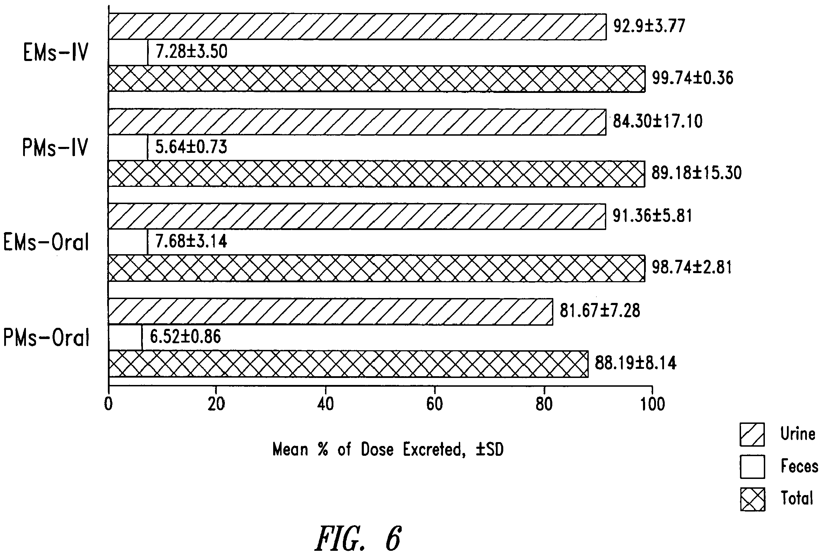

The mean recovery of ¹⁴C in urine was marginally higher in EMs than PMs after both IV infusion (92.9% vs 84.3%) and oral administration (91.4% vs 81.7%) (Figure 6). One PM, however, had suspected urine loss after IV dosing (corresponding urinary ¹⁴C recovery of 72.2%) and a discarded urine sample after oral dosing (corresponding urinary ¹⁴C recovery of 76.5%). Low levels of radioactivity-near the lower limit of quantitation of 10 dpm/mL-were detected in 3 subjects at the end-of-study assessment on day 42, likely reflecting the background noise of the ¹⁴C quantitation method. Fecal recovery of ¹⁴C averaged 7.3% in EMs and 5.6% in PMs after IV infusion, and 7.7% and 6.5%, respectively, after oral administration. No radioactivity was detected in fecal samples on day 42.

The recovery of ¹⁴C in urine and feces after administration of ¹⁴C-vernakalant hydrochloride was summed to provide a mass-balance estimate. Mean recovery was 99.7% in EMs and 89.2% in PMs after IV infusion and 98.7% in EMs and 88.2% in PMs after oral administration. The lower recovery in PMs presumably reflects the suspected urine loss and discarded sample in 1 subject.

### Safety

Treatment-emergent adverse events occurred in 6 (75%) of 8 subjects after IV and oral administration of ¹⁴C-vernakalant hydrochloride, but none discontinued for this reason. Headache, the most common adverse event, was reported by 3 subjects after each route of administration, but only 1 complained of headache after both IV and oral dosing. Dysgeusia, noted by 3 subjects after the IV infusion, did not occur after oral administration. The only other adverse events that affected more than 1 subject were paresthesia (2 subjects after IV infusion) and rash (2 subjects after oral dosing). The investigator rated all adverse events as mild except for episodes of influenza-related illness and anal fissure (1 each), which were moderate and not related to study treatment. Headache in 2 subjects after IV and oral dosing and the 3 incidents of dysgeusia after IV dosing were the only drug-related adverse events that occurred in more than 1 individual.

Decreases in pulse rate-maximum, 6 beats per minute-were observed within 60 minutes of each dose. Small transient increases in diastolic blood pressure occurred 30 minutes after IV infusion and 60 minutes after oral dosing. No consistent changes in systolic blood pressure were recorded. The ECG demonstrated no clinically significant changes. Four participants had ECG abnormalities the day before vernakalant hydrochloride administration-delayed intraventricular conduction in 3 and tachycardia in 1-that the investigator considered clinically insignificant. No clinically significant laboratory findings were reported as adverse events. Elevated creatine kinase levels that were evident in 4 participants primarily during the outpatient period were attributed to exercise.

### DISCUSSION

Vernakalant hydrochloride is distributed and metabolized rapidly and extensively, as evidenced by the low plasma concentrations of unchanged drug relative to those of its major metabolites 30 minutes after IV infusion and 60 minutes after oral administration in this study. The metabolites recovered were qualitatively similar with both routes of administration and depended on the individual's CYP2D6 genotype. In EMs, vernakalant hydrochloride was metabolized predominantly by CYP2D6 to the 4-O-demethylated metabolite Compound 2, most of which was rapidly glucuronidated. Vernakalant hydrochloride was also glucuronidated directly, which is especially prominent in PMs; metabolism by way of 3-O-demethylation to Compound 3 or by chiral inversion to Compound 4 appeared to be minor pathways in EMs and PMs, with even less importance in EMs. Glucuronide conjugates are typically inactive and are eliminated rapidly but in some cases may be recycled back to the parent drug (Kroemer H.K and Klotz, U. Clin. Pharmacokinet. 23:292-310 (1992)). The concentration-time profiles of vernakalant hydrochloride and Compound 2 showed no evidence of recycling, however. Moreover, vernakalant hydrochloride was eliminated rapidly, predominantly in urine, and substantial recovery of unchanged drug and metabolites was seen within the first several hours after administration.

Vernakalant hydrochloride was distributed extensively into tissue in both EMs and PMs. The mean V_{dz} and Vₛₛ were approximately 30 to 40 times greater than the total blood volume (approximately 5.2 L in a 70-kg man) and 4 to 5 times greater than total body water (approximately 42 L in a 70-kg man) (Davies, B. and Morris, T., Pharm Res. 10:1093-1095 (1993)). This rapid and extensive distribution was likely responsible for the lack of difference in Cₘₐₓ seen between EMs and PMs with IV dosing.

Vernakalant hydrochloride's metabolism was slower and less extensive in PMs than in EMs, as reflected by the higher plasma AUCs and urinary recovery of unchanged vernakalant hydrochloride in the PM subset. Consistent with the higher plasma levels of unchanged drug later after dose, vernakalant hydrochloride was cleared more slowly in PMs, with the result that the t_{1/2} of unchanged vernakalant hydrochloride was about 2.5 times longer in that subset. The metabolite profile also differed between EMs and PMs (Figure 7). As expected, Compound 2G, the major metabolite in EMs, was generated in much smaller amounts in PMs, in whom the predominant metabolite was vernakalant glucuronide. Compound 4, Compound 4G, and Compound 3G represented minor metabolites, although each was produced in higher amounts in PMs than in EMs.

Although vernakalant hydrochloride was rapidly absorbed after oral administration, oral bioavailability was approximately 2 times higher in PMs than in EMs (81.9% vs 40.2%), contributing to the higher drug exposure in PMs, likely as a result of lower presystemic clearance.

Mass balance was demonstrated with both the IV and oral doses by the end of the 7-day clinical portion of the study. In EMs, the mean recovery of the administered ¹⁴C dosage was 99.7% after IV infusion and 98.7% after oral administration. Mean recovery of ¹⁴C appeared lower in the PMs, but this subset had only 2 members, 1 of whom had suspected urine loss after the IV dose and a discarded urine sample after the oral dose. The demonstration of mass balance was supported by the absence of measurable radioactivity in plasma, urine, and feces 7 days after dosing and by the t_{1/2} of total ¹⁴C (4.59 h in EMs and 9.46 h in PMs after IV infusion; 4.21 h and 7.78 h after oral dosing).

The 240-mg dose of vernakalant hydrochloride used in this study is consistent with doses used in clinical trials of patients with AF (Roy, D. et al., J. Am. Coll. Cardiol. 44:2355-2361 (2004)). For a 70-kg person, 240 mg is equivalent to 3.4 mg/kg. In clinical trials, vernakalant hydrochloride was administered as a 3-mg/kg IV infusion over 10 minutes; if AF persisted after 15 minutes of observation, a second 10-minute infusion of 2 mg/kg was given. This regimen rapidly converted AF to sinus rhythm and was well tolerated. Similarly, a 300-mg twice-daily oral dose as a sustained-release formulation has been found to be well tolerated and efficacious in maintaining sinus rhythm following conversion from AF.¹¹

In the present study, the single dose of vernakalant hydrochloride was well tolerated. Headache and dysgeusia-the latter seen only after IV infusion-were the most common adverse events. No clinically significant changes were seen on vital signs or the ECG in subjects in the study.

### Additional Clinical Studies

The effect of CYP2D6 genotype on vernakalant hydrochloride pharmacokinetics and metabolism was also analyzed in two randomized, double-blind, placebo-controlled multicenter phase 3 studies, wherein patients aged ≥18 years with typical AF or nontypical AFL lasting >3 hours to ≤45 days were treated with either vernakalant hydrochloride 3 mg/kg or placebo via 10-minute infusion, followed by a second 10-minute infusion of vernakalant hydrochloride 2 mg/kg (or placebo) if AF or AFL was present after a 15-minute observation period. Plasma concentration of vernakalant hydrochloride and its 4-O-demethylated metabolite (Compound 2) were determined by a validated LC-MS/MS method with a lower quantitation limit of 0.005 µg/ml. CYP2D6 genotyping was performed by single nucleotide polymorphism analysis via real-time polymerase chain reaction (Capio Diagnostik AB, Eskilstuna, Sweden). CYPD6 genotype was assessed in 193 patients; only 7 patients had a PM genotype.

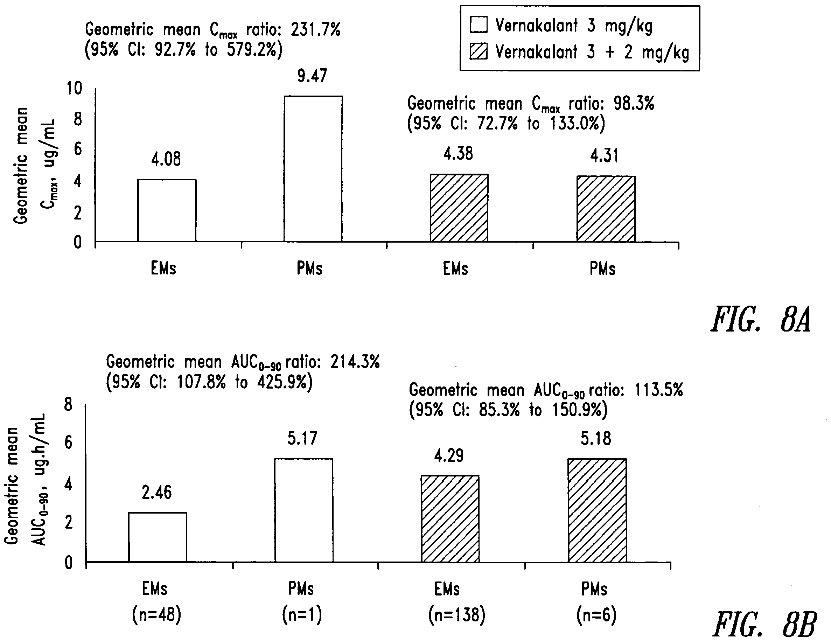

Low concentrations of the metabolite Compound 2 were present in blood samples collected at 10 to 35 minutes after the start of first infusion, with peak concentrations reached by 35 to 50 minutes. Of the six CYP2D6 PMs who received 2 doses of vernakalant hydrochloride, the Cₘₐₓ and AUC₀₋₉₀ of vernakalant hydrochloride were comparable to the values found in EMs (Figure 8). One CYP2D6 PM received 1 dose of vernakalant hydrochloride; the Cₘₐₓ and AUC₀₋₉₀ values in this subject were higher than the mean values for EMs, but within the range of values seen among EMs.

### Conclusions

The pharmacokinetics and metabolism of vernakalant hydrochloride depend on the CYP2D6 genotype. Vernakalant hydrochloride underwent rapid and extensive distribution during infusion, which resulted in similar Cₘₐₓ values in EMs and PMs with IV, but not oral, dosing. Compared with EMs, PMs had a higher overall exposure to unchanged vernakalant hydrochloride, a longer drug-elimination t_{½}, lower Compound 2G concentration, and higher concentration of vernakalant glucuronide, as well as the minor metabolites Compound 4, Compound 3, and their respective glucuronides. These alternate routes of clearance reduce the magnitude of difference in exposure to vernakalant hydrochloride between EMs and PMs. Given their magnitude, these differences are unlikely to be clinically important with short-term IV use, e.g., for immediate treatment of acute arrhythmia, but may be significant for long term use or oral administration, e.g., for the prevention of arrhythmia.

**Table 32. Pharmacokinetics of Vernakalant hydrochloride and Its Metabolites After IV or Oral Administration of a Single 240-mg Dose, by CYP2D6 Polymorphism**

| **Parameter Analyte** | | **IV Infusion** | | **Oral Administration** | |
|---|---|---|---|---|---|
| | | **EMs (n=5)** | **PMs (n=2)** | **EMs (n=5)** | **PMs (n=2)** |
| Cmax, ng/mL | | | | | |
| | Vernakalant | 3227±1374 | 3972±1047 | 481.2±240.4 | 1558±37.5 |
| | Vernakalant glucuronide | 455.6±118.9 | 942.0±213.5 | 496.0±158.5 | 1078±297.7 |
| | Compound 2 | 68.6±18.0 | 3.31±4.67 | 63.2±21.9 | 6.25±8.84 |
| | Compound 2 glucuronide | 3489±371.4 | 180.5±48.8 | 3824±349.3 | 230.5±4.99 |
| | Compound 3 | 2.85±3.94 | 3.72±5.26 | 1.41±3.16 | 9.08±2.16 |
| | Compound 3 glucuronide | 45.1±8.80 | 70.5±14.4 | 53.0±14.7 | 74.0±6.53 |
| | Compound 4 | 5.42±7.51 | 69.6±9.1 | 9.71±7.56 | 92.4±13.6 |
| | Compound 4 glucuronide | 0 | 38.5±29.9 | 1.26±2.81 | 32.8±10.7 |

| Tₘₐₓ, h | | | | | |
|---|---|---|---|---|---|
| | Vernakalant | 0.17±0.004 | 0.13±0.06 | 1.80±0.45 | 1.25±1.06 |
| | Vernakalant glucuronide | 0.48±0.13 | 1.67±0.71 | 1.80±0.45 | 1.38±0.88 |
| | Compound 2 glucuronide | 1.44±0.69 | 2.17±0.0 | 2.40±0.89 | 2.00±0.0 |

| **Parameter Analyte** | | **IV Infusion** | | **Oral Administration** | |
|---|---|---|---|---|---|
| | | **EMs (n=5)** | **PMs (n=2)** | **EMs (n=5)** | **PMs (n=2)** |
| | Compound 3 | 0.34±0.12* | 0.42^{†} | 0.77^{†} | 1.25±1.06 |
| | Compound 4 | 0.68±0.36* | 4.17±0.0 | 1.75±0.50 | 5.00±1.41 |
| | Compound 4 glucuronide | (n=0) | 2.67±2.12 | 6.00^{†} | 4.00±2.83 |

| AUC_{0-∞}, ng.h/mL | | | | | |
|---|---|---|---|---|---|
| | Vernakalant | 3605±914.4 | 11,035±1237 | 1504±748.0 | 9090±2032 |
| | Vernakalant glucuronide | 1791±764.1 | 8074±118.6 | 2076±809.3 | 7759±1252 |
| | Compound 2 | 202.5±66.6 | (n=0) | 225.2±76.8 | (n=0) |
| | Compound 2 glucuronide | 20,711±2708 | 1933±360.0 | 21,182±3137 | 2025±65.9 |
| | Compound 3 | (n=0) | (n=0) | (n=0) | (n=0) |
| | Compound 4 | 66.5^{†} | 1463±172.4 | (n=0) | 1745±386.9 |
| | Compound 4 glucuronide | (n=0) | 291.6^{†} | (n=0) | 430.3^{†} |

| t_{1/2}, h | | | | | |
|---|---|---|---|---|---|
| | Vernakalant | 2.19±0.23 | 5.66±0.20 | 1.89±0.21 | 4.73±0.14 |
| | Vernakalant glucuronide | 2.76±0.65 | 6.58±2.59 | 2.57±0.41 | 4.32±0.27 |
| | Compound 2 | 2.28±0.29 | (n=0) | 2.19±0.29 | (n=0) |

| **Parameter Analyte** | | **IV Infusion** | | **Oral Administration** | |
|---|---|---|---|---|---|
| | | **EMs (n=5)** | **PMs (n=2)** | **EMs (n=5)** | **PMs (n=2)** |
| | Compound 2 glucuronide | 3.29±0.21 | 5.59±0.73 | 3.20±0.14 | 5.01±0.49 |
| | Compound 3 glucuronide | 2.75±0.61 | 5.89±0.77 | 2.51±0.36 | 4.77±0.75 |
| | Compound 4 | 2.87^{†} | 11.9±0.16 | (n=0) | 10.4±0.10 |
| | Compound 4 glucuronide | (n=0) | 9.10^{†} | (n=0) | 16.3^{†} |

| | | | | | |
|---|---|---|---|---|---|
| *n=2; ^{†}n=1. | | | | | |

**Table 33. Cumulative Amount of Vernakalant and Its Metabolites Excreted in Urine During the 7 Days After Administration of ¹⁴C-Vernakalant**

| | **Cumulative Amount, mg** | | | |
|---|---|---|---|---|
| **Metabolites Parameter** | **IV Infusion** | | **Oral Administration** | |
| | **EMs (n=5)** | **PMs (n=2)** | **EMs (n=5)** | **PMs (n=2)** |
| Vernakalant | 18.39±3.23 | 49.06±8.42 | 7.58±3.57 | 47.47±8.34 |
| Vernakalant glucuronide | 8.91±2.86 | 44.22±15.10 | 11.31±6.48 | 50.20±14.99 |
| Compound 2 | 3.11±0.85 | 0.48±0.02 | 3.88±1.14 | 0.62±0.19 |
| Compound 2 glucuronide | 120.01±19.03 | 11.36±5.52 | 121.76±44.38 | 12.03±0.51 |
| Compound 3 | 0.04±0.02 | 0.16±0.12 | 0.05±0.03 | 0.12±0.05 |
| Compound 3 glucuronide | 1.99±0.61 | 4.30±2.18 | 1.97±0.42 | 4.68±0.19 |
| Compound 4 | 0 | 5.14±0.31 | 0.14±0.14 | 7.41±0.78 |
| Compound 4 glucuronide | 0.08±0.19 | 2.02±0.14 | 0.01±0.03 | 1.85±1.17 |

**Table 34. Pharmacokinetic Values for Total Radioactivity* After IV or Oral Administration of ¹⁴C-Vernakalant**

| | | **IV Infusion** | | **Oral Administration** | |
|---|---|---|---|---|---|
| **Value** | | **EMs (n=5)** | **PMs (n=2)** | **EMs (n=5)** | **PMs (n=2)** |
| Plasma | | | | | |
| | Cₘₐₓ, ng/mL | 5943±539 | 4914±1023 | 6348±813 | 3954±346 |
| | Tₘₐₓ, h | 1.02±0.22 | 0.17±0.00 | 2.20±1.10 | 1.50±0.71 |
| | AUC_{0-∞}, ng·h/mL | 32,920±5495 | 37,763±921 | 32,908±5480 | 36,955±4555 |
| | t_{1/2}, h | 4.59±0.60 | 9.46±0.74 | 4.21±0.52 | 7.78±2.21 |
| Whole blood | | | | | |
| | Cₘₐₓ, ng/mL | 3891±994 | 4818±1589 | 3748±581 | 2581±168 |
| | Tₘₐₓ, h | 0.72±0.37 | 0.17±0.00 | 2.20±1.10 | 1.50±0.71 |
| | AUC_{0-∞}, ng·h/mL | 18,450±3106 | 26,107±4594 | 19,399±3909 | 21,106±969 |
| | t_{1/2}, h | 3.15±0.35 | 5.97±1.61 | 3.25±0.24 | 6.73±0.33 |

| | | | | | |
|---|---|---|---|---|---|
| *Expressed as ng-equivalents of vernakalant. | | | | | |

The various embodiments described above can be combined to provide further embodiments. All of the U.S. patents, U.S. patent application publications, U.S. patent applications, foreign patents, foreign patent applications and non-patent publications referred to in this specification and/or listed in the Application Data Sheet are incorporated herein by reference, in their entirety. Aspects of the embodiments can be modified, if necessary to employ concepts of the various patents, applications and publications to provide yet further embodiments.

These and other changes can be made to the embodiments in light of the above-detailed description. In general, in the following claims, the terms used should not be construed to limit the claims to the specific embodiments disclosed in the specification and the claims, but should be construed to include all possible embodiments along with the full scope of equivalents to which such claims are entitled. Accordingly, the claims are not limited by the disclosure.

The invention is also directed to the following 62 embodiments:
1. A method of preventing arrhythmia in a mammal, comprising:
   (a) determining if the mammal is a cytochrome P450(CYP)2D6 poor metabolizer (PM) or a cytochrome P450(CYP)2D6 extensive metabolizer; and
   (b) administering to the mammal a therapeutically effective amount of a composition comprising an ion channel modulating compound having the structure: 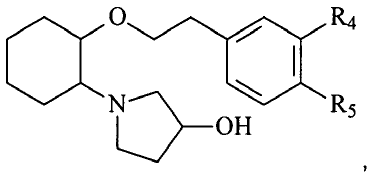
   including isolated enantiomeric, diastereomeric and geometric isomers thereof and mixtures thereof, or a solvate or pharmaceutically acceptable salt thereof; wherein R₄ and R₅ are independently selected from hydroxy and C₁-C₆alkoxy.
2. The method of embodiment 1 wherein the composition comprises a monohydrochloride salt of the formula: 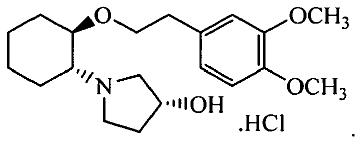
3. A method of preventing arrhythmia in a mammal, comprising:
   (a) determining if the mammal is a cytochrome P450(CYP)2D6 poor metabolizer (PM) or a cytochrome P450(CYP)2D6 extensive metabolizer; and
   (b) administering to the mammal an amount of an ion channel modulating compound sufficient to achieve a blood plasma concentration (Cmax) of between about 0.1 µg/ml and about 10 µg/ml for at least some time, wherein said ion channel modulating compound comprises an ion channel modulating compound of formula: 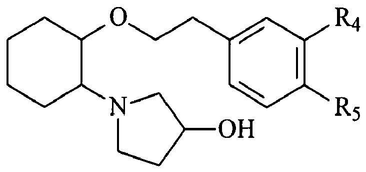
   including isolated enantiomeric, diastereomeric and geometric isomers thereof and mixtures thereof, or a solvate or pharmaceutically acceptable salt thereof; wherein R₄ and R₅ are independently selected from hydroxy and C₁-C₆alkoxy.
4. The method of embodiment 3 wherein the ion channel modulating compound is a monohydrochloride salt of the formula: 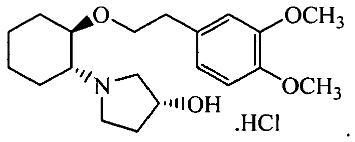
5. A method of preventing arrhythmia in a mammal who is a cytochrome P450(CYP)2D6 poor metabolizer (PM), comprising:
   (a) identifying the mammal as a cytochrome P450(CYP)2D6 PM; and
   (b) administering to the mammal a therapeutically effective amount of a composition comprising an ion channel modulating compound having the structure: 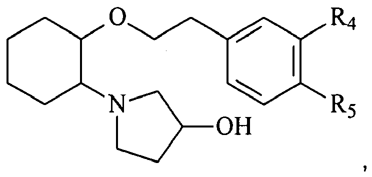
   including isolated enantiomeric, diastereomeric and geometric isomers thereof and mixtures thereof, or a solvate or pharmaceutically acceptable salt thereof; wherein R₄ and R₅ are independently selected from hydroxy and C₁-C₆alkoxy.
6. The method of embodiment 5 wherein the composition comprises a monohydrochloride salt of the formula: 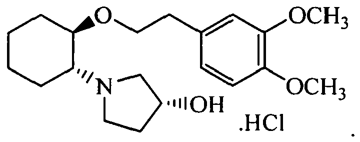
7. A method of preventing arrhythmia in a mammal who is a cytochrome P450(CYP)2D6 extensive metabolizer (EM), comprising:
   (a) identifying the mammal as a cytochrome P450(CYP)2D6 EM; and
   (b) administering to the mammal a therapeutically effective amount of a composition comprising an ion channel modulating compound having the structure: 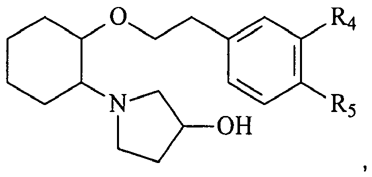
   including isolated enantiomeric, diastereomeric and geometric isomers thereof and mixtures thereof, or a solvate or pharmaceutically acceptable salt thereof; wherein R₄ and R₅ are independently selected from hydroxy and C₁-C₆alkoxy.
8. The method of embodiment 7 wherein the composition comprises a monohydrochloride salt of the formula: 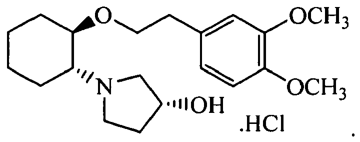
9. The method of any one of embodiments 1-8 wherein administration is by a route selected from the group consisting of: oral, topical, parenteral, sublingual, rectal, vaginal, and intranasal.
10. The method of embodiment 9 wherein the parenteral administration is selected from the group consisting of subcutaneous injection, intravenous injection, intramuscular injection, epidural injection, intrasternal injection, and infusion.
11. The method of embodiment 9 wherein the oral administration comprises administering an oral dosage form selected from the group consisting of a powder, a granule, a compressed tablet, a pill, a capsule, a cachet, a chewing gum, a wafer, and a lozenge.
12. The method of any one of embodiments 1-8 wherein the arrhythmia is atrial arrhythmia.
13. The method of embodiment 12 wherein the atrial arrhythmia is atrial fibrillation.
14. The method of any one of embodiments 1-8 wherein the arrhythmia is ventricular arrhythmia.
15. The method of embodiment 14 wherein the ventricular arrhythmia is ventricular fibrillation.
16. The method of embodiment 15 wherein the ventricular fibrillation occurs during acute ischemia.
17. The method of any one of embodiments 1-8 wherein the arrhythmia is a post-surgical arrhythmia.
18. The method of any one of embodiments 1-8 wherein the arrhythmia is a recurrent arrhythmia in a mammal who has previously undergone one or more arrhythmias.
19. The method of any one of embodiments 1-8 wherein the therapeutically effective amount is sufficient to achieve a total concentration of the ion channel modulating compound in the blood plasma of the mammal has a mean trough concentration of between about 1 ng/ml and about 10 µg/ml and/or a steady state concentration of between about 1 ng/ml and about 10 µg/ml.
20. The method of embodiment 19 wherein the total concentration of the ion channel modulating compound in the blood plasma of the mammal has a mean trough concentration of between about 0.3 µg/ml and about 3 µg/ml and/or a steady state concentration of between about 0.3 µg/ml and about 3 µg/ml.
21. The method of any one of embodiments 1-8 wherein the ion channel modulating compound is administered in two or more doses.
22. The method of any one of embodiments 1-8 wherein the ion channel modulating compound is administered in one or more doses of a tablet formulation comprising the ion channel modulating compound and at least one hydrophilic matrix system polymer selected from the group consisting of: carbomer, maltodextrin, hydroxyethyl cellulose, hydroxypropyl cellulose, hydroxypropyl methyl cellulose, and polyoxoacetate.
23. The method of any one of embodiments 1-8 wherein the ion channel modulating compound is administered at a dosage of about 50-1500 mg per day.
24. A method of increasing the bioavailability in a mammal of an ion channel modulating compound that is metabolized by cytochrome P450, comprising administering to said mammal the ion channel modulating compound and an effective amount of cytochrome P450-inhibiting compound.
25. A method of identifying a mammal suitable for long-term administration of an ion channel modulating compound that is metabolized by cytochrome P450(CYP)2D6 comprising:
   (a) identifying a mammal at risk for arrhythmia; and
   (b) determining that the mammal is a cytochrome P450(CYP)2D6 extensive metabolizer (EM).
26. The method of embodiment 25, wherein the arrhythmia is a recurrent arrhythmia or a post-operative arrhythmia.
27. The method of embodiment 25, wherein said mammal has previously undergone one or more arrhythmias.
28. A method of preventing an arrhythmia in a mammal, comprising:
   (a) identifying a mammal at risk for arrhythmia;
   (b) determining that the mammal is a cytochrome P450(CYP)2D6 extensive metabolizer (EM); and
   (c) administering to the mammal a therapeutically effective amount of a composition comprising an ion channel modulating compound having the structure: 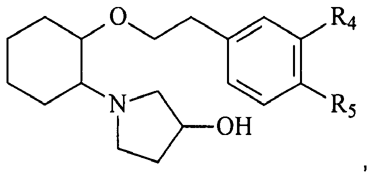
   including isolated enantiomeric, diastereomeric and geometric isomers thereof and mixtures thereof, or a solvate or pharmaceutically acceptable salt thereof; wherein R₄ and R₅ are independently selected from hydroxy and C₁-C₆alkoxy, and
   wherein said compound is administered to the mammal long-term.
29. The method of embodiment 28, wherein the compound is administered orally.
30. The method of embodiment 28 wherein the composition comprises a monohydrochloride salt of the formula: 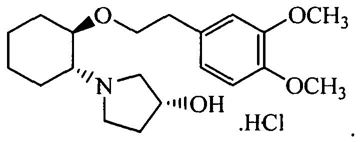
31. A method of identifying a mammal to exclude from long-term administration of an ion channel modulating compound that is metabolized by cytochrome P450(CYP)2D6 comprising: determining that the mammal is a cytochrome P450(CYP)2D6 poor metabolizer (PM).
32. A method of preventing an arrhythmia in a mammal, comprising:
   (a) identifying the mammal as a cytochrome P450(CYP)2D6 extensive metabolizer (EM) or a cytochrome P450(CYP)2D6 poor metabolizer (PM); and
   (b) administering to the mammal a therapeutically effective amount of a composition comprising an ion channel modulating compound having the structure: 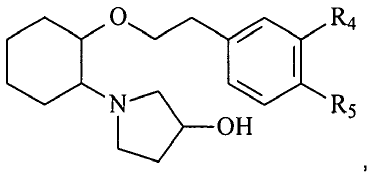
   including isolated enantiomeric, diastereomeric and geometric isomers thereof and mixtures thereof, or a solvate or pharmaceutically acceptable salt thereof; wherein R₄ and R₅ are independently selected from hydroxy and C₁-C₆alkoxy, if the mammal is identified as a cytochrome P450(CYP)2D6 EM.
33. The method of embodiment 32 wherein the composition comprises a monohydrochloride salt of the formula: 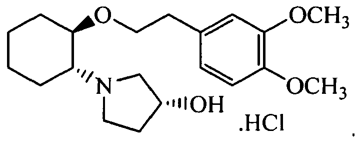
34. A method of preventing an arrhythmia in a mammal, comprising orally administering to a mammal an effective amount of a controlled release tablet formulation comprising vernakalant hydrochloride and one or more pharmaceutically acceptable excipients for a period of time.
35. The method of embodiment 34, wherein the amount of vernakalant hydrochloride administered to the mammal is greater than 600 mg/day.
36. The method of embodiment 35, wherein the amount of vernakalant hydrochloride administered to the mammal is between 600 mg/day and 1800 mg/day.
37. The method of embodiment 36, wherein the amount of vernakalant hydrochloride administered to the mammal is about 1000 mg/day.
38. The method of embodiment 34, wherein at least one of the one or more pharmaceutically acceptable excipients is a hydrophilic matrix system polymer selected from the group consisting of carbomer, maltodextrin, hydroxyethyl cellulose, hydroxypropyl cellulose, hydroxypropyl methyl cellulose, and polyoxoacetate.
39. The method of embodiment 34, wherein the controlled release tablet formulation comprises: about 250 mg of vernakalant hydrochloride; about 100 mg of hydroxypropyl methyl cellulose; about 25 mg preglatinized starch; about 75 mg silicified microcrystalline cellulose; about 67.5 mg of lactose monohydrate; about 3.75 mg stearic acid; and about 3.75 mg magnesium stearate.
40. The method of embodiment 34, wherein the controlled release tablet formulation comprises: about 300 mg of vernakalant hydrochloride; about 120 mg of hydroxypropyl methyl cellulose; about 30 mg preglatinized starch; about 90 mg silicified microcrystalline cellulose; about 81 mg of lactose monohydrate; about 4.5 mg stearic acid; and about 4.5 mg magnesium stearate.
41. The method of embodiment 34, wherein the controlled release tablet formulation comprises: about 300 mg of vernakalant hydrochloride; about 150 mg cetostearyl alcohol; about 105 mg silicified microcrystalline cellulose; about 111 mg of lactose monohydrate; about 4.5 mg stearic acid; and about 4.5 mg magnesium stearate.
42. The method of any one of embodiments 39 to 41, wherein the amount of vernakalant hydrochloride administered to the mammal is about 1000 mg/day.
43. The method of embodiment 34, wherein the effective amount of the controlled release tablet formulation is administered to the mammal in two or more doses per day.
44. The method of embodiment 43, wherein the effective amount of the controlled release table formulation is administered to the mammal in two doses per day, wherein each dose comprises about 500 mg of vernakalant hydrochloride.
45. The method of embodiment 34, wherein the arrhythmia is a recurring arrhythmia.
46. The method of embodiment 34, wherein the arrhythmia is a post-operative arrhythmia.
47. The method of embodiment 34, wherein the period of time is greater than 48 hours.
48. The method of embodiment 47, wherein the period of time is greater than one week.
49. The method of embodiment 48, wherein the period of time is greater than 30 days.
50. The method of embodiment 49, wherein the period of time is greater than 90 days.
51. A method of preventing or postponing the recurrence of an arrhythmia in a mammal, comprising orally administering to the mammal an effective amount of vernakalant hydrochloride, wherein said vernakalant hydrochloride is administered to the mammal at a dosage of about 500 mg b.i.d.
52. The method of embodiment 51, wherein the vernakalant hydrochloride is administered to the mammal in a controlled release oral tablet formulation, wherein each tablet comprises: about 250 mg of vernakalant hydrochloride; about 100 mg of hydroxypropyl methyl cellulose; about 25 mg preglatinized starch; about 75 mg silicified microcrystalline cellulose; about 67.5 mg of lactose monohydrate; about 3.75 mg stearic acid; and about 3.75 mg magnesium stearate.
53. A method of preventing or postponing the recurrence of an arrhythmia in a mammal, comprising orally administering to the mammal an effective amount of vernakalant hydrochloride, wherein said vernakalant hydrochloride is administered to the mammal at a dosage of about 300 mg b.i.d.
54. The method of embodiment 53, wherein the vernakalant hydrochloride is administered to the mammal in a controlled release oral tablet formulation, wherein each tablet comprises: about 300 mg of vernakalant hydrochloride; about 120 mg of hydroxypropyl methyl cellulose; about 30 mg preglatinized starch; about 90 mg silicified microcrystalline cellulose; about 81 mg of lactose monohydrate; about 4.5 mg stearic acid; and about 4.5 mg magnesium stearate.
55. The method of embodiment 53, wherein the vernakalant hydrochloride is administered to the mammal in a controlled release oral tablet formulation, wherein each tablet comprises: about 300 mg of vernakalant hydrochloride; about 150 mg cetostearyl alcohol; about 105 mg silicified microcrystalline cellulose; about 111 mg of lactose monohydrate; about 4.5 mg stearic acid; and about 4.5 mg magnesium stearate.
56. A unit dosage form of vernakalant hydrochloride, comprising between 150 mg and 500 mg of vernakalant hydrochloride.
57. The unit dosage form of embodiment 56, comprising about 250 mg of vernakalant hydrochloride.
58. The unit dosage form of embodiment 56, comprising about 300 mg of vernakalant hydrochloride.
59. The unit dosage form of embodiment 56, comprising about 500 mg of vernakalant hydrochloride.
60. A controlled release tablet formulation of vernakalant hydrochloride, comprising about 250 mg of vernakalant hydrochloride; about 100 mg of hydroxypropyl methyl cellulose; about 25 mg preglatinized starch; about 75 mg silicified microcrystalline cellulose; about 67.5 mg of lactose monohydrate; about 3.75 mg stearic acid; and about 3.75 mg magnesium stearate.
61. A controlled release tablet formulation of vernakalant hydrochloride, comprising: about 300 mg of vernakalant hydrochloride; about 120 mg of hydroxypropyl methyl cellulose; about 30 mg preglatinized starch; about 90 mg silicified microcrystalline cellulose; about 81 mg of lactose monohydrate; about 4.5 mg stearic acid; and about 4.5 mg magnesium stearate.
62. A controlled release tablet formulation of vernakalant hydrochloride, comprising: about 300 mg of vernakalant hydrochloride; about 150 mg cetostearyl alcohol; about 105 mg silicified microcrystalline cellulose; about 111 mg of lactose monohydrate; about 4.5 mg stearic acid; and about 4.5 mg magnesium stearate.

## Claims

1. Vernakalant hydrochloride for use in preventing or postponing the recurrence of an arrhythmia in a mammal, wherein said vernakalant hydrochloride is to be orally administered to the mammal at a dosage of about 500 mg b.i.d.

2. The compound for use according to claim 1, wherein the vernakalant hydrochloride is to be administered to the mammal in a controlled release oral tablet formulation, wherein each tablet comprises: about 250 mg of vernakalant hydrochloride; about 100 mg of hydroxypropyl methyl cellulose; about 25 mg preglatinized starch; about 75 mg silicified microcrystalline cellulose; about 67.5 mg of lactose monohydrate; about 3.75 mg stearic acid; and about 3.75 mg magnesium stearate.

3. Vernakalant hydrochloride for use in preventing or postponing the recurrence of an arrhythmia in a mammal, wherein said vernakalant hydrochloride is to be orally administered to the mammal at a dosage of about 300 mg b.i.d.

4. The compound for use according to claim 3, wherein the vernakalant hydrochloride is to be administered to the mammal in a controlled release oral tablet formulation, wherein each tablet comprises: about 300 mg of vernakalant hydrochloride; about 120 mg of hydroxypropyl methyl cellulose; about 30 mg preglatinized starch; about 90 mg silicified microcrystalline cellulose; about 81 mg of lactose monohydrate; about 4.5 mg stearic acid; and about 4.5 mg magnesium stearate.

5. The compound for use according to claim 3, wherein the vernakalant hydrochloride is to be administered to the mammal in a controlled release oral tablet formulation, wherein each tablet comprises: about 300 mg of vernakalant hydrochloride; about 150 mg cetostearyl alcohol; about 105 mg silicified microcrystalline cellulose; about 111 mg of lactose monohydrate; about 4.5 mg stearic acid; and about 4.5 mg magnesium stearate.
